(19) **Europäisches Patentamt / European Patent Office / Office européen des brevets**

(11) **EP 4 201 959 A1**

(12) **EUROPEAN PATENT APPLICATION**
published in accordance with Art. 153(4) EPC

(43) Date of publication:
**28.06.2023 Bulletin 2023/26**

(21) Application number: **21857059.6**

(22) Date of filing: **26.05.2021**

(51) International Patent Classification (IPC):
*C07K 16/28* $^{(2006.01)}$   *A61K 39/395* $^{(2006.01)}$
*C12N 1/21* $^{(2006.01)}$   *C12N 15/13* $^{(2006.01)}$
*C12N 15/63* $^{(2006.01)}$   *G01N 33/563* $^{(2006.01)}$
*A61P 29/00* $^{(2006.01)}$

(52) Cooperative Patent Classification (CPC):
**A61K 39/395; A61P 29/00; C07K 16/00; C07K 16/28; C12N 15/63; C12N 15/74; G01N 33/563**

(86) International application number:
**PCT/BR2021/050226**

(87) International publication number:
**WO 2022/036422 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.08.2020 BR 102020016890**

(71) Applicant: **Fundação Oswaldo Cruz**
**21040-360 Rio de Janeiro, RJ (BR)**

(72) Inventors:
• **ALMEIDA, Vinicius, Cotta de**
**22221-011 Rio de Janeiro - RJ (BR)**
• **CHAVES, Beatriz**
**60871-165 Fortaleza - CE (BR)**
• **AQUINO, Carolina, Lessa**
**22743-051 Rio de Janeiro - RJ (BR)**
• **DA SILVA, João, Hermínio, Martins**
**60160-110 Fortaleza - CE (BR)**
• **MEDEIROS, Marco Alberto**
**22795-060 Rio de Janeiro - RJ (BR)**
• **SAVINO, Wilson**
**24340-140 Niterói - RJ (BR)**
• **RIEDERER, Ingo**
**22245-100 Rio de Janeiro - RJ (BR)**

(74) Representative: **Zacco Denmark A/S**
**Arne Jacobsens Allé 15**
**2300 Copenhagen S (DK)**

Remarks:
The complete document including Reference Table(s) and the Sequence Listing(s) can be downloaded from the EPO website

(54) **PROTEIN, POLYNUCLEOTIDE, VECTOR, HOST CELL, COMPOSITION, METHOD FOR TREATING AN ILLNESS, IN-VITRO METHOD FOR PREDICTING MULTIPLE SCLEROSIS, AND USE OF A PROTEIN OR COMPOSITION**

(57) The present invention relates to a protein of the scFv type in which said protein comprises a first polypeptide chain and a second polypeptide chain joined by a ligand, having the formula as follows: (VH domain) - (ligand) - (VL domain). The present invention further relates to a polynucleotide comprising the nucleotide sequence shown in SEQ ID NO: 1; to a vector comprising the polynucleotide as defined above; to the host cell comprising the vector as previously defined; and the composition comprising the aforementioned protein and a pharmaceutically acceptable excipient. The present invention further relates to a method for treating a disease or condition that results directly or indirectly from $\alpha 4\beta 1$ integrin activity. The present invention further relates to an *in vitro* method for prognosing multiple sclerosis. The present invention further relates to the use of the previously defined protein or composition in the manufacture of a drug for the treatment of multiple sclerosis.

EP 4 201 959 A1

Key:
Pink: CDR1
Yellow: CDR2
Blue: CDR3

scFv - *Single Chain Fragment Variable*

FIGURE 1

**Description**

TECHNICAL FIELD

The present invention is in the field of immunology. The present invention relates to an scFv-like protein. The present invention further relates to a polynucleotide. The present invention further relates to a vector. The present invention further relates to a host cell. The present invention further relates to a composition. The present invention further relates to a method for treating a disease or condition that results directly or indirectly from $\alpha4\beta1$ integrin activity. The present invention further relates to an *in vitro* method for prognosticating chronic inflammatory diseases. The present invention further relates to the use of a protein or composition to prepare a drug to treat or prognosticate chronic inflammatory diseases.

BACKGROUND OF THE INVENTION

Multiple sclerosis is an autoimmune neurological disease characterized by the action of cytotoxic T lymphocytes against healthy cells that make up the myelin sheath. According to the International Multiple Sclerosis Federation, in 2013, 2.3 million people worldwide were affected by this disease.

For the functional disorder characterized by the attack on healthy tissue to occur, there must be a migration step of lymphocytes toward the target. This migration procedure, known as diapedesis, involves molecular mechanisms that will allow these leukocytes to pass through the endothelium. One of the proteins of great importance in this process are the $\alpha4\beta1$ type integrins, which are present in the membranes of lymphocytes and will mediate the process of adhesion of these cells to the endothelium, so that diapedesis occurs.

Because of its role in lymphocyte migration, integrin $\alpha4\beta1$ has been targeted for multiple sclerosis treatment, as its inhibition would prevent the leakage of these cells that would destroy the myelin sheath. One of the current treatments is the use of Natalizumab, a monoclonal antibody that interacts with these integrins. However, this antibody is not specific for $\alpha4\beta1$ integrins, and is able to interact with $\alpha4\beta7$ integrins as well. In addition, some patients who have undergone this treatment have developed Progressive Multifocal Leukoencephalopathy (PML), a fatal brain disease (HAANSTRA et al., 2013). The appearance of this condition was associated with the application of Natalizumab.

Bioinformatics tools have contributed intensively to the promotion of new drugs and biopharmaceuticals by enabling *in silico* protein engineering. Techniques such as Molecular Modeling, Docking, and Molecular Dynamics have promoted the development and improvement of molecules, in addition to conducting preliminary tests to evaluate their behavior and efficacy.

Against the background of the commercial absence of a specific antibody to $\alpha4\beta1$ integrins that can be used for the treatment of multiple sclerosis, with reduced PML-related side effect risks, or for prognostic purposes, the present work aims to develop a scFv-type antibody specific for these integrins, through *in silico* tools such as modeling, docking and Molecular Dynamics.

Integrins are transmembrane protein receptors that participate in the processes of modulating signaling pathways and cell adhesion. These proteins are heterodimeric consisting of one $\alpha$-subunit and one $\beta$-subunit. Each constituent monomer has an extracellular, transmembrane, and intracellular portion (HYNES, 2002).

In mammals, 18 types of $\alpha$-subunits and 8 types of $\beta$-subunits are reported. There are 24 types of integrins formed by the combination of different $\alpha$ and $\beta$ subunits (HYNES, 2002). The different subunit combinations have made integrins specific for one type of Extracellular Matrix component or other cell receptors, which allows us to differentiate this class of proteins according to their affinity and function. For example, integrins $\alpha3\beta1$, $\alpha6\beta1$, $\alpha6\beta4$ and $\alpha7\beta1$ are laminin receptors (TASHIRO et al., 1999)

The extracellular portion of the structure of an integrin is extremely important for the performance of its function and has some peculiar characteristics. Part of the extracellular $\alpha$-subunit consists of beta-sheets that form a structure called the *beta propeller* (XIONG, 2001). Some types of this subunit contain a kind of additional domain consisting of 200 amino acids, the 1-domain. Binding to integrins that have the 1-domain is through this domain, which makes it a structurally determinant portion for the recognition of the integrin and its ligand. The $\beta$ subunit is structurally similar to the I domain (SRICHAI; ZENT, 2010).

The presence of ions in the outermost portion of the subunits constitutes the so-called MIDAS site (Metal-Ion-Dependent-Adhesion-Site). MIDAS is a strong binding site for integrin and its targets, coordinating the interaction between both molecules. This recognition occurs in a small gap between the $\alpha$ and $\beta$ subunits. Therefore, the two subunits are important in binding the target molecules, so the same subunit can combine with several others, resulting in integrins of different functions and targets.

Integrins in a physiological state can assume two different conformations: activated and inactivated. In the inactivated form, the integrin assumes a conformation that decreases its affinity for the ligand. In the activated form, the structure is in an exposed state, making its binding site more accessible and thus increasing affinity for the ligand (SRICHAI; ZENT, 2010).

Integrin-mediated signaling can be of the extracellular to intracellular (outside-in) or intracellular to extracellular (inside-out) type. Outside-in type signaling refers to the interaction of the extracellular domain of the integrin with some ligand that results in intracellular signals that affect cell growth and differentiation or apoptosis (SRICHAI; ZENT, 2010). These signals are initiated by the formation of a multiprotein complex called FA, which is responsible for the initiation of the signal cascade.

Inside-out signaling is characteristic of cell migration processes, where interaction with the ligand occurs after integrin activation. This type of signaling is important for physiological processes, especially inflammation. Proteins, such as talins, bind to the intracellular domain of the integrin and promote its activation (RATNIKOV; PARTRIDGE; GINSBERG, 2005).

The distribution of $\alpha 4 \beta 1$ integrins, also called VLA-4, in the body is very wide, so that it can be expressed on different cell types, such as NK (Natural Killers) cells (GISMONDI et al. 1991), B lymphocytes (RYAN et al, 1991), T lymphocytes (SATO et al, 1995), monocytes (CHULUYAN & ISSEKUTZ, 1993), eosinophils, basophils (SCHLEIMER et al, 1992), smooth muscle cells (DUPLÀA et al, 1997), myoblasts and myotubes of skeletal muscle (MCDONALD et al, 1995), thymic epithelial cells (NIETO et al, 1996) and dendritic cells. Despite the range of cells that express the $\alpha 4 \beta 1$ integrin, T lymphocytes and monocytes are the main cell types related to VLA-4 expression in the context of inflammatory and $\alpha 4 \beta 1$-linked diseases, due to the transmigration of these cells mediated by such an integrin.

Seven extracellular ligands are recognized by VLA-4, these being: thrombospondin, MAd-CAM-1 (Mucosal vascular addressin Cell Adhesion Molecule 1), osteopontin, ADAM-28 (A Disintegrin And Metalloproteinase-28), ICAM-4 (Intercellular Adhesion Molecule 4), VCAM-1 (Vascular Cell Adhesion Molecule 1) and fibronectin (TAKEDA et al, 2007). Among these ligands, the last two are the most relevant regarding the function of VLA-4 and its involvement in different diseases. VCAM-1 is one of the two major ligands of VLA-4, this being the key molecule by which this integrin mediates the transendothelial migration of T lymphocytes and monocytes to the target tissue during an inflammatory process. The fibronectin (FN), in turn, is an ECM protein that is also a major ligand of VLA-4. The interaction mediated by VLA-4 and fibronectin is important for different cellular processes such as migration, survival and differentiation. This ligand has different interaction sites for $\alpha 4 \beta 1$ and is also recognized by integrin $\alpha 5 \beta 1$ (ROSEMBLATT et al., 1991).

In chronic diseases the inflammatory process occurs recurrently, and in the absence of a pathogen or damage that primarily induces this response. Chronic inflammation occurs in different systems of the body, and consequently various diseases are characterized, according to the site of inflammation. The main chronic inflammatory diseases with VLA-4 mediated steps are: Multiple Sclerosis (MS), asthma, rheumatoid arthritis, stroke, and inflammatory bowel disease (IBD), such as Crohn's disease, (HYUN et al., 2009) and Duchenne Muscular Dystrophy (DMD). Other inflammatory diseases also related to $\alpha 4 \beta 1$ are dry eye disease, uveitis, and allergic conjunctivitis (ECOIFFIER et al, 2008). Despite the association of VLA-4 proteins with inflammatory processes, they are also related to other physiological phenomena. For example, its role in hematopoiesis (LOBB; HEMLER, 1994) and in heart development (WINGERD et al., 2002) is described. Recent work has sought to evaluate the teratogenic potential of these integrins (SAKURAI et al., 2014), because of their involvement with cardiac myocytes. In addition, the association of these proteins with some types of cancer and their developmental stages is being investigated. As a result of these investigations, new associations have been made, such as the discovery that heparin is able to inhibit melanoma metastasis by blocking $\alpha 4 \beta 1$ integrins (SCHLESINGER et al., 2014).

Antibodies are proteins produced by B lymphocytes in protective response to a certain antigen. The functions of an antibody for the body are neutralization and opsonization of the antigen, activation of the complement system, and cell-mediated cytotoxicity (ABBAS, 2015).

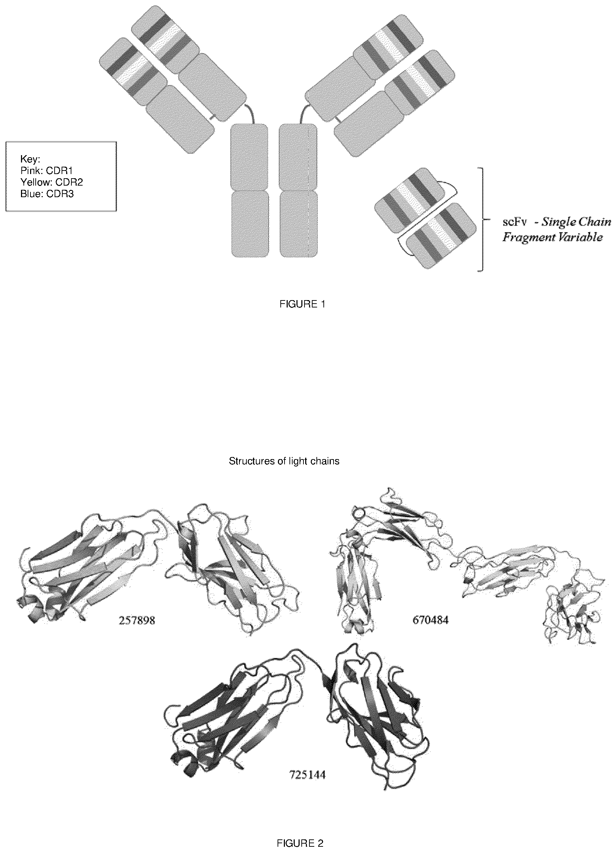

The structure of an antibody consists of two light chains and two heavy chains. Each chain is made up of constant and variable regions, so that heavy chains are made up of three or four constant regions (Ch) and one variable region (Vh). Light chains are made up of a constant (Cl) and a variable (Vl) region. The conformation of the four chains gives the antibody a "Y" shape (Figure 1).

The different regions that make up the antibodies are called Ig domains, that is, an immunoglobulin molecule. An Ig domain is formed by beta sheets that are connected by a disulfide bridge, forming a cylinder-like structure. The characteristic fold of an Ig domain is called an immunoglobulin fold (MURPHY; TRAVERS; WALPORT, 2010).

The variable regions, Vl and Vh, represent the portion of the antibody responsible for antigen recognition, and therefore have different compositions to ensure recognition specificity. The constant regions, Cl and Ch, function as signaling molecules for other molecules in the immune system, triggering the effector functions of the antibodies. Therefore, the composition of these regions is preserved (MURPHY; TRAVERS; WALPORT, 2010).

The portion of the antibody responsible for antigen recognition is called Fab while the portion responsible for effector functions is called Fc (figure 1). The Fab portion consists of the variable regions and a constant region of each chain. The Fc portion consists only of constant regions.

Antibodies can be divided into classes or isotypes according to the differences in the constant regions of the heavy chain and their conformation. According to these criteria, the antibodies can be of type IgA, IgD, IgE, IgG and IgM. IgA is a characteristic mucosal defense antibody, circulates in dimer form, and has three constant regions in the heavy chain. IgD is an antibody present only on the membrane of B lymphocytes. IgE is related to defense against helminths and allergic processes, circulates in monomeric form, and has four constant regions on the heavy chain. IgG is the most abundant isotype, is related to the processes of opsonization, complement activation, neonatal immunity and cell-mediated cytotoxicity, circulates in monomeric form and has three constant regions in the heavy chain. The IgM isotype may be present on the membrane of naive B lymphocytes as receptors and may circulate in the body in pentamer form. IgM is related to complement activation and its heavy chain is formed by four constant regions (MURPHY; TRAVERS; WALPORT, 2010).

In addition to their role in the immune system, antibodies are widely used biotechnological tools. These biomolecules can be used for identification of phenotypic markers, as immunodiagnostic tools, in tumor detection, therapies, and functional analysis of cells and molecules (ABBAS, 2015).

Within the variable regions of the antibodies, there are hypervariable portions that will effectively bind to the antigen. This portion is called Complementarity Determining Region (CDR). Each chain has three CDRs, and each antibody is highly polymorphic in these regions of hypervariability, maximizing recognition specificity (figure 1).

There is an antibody format consisting only of the Vl and Vh regions connected by a peptide ligand, retaining all the CDRs of each chain, totaling six CDRs (three from the heavy chain and three from the light chain) (figure 1). This is the smallest type of antibody that can maintain its recognition function and specificity and is called scFv, Single-Chain Fragment Variable (SHEN et al., 2005).

The use of these antibody fragments has advantages over the use of whole antibodies such as: better penetration of tumors, reduced immunogenicity, shorter retention time in undesirable tissues, and ease of expression in bacteria (AHMAD et al., 2012).

ScFvs are useful in many areas, ranging from medical applications to biosensors. The use of this type of antibody is described in the therapy of arthritis, Chron's disease, and breast cancer (AHMAD et al., 2012). There is also work that demonstrates the application of scFvs as tools for specific antigen detection (SPAIN et al., 2016). In addition, other studies explore the pharmacological and drug delivery applications of scFvs (LU et al., 2016).

The elucidation of the three-dimensional structures of proteins provides important information for the development of some areas of science, especially biotechnology. The knowledge of the three-dimensional structure of proteins allows the discovery and exploration of molecular targets to obtain drugs, regions prone to mutations to obtain enzymes and antibodies with greater effectiveness in their applications and helps in understanding the role of a particular protein in a biochemical pathway, making it a target of Metabolic Engineering.

Obtaining the tertiary structure of a protein experimentally occurs mainly by the technologies of X-ray diffraction - Biocrystallography - and Nuclear Magnetic Resonance (NMR). Despite their effectiveness, these techniques are laborious and expensive, constituting a limiting factor for the acquisition of new protein structures. Therefore, other tools for obtaining these structures were required, which led to the beginning of the development and use of molecular modeling methods.

Molecular modeling is the *in silico* prediction of the three-dimensional structure of a biomolecule, usually proteins. There are three ways to obtain the three-dimensional structure of proteins by molecular modeling: comparative modeling, threading, and *ab initio* (KHAN et al., 2016).

Comparative modeling is the most accurate method for *in silico* prediction of structures compared to the other methods (KHAN et al., 2016). This form of modeling consists of using a model sequentially similar to the target protein to assemble its structure. The template used has its three-dimensional structure elucidated and deposited in databases and is used as a reference for modeling according to the degree of identity between the proteins involved. The steps in comparative modeling consist of local alignment to identify candidate models and select the best model, global alignment to the template sequence, and structure construction and validation (SANCHEZ & SALI, 1997).

The choice of reference protein for modeling depends mainly on how identical this protein will be compared to the sequence to be modeled. Therefore, local alignment of the target sequence against databases of proteins with known three-dimensional structure, such as the Protein Data Bank, PDB, is required for candidate template discovery (BERMAN et al., 2000). The choice of template takes into consideration factors such as: having identity above 30% with the target sequence, protein-related parameters - such as pH and ligands -, and the quality of the deposited structure by evaluating criteria such as resolution, R-factor, and number of constraints per residue (KHAN et al., 2016).

After the choice of template, global alignment of the two protein sequences is required. The global alignment will allow for a fulllength pairing of the two sequences, which will be important for model building.

After the global alignment, the phase of obtaining the three-dimensional structure of the protein is initiated, which will subsequently be validated. There are three strategies adopted for model construction: rigid body assembly, segment matching, and spatial constraint satisfaction (KHAN et al., 2016).

The first strategy uses rigid bodies generated from the alignment of proteins to form a three-dimensional model. It is a method that relies on the conservation of the center regions and side chains that make up the protein's tangles (BLUNDELL et al., 1987).

Segment-matching assembly is based on the principle that there are one hundred classes of hexamers (peptides with six residues) that act as building blocks for structure assembly. The modeling occurs using hexamers as a base, which are replaced by the segments of the protein to be sequenced, according to conformational similarity (UNGER et al., 1989).

The last comparative modeling strategy uses constraints obtained from the alignment between the sequences. The fundament of the technique is that the equivalent angles and distances between aligned residues lead to the generation of constraints, that is, the spatial coordinates of a residue obey a limit, a constraint, that is imposed based on the biochemical fundamentals and the alignment made. The model is then built while respecting these constraints (KHAN et al., 2016).

Regardless of the modeling strategy used, it is necessary that the structure obtained be validated. For this purpose, there are servers, such as SAVES and MolProbity, that evaluate physical, stereochemical and statistical parameters for the built models, providing information about the quality and reliability of the structure.

There are situations where comparative modeling is not indicated, for example when the template protein has an identity of less than 30%. In this case, the three-dimensional structure can be obtained by threading or *ab initio.* Threading modeling does not require sequence similarity but is based on the principle that proteins can have similar cohesiveness even though their sequences are distinct. *Ab initio* modeling, on the other hand, uses algorithms that will predict the spatial coordinates without a referential as a model, based on physical fundamentals.

Molecular Modeling is therefore a potential methodology for obtaining the three-dimensional structure of antibodies and constructing scFvs, enabling the use of their structures in other computational tools.

The advances in the areas of drug discovery and drug design provided by the need for new drugs and biopharmaceuticals has generated a demand for a fast and effective tool for the evaluation of the ability of biomolecules to interact with certain ligands. Given this demand, molecular docking platforms have become important tools commonly used in these areas (JLTG; ANDERLUH; TOMAŽIĊ, 2015). Molecular docking is used to delineate interactions between certain molecules and a target protein at atomic levels, tracking the behavior of these molecules at the protein binding site and elucidating the biochemical process involved (MCCONKEY; SOBOLEV; EDELMAN, 2002). There are two basic steps in this process: the prediction of the conformation of the ligand and its orientation at the binding site, and the evaluation of the binding affinity (MENG et al., 2011). These two steps are done differently between the existing platforms, and there are therefore different algorithms for the spatial fitting of the ligands and the calculation of the affinity.

There are several algorithms for the spatial orientation phase of the ligand, most notably the Matching Algorithms (MA), Incremental Construction (IC), Monte Carlo (MC) and Genetic Algorithms (GA). MAtype algorithms rely on the molecular form of the ligand and its chemical properties to indicate the best pose within the binding site. IC algorithms seek to insert the ligand into the binding site in a piecemeal and refined manner to establish orientation. MC types, on the other hand, originate positions from the rotation and/or translation of rigid bodies and connections. GAs transform degrees of freedom of the ligand into binary codes that are said to be genes, the organization of these genes constitutes a chromosome that represents the position of the ligand (MENG et al., 2011).

The calculation of binding affinity is done by score functions, which can be divided into three types: force-field-based, empirical, and knowledge-based. Force field-based score functions are based on the sum of the interaction energies and the internal energy of the ligand. Empirical functions use experimental data of the molecules involved to obtain the score, resulting in a sum of several parameters. Knowledge-based functions, on the other hand, use interaction potentials between atoms (KITCHEN et al., 2004).

The two steps of docking are common to the programs and servers that perform it, however, different performance methodologies are employed. These methodologies differ in the options of fixing or relaxing the docking components, protein and ligand, for their realization. There are, therefore, three methodologies: rigid protein and ligand, adopted by programs such as DOCK and FLOG, rigid protein and flexible ligand, used by programs such as AutoDock and FlexX, and with both components flexible, as in the case of IFREDA and QXP programs (MENG et al., 2011).

The production of antibodies, engineered enzymes, or other proteins for commercial purposes has as one of its limitations the expenditure of resources in the test and development phase. It is necessary that these biomolecules are tested under different conditions according to their application in order to have efficiency in their use, thus justifying their commercialization. However, these tests require time, reagents, and equipment, and in case of negative results, there is a loss of these resources, which is undesirable for the institution or company that proposes to develop this process. Therefore, it is of great interest to researchers, entrepreneurs and scientists in the area that there is a way to computationally simulate the performance of their molecules, through an algorithm that provides these conditions *in silico.* This demand is met through Molecular Dynamics (MD) tools.

DM techniques use fundamentals of Classical Mechanics to predict the behavior of each atom in a given system over time (NAMBA; SILVA; SILVA, 2008). During the process, several factors are considered such as the coordinates of the atoms, the composition of the system, initial velocities and potentials that when combined with the appropriate algorithm for the system, and its conditions, will provide the results (PIÑEIRO, 2009).

To perform molecular dynamics, it is necessary to process the files that correspond to the molecules that will be submitted to this process. This treatment will originate the appropriate system for the simulation to take place, since initially you only have the coordinates of the atoms in the space of the molecule or complex that will be the focus of the simulation.

The first step in achieving an appropriate system for DM is the generation of the topology, which will start the creation of the system. The topology file will contain all the information regarding which atoms make up the molecules, what their positions, forces, velocities, what bond types are present, the angles between the atoms, and all the attributes regarding the molecule organized for the interpretation of the program so that all these conditions are taken into consideration at the time of simulation (GROMACS, 2016).

In order to create the topology, a force field must be defined, which will designate terms for the inclusion and organization of information, such as bond properties and potential energies (GROMACS, 2016). There are several types of force fields, which will vary in the way the parameters considered are combined. The force field is critical for summarizing and integrating all the data regarding the DM target. The choice of using one type of force field will depend on the type of simulation and the nature of the molecules involved.

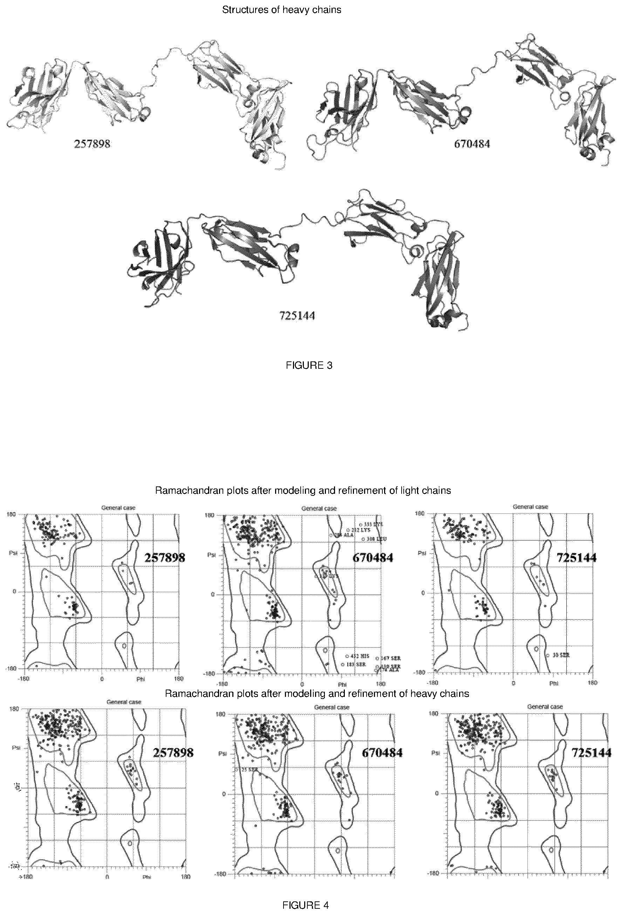

After the topology file has been generated, a so-called Periodic Boundary Condition (PBC) is applied. The PBC consists of delimiting the system by adding the construction of a box, where inside the box the DM will take place (figure 4). The application of this condition serves to reduce the surface effect. In addition, this condition ensures that the number of atoms in the system is conserved during the simulation, because the box containing the system is replicated in various directions in space, thus ensuring that when an atom leaves from one side of the box it is able to return from the other side (NAMBA; SILVA; SILVA, 2008). The bounding box can have different shapes, such as cubic and triclinic.

It is necessary that the molecules are immersed in a solvent for the simulation to be faithful to real conditions, so the next step is to add a solvent to the system (figure 4). Usually, the solvent used is water. There are several water models that vary in terms of their diffusivity, radial distribution, and other factors (KHAN et al., 2016). As with the choice of force field, the choice of water model type varies with the type of simulation and the nature of the system. Once the system is immersed in a solvent, neutralization of charges must occur through the addition of ions, such as sodium or chlorine, to the medium.

Once the system is solvated and neutralized, energy minimization is required before the simulation can be run for unrealistic interactions (PIÑEIRO, 2009) and thus adapt the system to a thermodynamically favorable state. The main algorithms used for minimization are steepest descent, conjugate gradients, and Newton-Raphson (NAMBA; SILVA; SILVA, 2008).

Energy minimization algorithms vary in the way of obtaining an energetically most favorable state. The steepest descent method makes corrections to the unfavorable geometries of the molecules in the system. The conjugate gradient method is a derivation of steepest descent, with the advantages of being faster and including steering arrangements. The Newton-Raphson method is based on the use of the hessian function (KHAN et al., 2016).

After minimization, it is recommended that an equilibrium phase or thermalization of the system be performed. This thermalization is a short DM simulation. After this equilibrium state, the simulation itself is performed, also called production dynamics.

DM techniques consist of integrating the Newtonian equations of motion, so that the forces acting on the atoms in the system are supervised over time, as well as the trajectory of each atom. The main algorithms used that conduct DM, responsible for integrating the equations of motion, are Verlet's algorithm, Beeman's algorithm, and the leap-frog method (NAMBA; SILVA; SILVA, 2008).

During the integration phase, the system must be in a state where certain attributes remain constant. This state is referred to as *ensemble* and is classified according to the properties that are held constant (REBOUQAS, 2015). The most commonly used ensembles are of the NVT (constant number of moles, constant volume, constant temperature) and NPT (constant number of moles, constant pressure, constant temperature) type.

Once the *ensemble* and the algorithm for DM are defined, the production simulation takes place. Different results are obtained from the simulation output files. Parameters such as: energy, RMSD (Root Mean Square Deviation), RMSF (Root Mean Square Fluctuation) can be evaluated, hydrogen bonds, salt bridges, protein secondary structure, spin radius, angles and dihedra, and protein-related items and interfaces (GROMACS, 2016).

Molecular Dynamics, therefore, can be used for the evaluation of the behavior of an isolated scFv in contact with antigen, allowing the analysis of the interaction between antibody and antigen.

Due to the involvement of VLA-4 in various pathological processes, the development of inhibitors has become an important therapeutic strategy. Despite several studies aimed at developing anti-VLA-4 molecules, according to the Biocentury® database, only three anti-VLA-4 inhibitors are already on the market or in clinical trials 2 or 3: Firategrast, AJM300 and Natalizumab.

Firategrast ($C_{27}H_{27}F_2NO_6$) is a small molecule antagonist to $\alpha4\beta1$ and $\alpha4\beta7$ (LPAM-1) (GROVE RA et al, 2013) and has been investigated for the treatment of MS, presenting advantages such as oral availability and low half-life (2 to 5 hours) (MILLER et al, 2012). Oral administration makes the drug easier to apply and accept, while the low half-life decreases safety-related risks of anti-VLA-4 molecules, thus reducing the chances of the patient developing an opportunistic disease during treatment. This drug has undergone phase 2 clinical evaluation, in which doses of 900 mg or 1200 mg applied twice daily demonstrated significant reduction in the number of MS lesions, showing it to be a potential drug to be used in the treatment of this disease (MILLER et al, 2012).

AJM300 ($C_{25}H_{19}C_{12}N_3O_5$), Like Firategrast, it is a small molecule $\alpha 4$ integrin antagonist, currently in phase 3 clinical evaluation. This drug has oral availability, a low half-life, and is being investigated for the treatment of IBD (SUGILTRA T et al, 2013). During phase 2 evaluation, it was shown that doses of 960 mg applied three times daily in patients with ulcerative colitis led to a significant increase in clinical response scores, clinical remission, and mucosal healing, and this drug is therefore a VLA-4 inhibitor with potential use for treating this disease (YOSHIMURA et al, 2015).

Natalizumab, or Tysabri, is a drug composed of humanized monoclonal antibodies, which contain CDRs (Complementarity-Determining Regions) derived from a murine anti-CD49d (anti-$\alpha 4$) antibody inserted into a human IgG4 frame (SCHWAB N et al, 2015). It is able to bind to the integrins VLA-4 and LPAM-1 through recognition of the $\alpha 4$ subunit, and is approved for use in the treatment of Crohn's disease and MS.

Natalizumab is currently used in cases of relapsing remitting multiple sclerosis (RRMS), which is the recurrence of acute clinical episodes of the disease. This form is usually characterized by symptomatic flare-ups lasting from days to weeks and may lead to the formation of new brain lesions during the flare-up period, followed by a period of improvement. Natalizumab is indicated for patients who have had at least one to two flare-ups within 12 months of starting treatment (such as IFN-$\gamma$ and glatiramer acetate), have easily detectable lesions and an increase in the number of these lesions within 6 to 12 months after the start of a previous treatment. In such cases, Natalizumab is applied once every four weeks at a dose of 300 mg (SCHWAB N et al, 2015).

Despite the efficacy of using Natalizumab in the treatment of RMS, it can reduce the frequency of outbreaks by up to 68 % (POLMAN CH et al, 2006), One of the limitations related to the use of this drug is its association with the risk of progression of a serious brain disease, PML (Progressive Multifocal Leukoencephalopathy). PML is an opportunistic brain infection caused by the JC virus (John Cunningham virus). During clinical evaluation phase 3 of Natalizumab, three patients undergoing treatment for RMS manifested the disease after starting the drug and died due to progression of PML, leading to withdrawal of the drug in 2005 and its reintroduction in 2006 (BLOOMGREN et al, 2012). Studies show that some factors influence patients' risk of developing PML, such as prior use of immunosuppressants, presence of anti-JC virus antibodies, and increased duration of Natalizumab treatment (BLOOMGREN et al, 2012), which also causes immunosuppressive effects.

Currently, a 15 mL bottle of Natalizumab 20 mg/mL, referring to one dose, is marketed at a price between R$ 6,200.00 and 7,163.65 (https://consultaremedios.com.br/tysabri/p). 2013, the Ministry of Health has published a report regarding the distribution and use of this drug as a secondline treatment for RMS. The cost of one dose of Natalizumab for SUS (Brazilian Unified Health System) is R$2,245.00, so that during the implementation phase, the company Biogen - responsible for the production and commercialization of Natalizumab - estimated a budget impact, related to the supply of Natalizumab, of R$8 million over 5 years (www.saude.gov.br/sctie). Therefore, the cost of treating EMRR using this drug is extremely high, both for supplying it through the government and for private purchase.

Although current anti-VLA-4 drugs are aimed at treating MS and IBD, blocking VLA-4 by various inhibitors has shown promise in different diseases. Studies in mice show that application of anti-$\alpha 4$ antibodies inhibited the growth of myelomas, highlighting their potential in anti-cancer therapy (Olson et al, 2005). Other work, using an ovine model, has demonstrated the promising application of VLA-4 inhibitor molecules in the treatment of asthma (SINGH J et al, 2004). In addition, several papers present the use of inhibitors of this integrin in the treatment of less obvious diseases, such as dry eye disease (ECOIFFIER et al, 2008) and sickle cell disease (WHITE et al, 2016).

CHAVES, B. (2016 (1)) aimed to construct a single-chain variant antibody dedicated to having affinity only for integrin $\alpha 4\beta 1$. All work was done *in silico,* and the chains were modeled and tested in various programs (Modeller, EMBOSS, BLAST, MolProbity, Verify 3D, Coot, PyMol, and servers GetArea, Robetta Alanine Scaning, Haddock, PDBePISA, Gromacs 5.1 package,). Six possible scFvs were modeled and evaluated, and after mutagenesis step, only two potential sequences and the control sequence of the scFvs were selected for molecular dynamics simulation (scFv B, scFv X and native scFv). Important to note is item 4.9 about targeted mutagenesis of scFvs, directed at interacting hotspots to make the surroundings a strong point of interaction. It was concluded that the modified scFv 257898 had better simulations. No specific scFv sequence was revealed, but native sequence codes (prior to bioinformatics steps) obtained from Integrity (Ex: 257898 - Takedas's Vedozulimab).

CHAVES, B. et al. (2016 (2)) released three Integrity sequence sources used for genetic modifications (257898, 670484, 725144). A GGGGS binder was also revealed. All development is *in silico* and highlights the potential of a specific, undisclosed molecule.

YUAN Q et al. (1996) describes the use of a single chain antibody (sFv) prepared from the HP1/2 antibody, with an already known anti integrin $\alpha 4$ effect (YEDNOCK et al., 1992). An interchain ligand (GGGGS)3 and retention sequence in the endoplasmic reticulum (KDEL) were added. The humanized sequence of the mouse mAb HP1/2 was designed, named hHP1/2, and used to construct the antibody (sFv-195). It was then modified by PCR and synthetic double-stranded oligonucleotides inserted. The two domains (heavy portion - VH - and light portion - VL), ligands (GGGGS)3 , KDEL sequences, and the convenient restriction sites, are depicted in the schematic below (figure 1). The above construct was verified, inserted into plasmid (pUC19) used to transform bacteria. After confirmation, it was included in mammalian expression plasmid using the expression vector pMHneo and transfected into Jurkat cells. It was possible to observe at least 65% to 100% decrease, in some samples, of $\alpha4\beta1$ integrin expression in Jurkat cells, in addition to virtually extinguishing cell motility in *in vitro* tests.

CLARK, L. A. et al. (2006) discloses the optimization of the affinity of a monoclonal antibody, said AQC2, and integrin $\alpha1\beta1$ (VLA-1), specifically, aiming to inhibit the transmigration of T lymphocytes and monocytes in the regions of inflammation remedying arthritis. The strategy used was single-point mutation of strategic locations, e.g., periphery of the antibody-antigen interface. The antibody sequence with the highest optimization were those mutated at sites S28Q and N52E of the heavy chain, and at T50V and K64E of the light chain. The final affinity obtained was 10 times higher.

Document US 2018/0369330 points out proteins to inhibit the binding of an integrin by stabilizing the "E-H+" protein conformation, increasing its occurrence and/or duration, using integrin-based therapeutic proteins (antibody, antibody fragment, synthetic antibody, fusion protein, small protein molecule) for the purpose of treating inflammatory and/or immune system-modulated diseases. The fragment includes the embodiment of scFv. From the presented technical problem related to the occurrence of PML in drug treatments targeting integrins $\alpha4\beta1$ and $\alpha4\beta7$, i.e., natalizumab, stabilizing and/or modifying compounds are proposed by the present invention. Such a protein is therapeutic, as described above, and is able to inhibit one of the integrins, including $\alpha4\beta1$.

Document US 10,335,485 lists antibody specific for VLA-4, with novel advantage with increased affinity for integrin $\alpha4\beta1$ when compared to natalizumab (p.11, 1.57) generating a decrease in the occurrence of PML. The gain in affinity is possible through humanization of the murine HP1/2 parental sequence already known in the prior art (HANF et al., 2014). The described therapy targets multiple sclerosis, asthma (moderate to severe), rheumatoid arthritis, diabetes, and Crohn's disease. It also describes that the protein sequence would be a single chain Fv molecule (scFv).

## SUMMARY OF THE INVENTION

Currently, the only commercially available therapeutic monoclonal antibody that interacts with $\alpha4\beta1$ integrins is Natalizumab, used in multiple sclerosis treatments. However, this antibody is not only specific for $\alpha4\beta1$, but also interacts with $\alpha4\beta7$. In addition, its application in some patients provided the recrudescence of a brain disease, Progressive Multifocal Leukoencephalopathy. Therefore, its use is limited. In this context, the aim of this work was to perform the *in silico* construction of an $\alpha4\beta1$ integrinspecific scFv. Antibodies of the scFv type, *Single Chain Fragment Variable,* which are a small antibody format, conserving the hypervariable regions of the light and heavy chains and thus preserving their specificity, were tested. Among the main advantages of the therapeutic use of these antibody fragments are the ability to penetrate tissues inaccessible to complete antibodies, lower associated production cost, since these fragments can be produced in simpler heterologous systems such as bacteria and yeast, absence of the Fc portion, lower immunogenicity, and shorter retention time in unwanted tissues.

In one aspect, the present invention relates to a scFv-like protein comprising a first polypeptide chain and a second polypeptide chain joined by a ligand, having the formula as follows: (VH domain) - (ligand) - (VL domain), where the VH domain comprises at least amino acids N173, Y176, K181, Y217, Y222 from SEQ ID NO: 3 and the VL domain comprises at least amino acids K31, Y33, N35 from SEQ ID NO: 3. In one embodiment, domain VH comprises the complementarity-determining regions (CDR1, CDR2, CDR3) consisting of SEQ ID NO: 10, SEQ ID NO: 11 and SEQ ID NO: 12 and domain VL comprises the complementarity-determining regions (CDR1, CDR2, CDR3) consisting of SEQ ID NO: 7, SEQ ID NO: 8 and SEQ ID NO: 9. In another embodiment, the VH domain comprises amino acids Q117 to S237 of SEQ ID NO: 3 and the VL domain comprises amino acids D1 to I111 of SEQ ID NO: 3. In another embodiment, the ligand is 5 to 15 amino acids in length. In another embodiment, the binding peptide comprises at least the sequence GGGGS. In another embodiment, the protein selectively binds to integrin $\alpha4\beta1$. In another embodiment, the protein is for use in a method for the prognosis or treatment of chronic inflammatory diseases, preferably multiple sclerosis.

In another aspect, the invention relates to a polynucleotide comprising the nucleotide sequence shown in SEQ ID NO: 1.

In another aspect, the invention relates to a vector comprising the polynucleotide as previously defined.

In another aspect, the invention relates to a host cell comprising the vector as previously defined. In one embodiment, the host cell is a bacterial cell. In another embodiment, the bacterial cell is an *E. coli* cell.

In another aspect, the invention relates to a composition, comprising the aforementioned protein and a pharmaceutically acceptable excipient. In one embodiment, the composition is for use in the treatment of or prognosis of chronic inflammatory diseases, preferably multiple sclerosis.

In another aspect, the invention relates to method for treating a disease or condition that results directly or indirectly from $\alpha4\beta1$ integrin activity, comprising administering to a human being a protein a composition as previously defined. In one embodiment, the disease or condition is a chronic inflammatory disease, preferably multiple sclerosis.

**EP 4 201 959 A1**

In another aspect, the invention relates to an *in vitro* method for prognosing chronic inflammatory diseases comprising contacting at least one protein as previously defined or a composition as previously defined with a cell, tissue or sample from an individual, detecting the binding of the protein to the cell, tissue or sample, quantifying the expression of VLA-4 and indicating a most suitable treatment for the patient.

In another aspect, the invention relates to the use of the previously defined protein or composition to prepare a medicament to treat or prognosticate chronic inflammatory diseases, preferably multiple sclerosis .

BRIEF DESCRIPTION OF THE FIGURES

The purpose of the invention, together with additional advantages thereof, may be better understood by reference to the attached figures and the following descriptions:

Figure 1 - Conformation of the chains of an antibody and an scFv antibody fragment. Pink: CDR1; Yellow: CDR2; Azul: CDR3

Figure 2 - Obtained structures of the antibody light chains;

Figure 3 - Obtained structures of the antibody heavy chains;

Figure 4 - Ramachandran plots generated by MolProbity for the modeled chains. The dots represent the amino acid residues of each antibody chain. The regions outlined in light blue represent favorable regions, in dark blue allowed regions, and those not outlined represent impermissible regions

Figure 5 - Structures of all obtained scFvs

Figure 6 - Haddock Score values of the dockings of native and modified scFvs with the $\alpha4\beta1$ and $\alpha5\beta1$ integrins;

Figure 7 - Cluster size values of the dockings of native and modified scFvs with the integrins $\alpha4\beta1$ and $\alpha5\beta1$

Figure 8 - RMSD in Å dockings of native and modified scFvs with the integrins $\alpha4\beta1$ and $\alpha5\beta1$

Figure 9 - RMSD values of simulations of the native scFv with the integrins $\alpha4\beta1$, $\alpha5\beta1$ and $\alpha4\beta7$

Figure 10 - RMSD values of scFv B simulations with the integrins $\alpha4\beta1$, $\alpha5\beta1$ and $\alpha4\beta7$

Figure 11 - RMSD values of the scFv X simulations with the integrins $\alpha4\beta1$, $\alpha5\beta1$ and $\alpha4\beta7$

Figure 12 - RMSF values of the native scFv, B and X simulations with the $\alpha4\beta1$ integrins

Figure 13 - RMSF values of the simulations of the native scFv, B and X with the $\alpha4\beta7$ integrin

Figure 14 - RMSF values of the simulations of the native scFv, B and X with the $\alpha5\beta1$ integrin

Figure 15 - Spin radius values from simulations of the native scFv with the integrins $\alpha4\beta1$, $\alpha5\beta1$, and $\alpha4\beta7$

Figure 16 - Spin radius values from scFv B simulations with integrins $\alpha4\beta1$, $\alpha5\beta1$, and $\alpha4\beta7$

Figure 17 - Spin radius values from scFv X simulations with the integrins $\alpha4\beta1$, $\alpha5\beta1$, and $\alpha4\beta7$

Figure 18 - Number of hydrogen bonds during simulations of native scFv with the integrins $\alpha4\beta1$, $\alpha5\beta1$ and $\alpha4\beta7$

Figure 19 - Number of hydrogen bonds during simulations of scFv B with the integrins $\alpha4\beta1$, $\alpha5\beta1$ and $\alpha4\beta7$

Figure 20 - Number of hydrogen bonds during the scFv X simulations with the integrins $\alpha4\beta1$, $\alpha5\beta1$ and $\alpha4\beta7$

Figure 21 - Salt bridges between native scFv and integrin $\alpha4\beta1$ during simulation

Figure 22 - Salt bridges between native scFv and integrin $\alpha4\beta7$ during simulation

Figure 23 - Salt bridges between native scFv and integrin $\alpha5\beta1$ during simulation

Figure 24 - Salt bridges between scFv B and integrin $\alpha4\beta1$ during simulation

Figure 25 - Salt bridges between scFv B and integrin $\alpha4\beta7$ during simulation

Figure 26 - Salt bridges between scFv B and integrin $\alpha5\beta1$ during simulation

Figure 27 - Salt bridges between scFv X and integrin $\alpha4\beta1$ during simulation

Figure 28 - Salt bridges between scFv X and integrin $\alpha4\beta7$ during simulation

Figure 29 - Salt bridges between scFv X and integrin $\alpha5\beta1$ during simulation

Figure 30 - Total energy values from simulations of the native scFv with the integrins $\alpha4\beta1$, $\alpha5\beta1$, and $\alpha4\beta7$

Figure 31 - Total energy values of scFv B simulations with the integrins $\alpha4\beta1$, $\alpha5\beta1$ and $\alpha4\beta7$

Figure 32 - Total energy values of the scFv X simulations with the integrins $\alpha4\beta1$, $\alpha5\beta1$ and $\alpha4\beta7$

Figure 33 - Coulomb Potential values from simulations of the native scFv with the integrins $\alpha4\beta1$, $\alpha5\beta1$, and $\alpha4\beta7$

Figure 34 - Coulomb Potential values from scFv B simulations with the integrins $\alpha4\beta1$, $\alpha5\beta1$ and $\alpha4\beta7$

Figure 35 - Coulomb Potential values from the scFv X simulations with the integrins $\alpha4\beta1$, $\alpha5\beta1$ and $\alpha4\beta7$

Figure 36 - Lennard-Jones Potential values from simulations of native scFv with the integrins $\alpha4\beta1$, $\alpha5\beta1$, and $\alpha4\beta7$

Figure 37 - Lennard-Jones Potential values from scFv B simulations with the integrins $\alpha4\beta1$, $\alpha5\beta1$, and $\alpha4\beta7$

Figure 38 - Lennard-Jones Potential values from scFv X simulations with the integrins $\alpha4\beta1$, $\alpha5\beta1$, and $\alpha4\beta7$

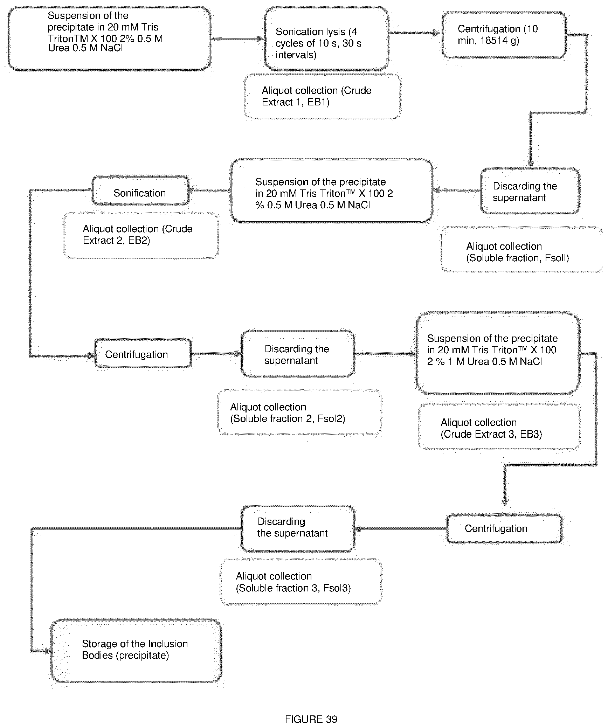

Figure 39 - Step by step for lysis, washing and obtaining the inclusion bodies formed by scFv

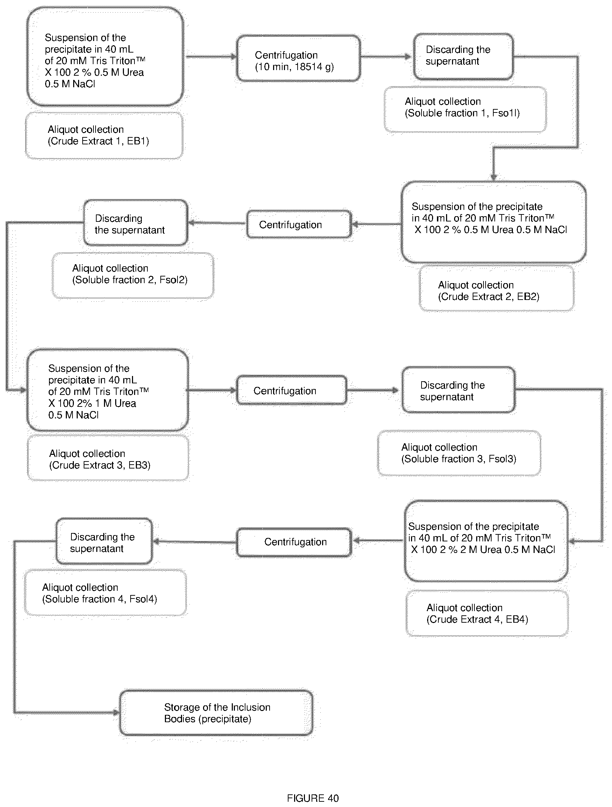

Figure 40 - Step by step for lysis, washing and obtaining inclusion bodies formed by scFv in staged production

Figure 41 - Molecular cloning: construction of the recombinant vector pET28a+scFv. P - Invitrogen® 1 kb DNA ladder standard; G.A - amplified scFv-anti-VLA-4 gene; C.N - negative control; 11 to 21 - identification of recombinant clones; C.P - positive control

Figure 42 - Evaluation of scFv expression before and after the addition of 0.5 mM IPTG, by SDS-PAGE and Western blotting. P1 - BenchMark™ Protein Ladder; P2 - Pre-stained Bio-Rad 1610305® standard; 12, 15 and 20 - clone identification; NI - total protein fraction before induction of expression; I - total protein fraction after induction of expression

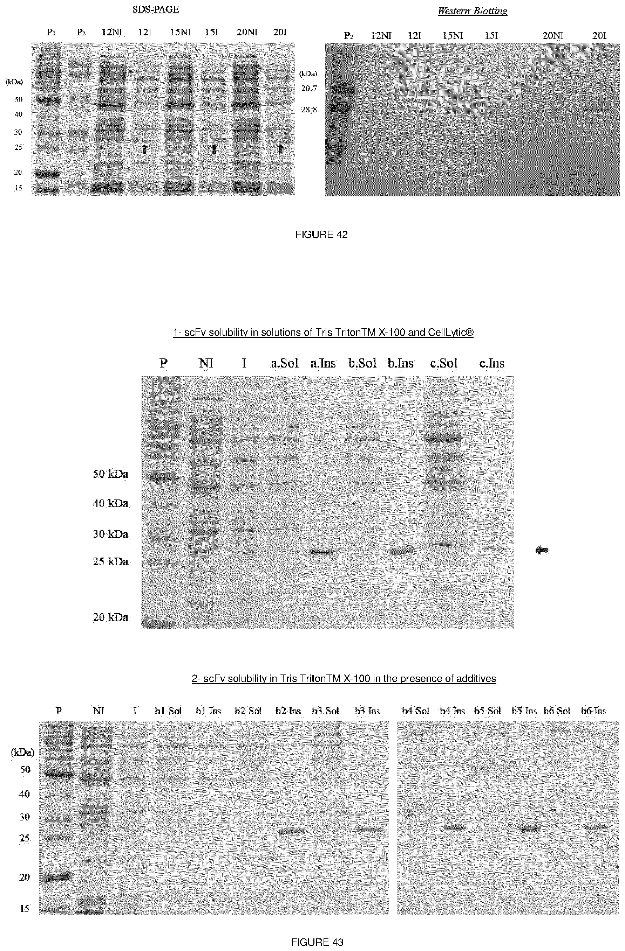

Figure 43 - Initial evaluation of the solubility of scFv in base and additive buffer solutions. P - BenchMark™ Protein Ladder; NI - total protein fraction before expression induction; I - total protein fraction after expression induction; a. - Tris 20 mM Triton™ X-100 0.1 %; b. - Tris 20 mM Triton X-100™ 0.5 %; c. Cell Lytic®; Sol - Soluble fraction of protein in the indicated solution; Ins - Insoluble fraction of protein in the indicated solution; b.1 - Tris 20 mM Triton™ X-100 0.5 % NaCl 150 mM; b.2 - Tris 20 mM Triton™ X-100 0.5 % NaCl 300 mM; b3. - Tris 20 mM Triton™ X-100 0.5 % L-arginine 0.2 M; b4. - Tris 20 mM Triton™ X-100 0.5 % L-arginine 0.4 M; b5. Tris 20 mM Triton™ X-100 0.5 % Glycerol 10 %; b6. 20 mM Tris Triton™ X-100 0.5 % $MgCl_2$

Figure 44 - Evaluation of scFv solubility after expression at reduced temperatures (30, 23 and 16°C). P - BenchMark™ Protein Ladder; NI - total protein fraction before expression induction; I - total protein fraction after expression induction; stb - 20 mM Tris base buffer solution Triton™ X-100 0.1 %; sta - Tris 20 mM Triton™ X-100 0.5 % L-arginine 0.4 M NaCl 50 mM additive buffer solution; EB - Total protein fraction in the indicated solution; Sol - Soluble protein fraction in the indicated solution; Ins - Insoluble protein fraction in the indicated solution

Figure 45 - Evaluation of the solubility of scFv in solutions containing 1, 4 and 8 M urea. P - BenchMark™ Protein Ladder; i - 20 mM Tris Triton™ X 100 0.5 % 1 M Urea; ii - Tris 20 mM Triton™ X 100 0.5 % 4 M Urea; iii - Tris 20 mM Triton™ X 100 0.5 % 8 M Urea; EB - Total protein fraction in the indicated solution; Sol - Soluble protein fraction in the indicated solution; Ins - Insoluble protein fraction in the indicated solution

Figure 46 - Expression of scFv under different conditions. Left, SDS-PAGE gels demonstrating the profile of proteins produced during expression, before and after the addition of IPTG. The purple arrows point to protein band corresponding to scFv. On the right, graph with the percentage of scFv relative to each SDS-PAGE gel shown. The percentage referring to TB, 30 °C in bioreactor corresponds to the average of three different experiments. The error bar for this condition refers to the calculated standard deviation

Figure 47 - Glucose concentration and bacterial biomass during scFv expression processes in single batch (left) or glucose-fed (right). In the graph, the glucose concentration in the inoculum (g/L) is shown on the y-axis and the biomass (g/L) is shown on the secondary axis versus time on the x-axis. Growth was performed for 2 h and induction of expression for 4 h in the expression process at 30 °C, medium TB, in a FOGALE BIOPOD F800 micro bioreactor

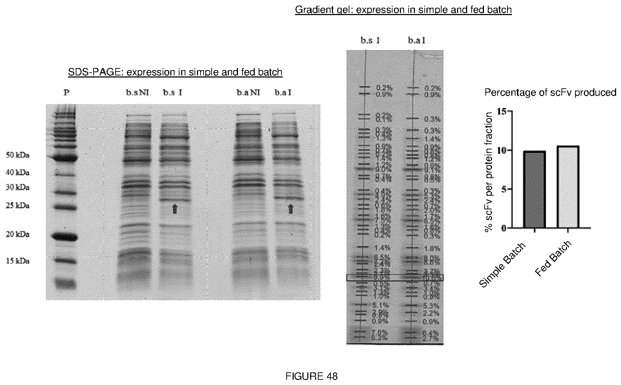

Figure 48 - Expression of scFv in single and glucose-fed batch. Left, SDS-PAGE gel demonstrating the uninduced and induced fractions from each process. P - Invitrogen® Bench Marker Protein ladder molecular weight standard; s.b. - simple batch; f.b. - fed-batch; NI - total protein fraction before induction of expression; I - total protein fraction after induction of expression. In the middle, gradient gel image with the protein fractions after induction of expression for single (left) and fed (right) batch. The numerical percentage markings were generated by the Quantity One program. Left, graphical representation of the percentages of scFv expressed in both processes

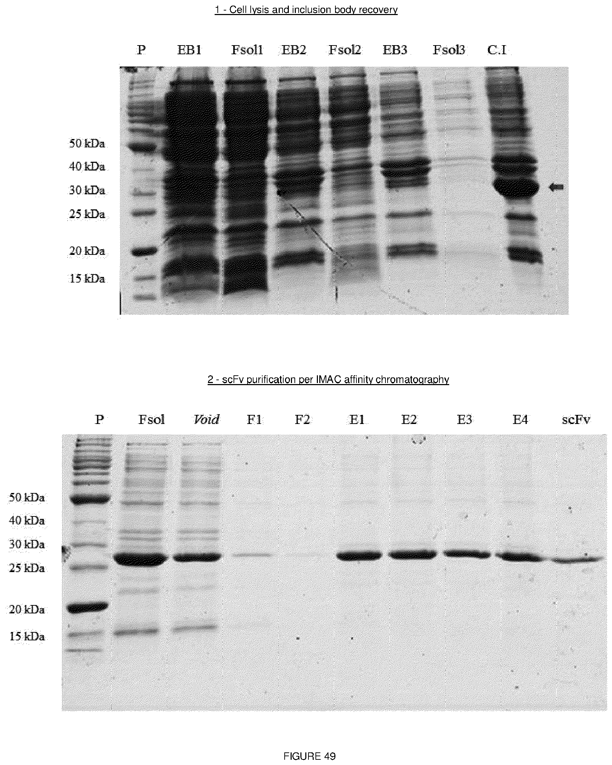

Figure 49 - ScFv purification steps. P - BenchMark™ Protein Ladder; EB1, Fsol1, EB2, Fsol2, EB3, Fsol3 - total and soluble protein fractions according to the urea wash step, being 1 and 2 washes with Tris 20 mM Triton™ X 100 2 % NaCl 0.5 M and urea 0.5 M, and 3 the wash with Tris 20 mM Triton™ X 100 2 % NaCl 0.5 M urea 1 M; C.I - inclusion bodies; Fsol - protein fraction soluble in Tris 50 mM NaCl 0.5 mM urea 8 M imidazole 1 mM, applied to the IMAC column for purification; Void - protein fraction that did not bind the nickel resin; F1 - protein fractions eluted with Tris 50 mM NaCl 0.5 mM urea 8 M imidazole 1 mM; F2 - protein fractions eluted with Tris 50 mM NaCl 0.5 mM urea 8 M imidazole 5 mM; E1 to E4 - recombinant protein fractions eluted with Tris 50 mM NaCl 0.5 mM urea 8 M imidazole 1 M; scFv - recombinant protein after renaturation

Figure 50 - Purity plot of scFv throughout the purification steps. The results are shown as the average of two independent experiments. The error bars represent the calculated standard deviation of the values obtained in the experiments. $P < 0.05$ (one-way ANOVA)

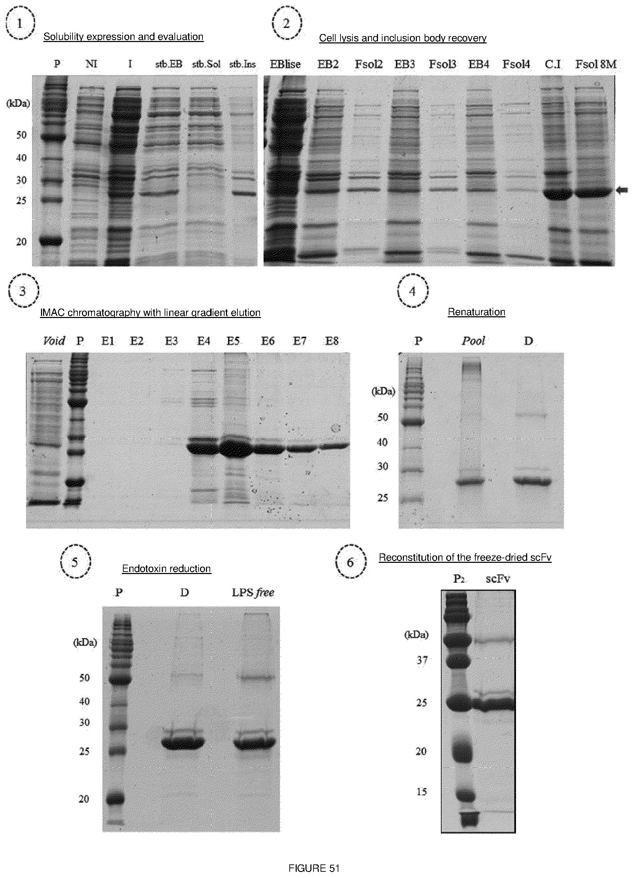

Figure 51 - Stages of scFv production increase (1 to 6). P - BenchMark™ Protein Ladder; NI - total protein fraction before expression induction; I - total protein fraction after expression induction; stb - Tris 20 mM Triton™ X-100 0.1 % base buffer solution; EB - total protein fraction in the given solution; Sol - soluble protein fraction in the given solution; Ins - insoluble protein fraction in the given solution. EB2, Fsol2, EB3, Fsol3, EB4, Fsol4 - total and soluble protein fractions according to the urea wash step, 2 being the wash with 20 mM Tris Triton™ X 100 2 % NaCl 0.5 M urea, 3 the wash with 1 M urea and 4 with 2 M urea; C.I - inclusion bodies; Fsol 8M - fraction of proteins soluble in Tris 50 mM NaCl 0.5 mM urea 8 M, applied to the IMAC column for purification; Void - fraction of proteins that did not bind the nickel resin; E1 to E4 - recombinant protein fractions eluted with Tris 50 mM NaCl 0.5 mM urea 8 M and 1 M imidazole gradient; Pool - pooling of scFv fractions after elution; D - sample after dialysis; LPS free - sample after endotoxin reduction; scFv - recombinant protein reconstituted after lyophilization; P2 - Precision Plus Protein™ Dual Color Standards

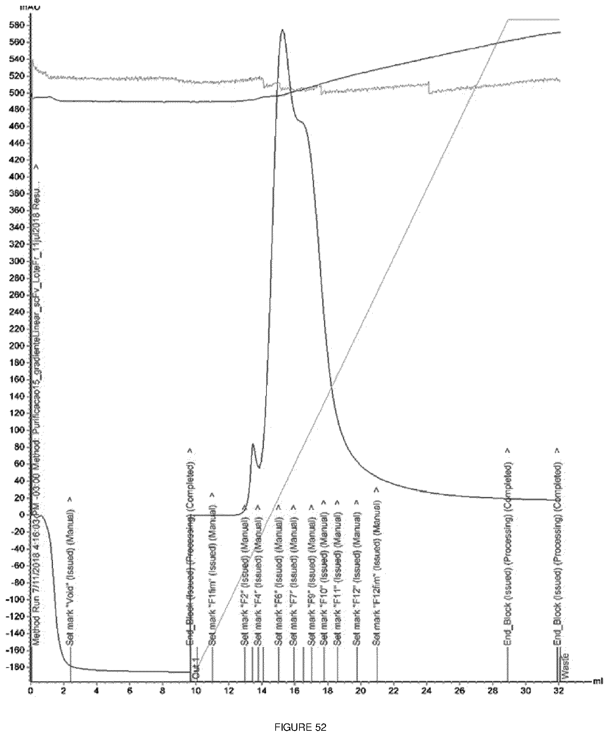

Figure 52 - Chromatogram of scFv purification in IMAC with linear gradient of imidazole. The y-axis represents the absorbance in mAU (1 = 280 nm) and the x-axis indicates the volume of liquid that is fed into the system. The blue line represents the absorbance, so the peak formed corresponds to the elution of scFv and the collection of fractions. The green line represents the imidazole gradient, from 0 to 1 M. The orange line represents system pressure, and the brown line represents conductivity. The markings in red below the blue line the collections of the 1 mL fractions corresponding to the scFv during the elution process. The graph is representative of one experiment, out of a total of three, and was automatically generated by the UNICORN® program

Figure 53 - Number of Jurkat cells adhered on surfaces with increasing concentrations of scFv. Each point corresponds to the number of cells observed in a 1 mm2 area coated with the respective scFv concentration. The experiments are shown as mean and standard deviation. Each experiment was performed in triplicate. N = 3. P < 0.05 (one-way ANOVA)

Figure 54 - ELISA for recognition of VLA-4 by scFv. The graph is plotted as the mean and standard deviation of the values for one experiment (n=1), representative of a total of 3 experiments, carried out in technical triplicate. P < 0.05 (one-way ANOVA)

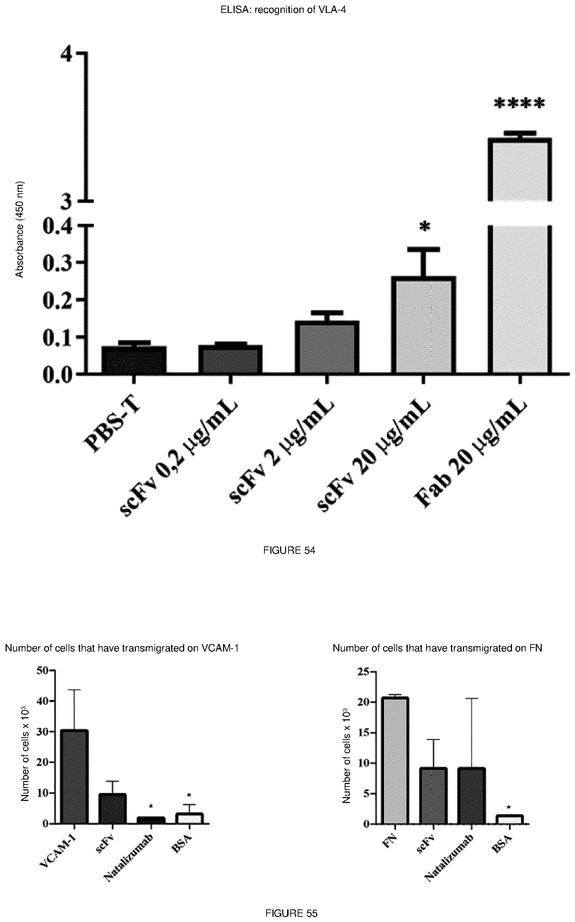

Figure 55 - Reduced transmigration of Jurkat cells on VLA-4 ligands. On the left, the graph of the transmigration experiment using VCAM-1, and BSA as a negative control of transmigration is shown. The results are presented as mean and standard deviation for three experiments (n = 3), with the exception of scFv, whose graph represents two experiments (n = 2). On the right is the graph of the transmigration experiment using fibronectin and BSA. The results are presented as mean and standard deviation for three experiments (n =3), with the exception of Natalizumab, whose graph represents two experiments (n =2). P < 0.05 (one-way ANOVA)

Figure 56 - Adhesion frequency of memory T lymphocytes on VCAM-1, treated with different concentrations of scFv, under different flow conditions (0.13; 0.06 and 0.03 dyn/cm$^2$). The y-axis of each graph shows the calculated adhesion frequency in $\mu$m-1. Each point shown in the graphs represents the calculated frequency in an experiment. The results are shown as mean and standard deviation. N = 5 experiments in general, except for the 0.2 $\mu$g/mL scFv condition, as n = 3 experiments. P < 0.05 (one-way ANOVA)

Figure 57 - Migration profile of memory T lymphocytes on VCAM-1, treated with different concentrations of scFv under 1 dyn/cm$^2$ flow rate for 10 minutes. Each point shown in the graphs represents the values of an experiment. The results are shown as mean and standard deviation. N = 5 experiments in general, except for the 0.2 $\mu$g/mL scFv condition, as n = 3 experiments. P < 0.05 (one-way ANOVA)

Figure 58 - Trajectories of memory T lymphocytes on VCAM-1, treated or not with 20 $\mu$g/mL of scFv, under 1 dyn/cm$^2$ flow rate. Each line represents the trajectory of a cell. The x-axis of each graph represents time in seconds. The y-axis represents the distance traveled $\mu$m/min. Each graph is representative of a single experiment, where n=3 per condition (untreated or treated with scFv)

Figure 59 - Adhesion frequency of memory T lymphocytes on FN, treated with scFv 2 $\mu$g/mL and Fab, under different flow conditions (0.13; 0.06 and 0.03 dyn/cm$^2$). The y-axis of each graph shows the calculated adhesion frequency in $\mu$m-1. Each point shown in the graphs represents the calculated frequency in an experiment. The results are shown as mean and standard deviation. N = 5 experiments overall, except for the Fab treatment condition, as n = 3 experiments. P < 0.05 (one-way ANOVA)

Figure 60 - Migration profile of memory T lymphocytes on FN, treated scFv 2 $\mu$g/mL and Fab under 1 dyn/cm$^2$ flow for 10 minutes. Each point shown in the graphs represents the values of an experiment. The results are shown as mean and standard deviation. N = 5 experiments overall, except for the Fab treatment condition, as n = 3 experiments. P < 0.05 (one-way ANOVA)

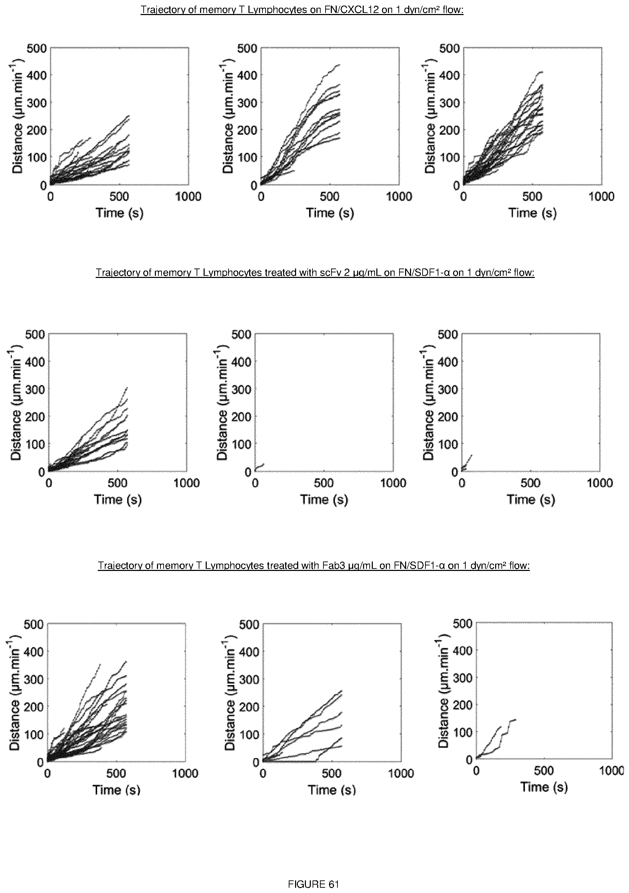

Figure 61 - Trajectories of memory T lymphocytes on FN, treated with scFv and Fab, under 1 dyn/cm$^2$ flow rate. Each line represents the trajectory of a cell. The x-axis of each graph represents time in seconds. The y-axis represents the distance traveled $\mu$m/min. Each graph is representative of a single experiment, where n=3 per condition (untreated or treated with scFv or Fab)

Figure 62 - Visualization of CD8+ T lymphocytes treated or not treated with scFv 2 $\mu$g/mL. In green is represented the labeling of the $\alpha$4 subunit of integrins, in red we observe the actin macromolecules and in pink points of phosphorylated tyrosine. The cells shown are independent and photographed from one experiment (n=1). 63X magnification

Figure 63 - Visualization of actin distribution of CD8+ T lymphocytes treated or not treated with scFv 2 $\mu$g/mL. The cells shown are independent and photographed from one experiment (n=1). 63X magnification

Figure 64 - Quantification of actin foci present in CD8+ T lymphocytes treated or not treated with scFv 2 $\mu$g/mL. Each point represents one cell, for a total of 40 cells per condition from a single experiment (n=1). The results are shown as mean and standard deviation of the quantified actin foci for each condition. P < 0.05 (T-student)

Figure 65 - Visualization of tyrosine phosphorylated foci of CD8+ T lymphocytes treated or not treated with scFv 2 $\mu$g/mL. The cells shown are independent and photographed from one experiment (n=1). 63X magnification

Figure 66 - Quantification of phosphorylated tyrosine foci present in CD8+ T lymphocytes treated or not treated with scFv 2 $\mu$g/mL. Each point represents one cell, for a total of 40 cells per condition from a single experiment (n=1).

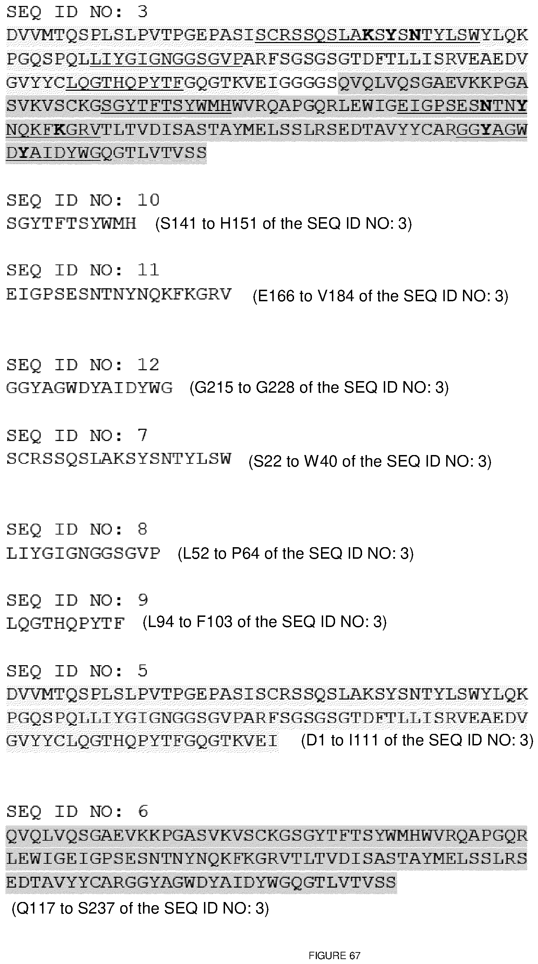

The results are shown as mean and standard deviation of the quantified foci for each condition. P < 0.05 (T-student) Figure 67 - Detailed explanation of how the sequences SEQ ID NO: 3 and 5 to 12 are related. In SEQ ID NO: 3, the yellow region corresponds to SEQ ID NO: 5 (D1 to I111 of SEQ ID NO: 3) and the blue region corresponds to SEQ ID NO: 6 (Q117 to S237 of SEQ ID NO: 3). In SEQ ID NO: 3, the underlined yellow regions correspond to SEQ ID NO: 7, 8 and 9 respectively; and the blue underlined regions correspond to SEQ ID NO: 10, 11 and 12, respectively. The bold amino acids in SEQ ID NO: 3 are the main interaction residues: K31, Y33, N35, N173, Y176, K181, Y217 and Y222.

## DETAILED DESCRIPTION OF THE INVENTION

While the present invention may be susceptible to different embodiments, a preferred embodiment is shown in the drawings and the following detailed discussion with the understanding that the present description is to be considered an exemplification of the principles of the invention and is not intended to limit the present invention to what has been illustrated and described herein.

### scFv-like protein

In a first embodiment, the present invention relates to a scFv-like protein comprising a first polypeptide chain and a second polypeptide chain joined by a ligand, presenting the formula as follows:

(VH domain) - (ligand) - (VL domain), where the VH domain comprises at least amino acids N173, Y176, K181, Y217, Y222 from SEQ ID NO: 3 and the VL domain comprises at least amino acids K31, Y33, N35 from SEQ ID NO: 3. In one embodiment, domain VH comprises the complementarity-determining regions (CDR1, CDR2, CDR3) consisting of SEQ ID NO: 10, SEQ ID NO: 11 and SEQ ID NO: 12 and domain VL comprises the complementarity-determining regions (CDR1, CDR2, CDR3) consisting of SEQ ID NO: 7, SEQ ID NO: 8 and SEQ ID NO: 9. In another embodiment, the VH domain comprises amino acids Q117 to S237 of SEQ ID NO: 3 and the VL domain comprises amino acids D1 to I111 of SEQ ID NO: 3. In another embodiment, the ligand is 5 to 15 amino acids in length. In another embodiment, the binding peptide comprises at least the sequence GGGGS. In another embodiment, the binder can be GGGGSGGGGS or GGGGSGGGGSGGGGS. In another embodiment, the protein selectively binds to integrin $\alpha 4\beta 1$. In another embodiment, the protein is for use in a prognostic method or for the treatment of chronic inflammatory diseases.

In an alternative form, the protein may be for use in a method for treating multiple sclerosis, asthma, rheumatoid arthritis, stroke, and inflammatory bowel disease (IBD), such as Crohn's disease, Duchenne Muscular Dystrophy (DMD), Dry eye disease, uveitis, or allergic conjunctivitis.

### Polynucleotide

In a second embodiment, the invention relates to a polynucleotide comprising the nucleotide sequence shown in SEQ ID NO: 1.

SEQ ID NO: 1 is the nucleotide sequence. SEQ ID NO: 2 is the nucleotide sequence optimized for expression in *E. coli.* And SEQ ID NO: 4 is the protein sequence with a histidine tail.

### Vector

In a third embodiment, the invention relates to a vector comprising the polynucleotide as previously defined. In yet another embodiment, the vector consists of a pET28a cloned with the scFv sequence (SEQ ID NO: 1) at the NcoI and XhoI restriction sites.

### Host cell

In a fourth embodiment, the invention relates to a host cell comprising the vector as previously defined. In another embodiment, the host cell is a bacterial cell or mammalian cell. In a preferred embodiment, the host cell is a bacterial cell. In yet another embodiment, the bacterial cell is an *E. coli* cell.

### Composition

In a fifth embodiment, the invention relates to a composition, comprising the aforementioned protein and a pharmaceutically acceptable excipient. In another embodiment, the composition is for use in the treatment or prognosis of chronic inflammatory diseases.

In an alternative form, the composition may be for use in the treatment of multiple sclerosis, asthma, rheumatoid arthritis, stroke, and inflammatory bowel disease (IBD), such as Crohn's disease, Duchenne Muscular Dystrophy (DMD), Dry eye disease, uveitis, or allergic conjunctivitis.

In certain forms of realization, excipients may include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, talc; or even L-arginine.

Method for treating a disease or condition

In a sixth embodiment, the invention relates to a method for treating a disease or condition that results directly or indirectly from $\alpha 4\beta 1$ integrin activity, comprising administering to a human being a protein or a composition as previously defined. In another embodiment, the disease or condition is multiple sclerosis.

In an alternative embodiment, the disease or condition is Asthma, Rheumatoid Arthritis, Stroke, Inflammatory Bowel Disease (IBD), such as Crohn's disease, Duchenne Muscular Dystrophy (DMD), Dry eye disease, uveitis, or allergic conjunctivitis.

**Method *in vitro* to prognosticate a chronic inflammatory disease**

In a seventh embodiment, the invention relates to an *in vitro* method for prognosticating chronic inflammatory diseases comprising: contacting at least one protein as previously defined or a composition as previously defined with a cell, tissue or sample from an individual, detecting binding of the protein to the cell, tissue or sample, quantifying VLA-4 expression, and indicating a most suitable treatment for the patient.

In an alternative form, the drug can be for the treatment of multiple sclerosis, asthma, rheumatoid arthritis, stroke, and inflammatory bowel disease (IBD), such as Crohn's disease, Duchenne Muscular Dystrophy (DMD), Dry eye disease, uveitis, or allergic conjunctivitis.

In an alternative embodiment, the sample can be blood or serum. The tissue can also be cerebrospinal fluid (CSF).

**Use of protein**

In an eighth embodiment, the invention relates to the use of the previously defined protein or composition in the preparation of a drug for the treatment of chronic inflammatory diseases.

In an alternative form, the drug can be for the treatment of multiple sclerosis, asthma, rheumatoid arthritis, stroke, and inflammatory bowel disease (IBD), such as Crohn's disease, Duchenne Muscular Dystrophy (DMD), Dry eye disease, uveitis, or allergic conjunctivitis.

EXAMPLES

1. Prospecting

The Integrity database was used, to obtain antibody sequences that recognize integrins composed of the $\alpha 4$ or $\beta 1$ subunits.

2. Comparative modeling

From the sequences obtained, a local alignment was performed using BLAST (Basic Local Alignment Search Tool) (ALTSCHUL et al., 1990) against the PDB database to choose and select the template proteins. The selection of the templates was based on the following parameters: identity of the template sequences with the antibody light and heavy chain sequences, coverage of these sequences, the E-value and the resolution of the template structures. The identity, coverage and E-value were reported by BLAST as a result of the alignment. The resolution of the structures was reported by the PDB.

In possession of the template proteins, a global alignment of all the proteins to be modeled with their respective reference sequences was performed. The alignments were done through the EMBOSS Needle server.

The Modeller program was used to build the model. This program uses the method of satisfying spatial constraints to construct the tertiary structure of proteins. For each chain modeled, 200 structures were made.

3. Evaluation and refinement of the modeled structures

For the light and heavy chains of each antibody, there were a series of 200 structures generated. The choice of the best structure within the series was made based on Modeller's objective function, so that the model with the lowest objective function value would be the best model among the 200. Therefore, for each chain, one model out of the 200 generated was selected.

The selected models were submitted to the MolProbity (HINTZE et al., 2016) and Verify 3D (LÜTHY; BOWIE; EISENBERG, 1992) servers for evaluation. After the preliminary evaluation provided by the servers, the structures were refined by correcting for bad rotamers and unfavorable phi and psi angle conformations according to the e Ramachandran graph. This refinement was done using the program Coot (EMSLEY et al., 2010). After the adjustments, the structures were resubmitted to the servers for validation.

4. Obtaining the scFvs

In possession of the light and heavy chain structures, two scFv shapes were constructed for each antibody. The first format links the light and heavy chains via a short GGGGS-like peptide ligand. The second format uses a long peptide ligand of sequence GGGGSGGGGGGGSGGGGS. The insertion of the peptide ligand was done with the aid of the program Modeller. The use of different sizes of binding peptides aims to obtain the best scFv, since its size interferes with the performance of the scFv.

5. Mapping of CDRs and active waste

The CDRs of each antibody were defined by comparison with other antibodies of mapped CDRs, with the aid of the PyMol program (PyMOL Molecular Graphics System, Version 1.8 Schrödinger, LLC).

The GetArea server (HAYRYAN et al., 2005) was used for elucidation of the exposed residues of each scFv. The waste exposed in the CDR regions was then defined as active waste.

6. Molecular docking with integrin $\alpha 4 \beta 1$

Docking of each scFv with the $\alpha 4 \beta 1$ integrin was performed using the Haddock server. Haddock (High Ambiguity Driven protein-protein *Docking*) is a server that performs protein-protein and protein-DNA/RNA docking, using Monte Carlo algorithm for orientation and docking of the molecules involved and force-field based score function. Haddock uses three main steps to perform docking: 1 - orientation randomization and energy minimization of the rigid bodies, also referred to as rigid body docking; 2 - simulated semi-rigid annealing in space angle twists, also referred to as semiflexible refinement; 3 - final refinement in Cartesian space with explicit solvent, also referred to as water refinement (DOMINGUEZ; BOELENS; BONVIN, 2003) (VAN DIJK, 2006).

The best integrin-scFv complexes provided by Haddock were selected according to the Haddock score, RMSD and cluster size parameters. The Haddock score represents a weighted sum of intermolecular energies, such as electrostatic, Van de Waals, and desolvation (VAN DIJK, 2006). The more negative the Haddock score, the more energetically favorable is the interaction between the two molecules, and, in a way, the higher the affinity, because of the thermodynamic favoritism. Cluster size indicates numerically how preferable that interaction position between the proteins was, so the higher this value, the better. The RMSD, Root Mean Square Deviation, represents the deviation of the average distance between the atoms, and it is recommended that its value should not exceed 2 Å (VAN DIJK, 2006). The closer this value is to zero, the better and more reliable the docking result will be.

7. Searching for hotspots from the best complexes

The best complexes resulting from docking - one complex for each scFv-integrin assembly - , according to the mentioned parameters, were submitted to hotspot analysis and search, via the Robetta Alanine Scanning server. This analysis allows us to observe the key residues in protein stability and interaction, so that the removal or replacement of one of these hotspot residues causes a change in the free energy of the system of at least 1kcal/mol (KORTEMME, 2004).

8. Interaction analysis of the complexes and identification of the interacting hotspots

The complexes were submitted to the PDBePISA Interfaces server (KRISSINEL; HENRICK, 2007), which reports all hydrogen bonds and salt bridges made between integrin residues and scFv. The data provided by PDBePISA were qualitatively compared with the results of Robetta Alanine Scanning, resulting in the identification of residues crucial for the interaction between both proteins, the so-called interactive hotspots.

9. Targeted mutagenesis of scFvs

Using the Coot program, mutations were made in amino acid residues near the interacting hotspots. Modifications were made to the residues around these hotspots to make this region of scFv an interaction hotspot, i.e., a region where multiple interactions between scFv and integrin are performed.

10. Docking of modified scFvs against integrin $\alpha4\beta1$

New docking was performed with each modified scFv and the $\alpha4\beta1$ integrin in order to assess whether the modifications were effective in improving the interaction with $\alpha4\beta1$. The results of the native and modified scFvs were compared.

11. Docking *of* scFvs against integrin $\alpha5\beta1$ and $\alpha4\beta7$

In order to verify the specificity of the scFvs, dockings of each scFv, native and modified, were made with the $\alpha5\beta1$ integrin. The best scFv among all was chosen, that is, the one that showed satisfactory Haddock Score and Cluster size values - based on reliable RMSD values - for $\alpha4\beta1$ and, according to the same parameters, showed no affinity with $\alpha5\beta1$. With this native and modified scFv, new docking with the $\alpha4\beta7$ integrin was performed.

12. Increase specificity of the best scFv and repeat docking

To make scFv specific for $\alpha4\beta1$ against $\alpha4\beta7$, two new sets of mutations were carried out, aimed at weakening the interaction of scFv with the $\alpha4\beta7$ integrin. The residues chosen to be modified were those that were important for the scFv-$\alpha4\beta7$ interaction but did not interact with $\alpha4\beta1$ residues. Unlike the first series of mutations, we have tried to eliminate interactions by modifying residues. Thus, the affinity of scFv for $\alpha4\beta7$ was reduced.

13. Molecular Dynamics

Three scFvs were chosen to undergo DM simulation: the short peptide-binding native scFv 257898, called native scFv, the best scFv with two sets of mutations, called scFv B, and the same scFv with three sets of mutations, called scFv X. Nine dynamics simulations were performed with the following complexes: 1 - native scFv with integrin $\alpha4\beta1$, 2 - native scFv with integrin $\alpha5\beta1$, 3 - native scFv with integrin $\alpha4\beta7$, 4 - scFv B with integrin $\alpha4\beta1$, 5 - scFv B with integrin $\alpha5\beta1$, 6 - scFv B with integrin $\alpha4\beta7$, 7 - scFv X with integrin $\alpha4\beta1$, 8 - scFv X with integrin $\alpha5\beta1$, and 9 - scFv X with integrin $\alpha4\beta7$. The same protocol was applied for the six simulations and the program used was the Gromacs 5.1 package.

The program pdb2gmx was used to generate the topologies, with the gromos9653a6 force field. A cubic box with a cut-off radius of 1nm was inserted for the system boundary, via the editconf option. The system was solvated by adding water molecule by gmx solvate and neutralized with the help of gmx grompp and gmx genion.

Two minimizations were done following the steepest descent algorithm with the first being a vacuum minimization and the second with the solvent. Prior to the production dynamics, two constraint dynamics and thermalization were required to ensure that the system was balanced. The first constraint dynamics used NVT-type ensemble, and the second used NPT, with the aid of the Parrinello-Rahman barostat. Finally, 10 nanosecond thermalization dynamics were performed in the NPT condition. For pressure control in all these stages, v-rescale thermostat was used.

The production DM was done at 200 nanoseconds, following the same conditions. All these stages of constraint DM, thermalization and production used Verlet's algorithm.

14. Analysis of data from DM simulations

For all six simulations, eight parameters were evaluated: RMSD, RMSF, spin radius, hydrogen bonds, salt bridges, total energy, Coulomb potential, and Lennard-Jones potential.

15. Cloning of the scFv-anti-VLA-4 gene in plasmid pET28a

Obtaining the synthetic gene and oligonucleotides for amplification of the scFv-anti-VLA-4 gene

The scFv-anti-VLA-4 gene sequence was previously developed through computational methodologies such as modeling, docking and molecular dynamics, and directed site mutagenesis (Chaves, 2016). Synthesis of the scFv anti-VLA-4 gene and insertion of it into a pUCIDT plasmid vector (pUCIDT+scFv) were performed by the company IDT (Integrated DNA Technology).

Oligonucleotides complementary to the 5'-3' (sense) and 3'-5' (antisense) ends of the gene were synthesized by the same company and had restriction sites for the enzymes NcoI (CCATGG) and XhoI (GAGCTC), respectively, in order to allow the construction of the recombinant vector. The synthesized oligonucleotides were resuspended in a volume in μL of TE buffer (10 mM Tris-HCl pH 8 EDTA 0.1 mM, Thermo Fisher Scientific) corresponding to the numerical value of their molar masses, getting to a final concentration of 1nmol/μL. Working solutions at 10 μM were prepared by diluting the stock 1: 100 in ultrapure water.

16. Amplification of the scFv-anti-VLA-4 gene and insertion of restriction sites at its ends

Polymerase Chain Reaction (PCR) was used to amplify the gene from the synthetic plasmid and insert the restriction sites. Amplification was performed with the enzyme Platinum® Taq DNA polymerase High Fidelity (Life Technologies), in 25 μL reactions, following the manufacturer's recommendations, from 8 ng of template DNA.

The reactions were performed in a Veriti Thermal Cycler (Applied Biosystems), with the following cycling: 94 °C for 2 minutes, followed by 35 cycles of 94 °C for 30 seconds, 56 °C for 30 seconds and 68 °C for 1 minute plus a final extension at 68 °C for 5 minutes.

17. Visualization, purification and quantification of the scFv-anti-VLA-4 gene after amplification

The products of the amplification reaction were visualized by the technique of horizontal electrophoresis in 1% (w/v) agarose gel. A volume of 3 μL of the PCR products were diluted in sample buffer (bromophenol blue) and applied individually to the agarose gel. Electrophoresis was performed at 90 V for approximately 30 minutes. The gel was subjected to staining with GelRed™ solution (Biotium), following the manufacturer's recommendations and visualized on the Lpix Image photo-documenter (Loccus) under UV (ultraviolet) light.

Purification of the amplified scFv-anti-VLA-4 gene was done using the Wizard SV gel and PCR clean Up System kit (Promega), according to the instructions described in the manufacturer's manual. After purification, quantification of the material was performed, using the Molecular probes Qubit dsDNA HS Assay kit, 500 assays (Life Technologies), according to the manufacturer's instructions.

18. Construction of the recombinant vector pET 28a+scFv

The plasmid used for cloning and expression of the scFv-anti-VLA-4 gene was the expression vector pET 28a (pET Expression System 28, Merck, Darmstadt, Germany). Initially, approximately 500 ng of the insert, amplified scFv gene, and 630 ng of the vector, pET 28a, were subjected to the first digestion, with the endonuclease XhoI (Life Technologies) for 90 minutes at 37°C, according to the manufacturer's recommendations. After the first digestion, the products were purified and quantified, according to the methodology mentioned in section 3.1.3. Then, the plasmid and gene digested with XhoI were subjected to the enzymatic reaction with the endonuclease NcoI (Life Technologies), also for 90 minutes at 37°C. Similarly, the digestion products were subjected to purification and quantification.

The vector and insert binding reaction was performed using T4 DNA ligase (Life Technologies), at an insert: vector molar ratio of 3:1 starting at 60 ng vector. The following calculation was used to obtain the precise amounts of DNA needed for the reaction:

$$\text{ng (insert)} = \frac{\dfrac{(\text{ng (vector)} \times \text{Kb (insert)})}{\text{Kb (vector)}}}{\text{proportion insert: vector} \left(\dfrac{3}{1}\right)}$$

The reagents were added according to the manufacturer's instructions and the reaction was incubated for 16 h at 4°C.

Cleavage and binding of the vector and insert in the regions corresponding to the NcoI and XhoI restriction sites is strategic for scFv expression. This form of insertion allows the scFv gene to be in a position after the T7 promoter region and before the T7 terminator region, in the 5'-3' direction. Thus, this protein can be expressed by bacteria that contain the polymerase that recognizes the T7 promoter and terminator. Furthermore, this insertion allowed scFv to be expressed fused to a six histidine sequence in its C-terminal portion. This histidine tail is important for the expression evaluation and scFv purification steps.

19. Transformation of *Escherichia coli* DH5α cells with recombinant plasmid pET28a+scFv

Electrocompetent *E.coli* DH5α cells (http://mcb.berkeley.edu/labs/krantz/protocols/electrocomp_cells.pdf) were used for transformation of the binding reaction. To do so, 3 μL of the binding reaction was added to 50 μL of cells, this content was transferred to an appropriate cu-β and taken to the Micropulser™ electroporator (BioRad). After the electrical pulse (Eco02 setting), 450 μL of SOC (Super Optimal broth with Catabolite repression, Thermo Fisher Scientific) culture medium was added to the cuvette, and the contents were carefully and quickly transferred into a 15 mL Falcon® tube. The transformed cells were then incubated at 37°C, in SOC medium without the presence of antibiotic, at a shaking rate of 200 rpm (Revolutions Per Minute) for a period of 1 hour.

After the incubation period, 100 and 50 μL of the cells were individually spread on surfaces of LB-agar medium (Luria Bertani) supplemented with 50 μg/mL kanamycin. The plates were then incubated at 37°C for a period of 16 h.

20. Selection of *E. coli* DH5-α clones containing the pET28a+scFv vector

To select a recombinant clone, ten colonies were randomly selected and inoculated into 0.2 mL microtubes containing 15 μL of ultrapure water. The microtubules were homogenized and 3 μL of this inoculum was transferred to 1.5 mL microtubules containing 300 μL of LB medium supplemented with kanamycin. The microtubes were identified according to the colony of origin, numbered 11 to 20, and then incubated at 37°C for approximately 24 h without shaking.

The in-water inocula were thermally lysed in a thermocycler at 95°C for 5 minutes. After this period, the microtubes were centrifuged at 12000 rpm (13523 x g) for 5 minutes in an Eppendorf 5424R centrifuge. The supernatant was then transferred to another microtube and used in PCR to identify the recombinant clones.

To perform the PCR we used sense and antisense oligonucleotides respectively complementary to the T7 promoter and terminator regions, present in the plasmid pET28a. For this procedure a positive control, the intact pET28a vector, and water as a negative control were used. The reaction was performed according to item 3.1.2. The products of these reactions were subjected to agarose gel electrophoresis, as described in section 3.1.3, and identification of the recombinant clones was done by visualizing the sizes of the DNA segments.

The inocula on LB medium from the colonies identified as recombinant by PCR were then selected for extraction of the recombinant plasmid. Twenty microliters of these inocula were transferred to 10 mL of 50 μg/mL kanamycin LB medium in 50 mL Falcon® tubes. The tubes were identified according to the colony of origin and incubated for 16 h, 37°C, with 200 rpm rotation.

After the incubation period, glycerol stocks of 25% of the clones were prepared and stored at -80°C. The rest of the cultures were submitted to purification of the recombinant plasmids using the High Pure Plasmid Isolation Kit (Roche), according to the instructions in the manufacturer's manual. After purification the vectors were quantified according to the methodology described in 3.1.3 and stored at -20°C.

21. Evaluation of plasmid pET28a+scFv by PCR and enzymatic digestion

The extracted recombinant vectors were each subjected to four different PCRs with the following combinations of sense and antisense oligonucleotides and reaction controls:

    a. Oligonucleotides for T7 promoter and terminator regions. Plasmid pET28a intact as positive reaction control
    b. Oligonucleotides for 5' and 3' regions of the scFv-anti-VLA-4 gene, described previously. Plasmid pUCIDT+scFv intact as positive reaction control
    c. Sense oligonucleotide for T7 promoter region and antisense for 3' region of the scFv gene
    d. Oligonucleotide sense for 5' of the scFv gene and antisense for T7 terminator region.

In all conditions, ultrapure water was used as a negative control. The reactions were prepared according to 3.1.2.

In addition to performing PCRs with different oligonucleotide combinations, the recombinant vectors were subjected to digestion with XhoI enzyme, according to the methodology described in section 3.1.4. As a reaction control, intact plasmid pET28a was used.

The products of the PCRs and XhoI digestion were subjected to horizontal 1% (w/v) agarose gel electrophoresis and visualized as per item 3.1.3.

22. Sequencing of recombinant plasmids pET28a+scFv

To confirm the correct insertion and sequence integrity of the scFv-anti-VLA-4 gene in the recombinant plasmids, we first performed the sequencing reaction using BigDye™ Terminator v3.1 Cycle Sequencing Kit (Thermo Fisher Scientific), following the manufacturer's recommendations. For each recombinant clone obtained, each of the reactions below were prepared using approximately 80 ng of recombinant plasmid:

    a. Sense oligonucleotide for T7 promoter region
    b. Antisense oligonucleotide for T7 terminator region
    c. Sense oligonucleotide for the 5' region of the scFv-anti-VLA-4 gene
    d. Antisense oligonucleotide for the 3' region of the scFv-anti-VLA-4 gene

At the end of the reaction, the plate was forwarded to the precipitation step, according to the manufacturer's suggested protocol, and then taken to the ABI 3500 XL automated sequencer (Life Technologies) for sequencing. After this procedure, the data generated by the sequencer was collected and analyzed using the DNAstar program.

23. Expression of scFv in *Escherichia coli* SHuffle®

Transformation of *E. coli* SHuffle® cells with plasmid pET28a+scFv

The *E. coli* SHuffle® bacterial strain (Lobstein *et al*, 2012) was used for the expression of anti-VLA-4 scFv. To this end, 1 μL of each recombinant plasmid clone at a concentration of 5 ng/μL was added to 50 μL of electrocompetent cells. The next steps of electroporation proceeded according to the previously described methodology. After the incubation period, 20 and 40 μL of the transformed cells were seeded in LB-agar kanamycin 50 μg/mL medium and then incubated at 37°C for 16 h.

To prepare the pre-inocula required for expression, isolated colonies were inoculated in 10 mL of TB (Terrific-Browth) medium supplemented with 50 μg/mL kanamycin and 1% (w/v) glucose incubated at 37°C for 16 h under 200 rpm orbital shaking. After the incubation period, glycerol stocks of 25% of the clones were prepared and stored at -80°C. The rest of the cultures were used in a new inoculum to verify the expression of scFv.

24. Expression of scFv

The overnight-grown pre-inocula had their ODs (Optical Density) measured by reading in a U-5100 spectrophotometer (Hitachi) at a wavelength of 600 nm. For the preparation of the inocula, the volume of pre-inoculum to be added to the expression medium for an initial OD of 0.15 was calculated.

The inocula were then made in expression medium, TB kanamycin 50 μg/mL glucose 1%, to a final volume of 5 mL, with growth in 50 mL Falcon® tubes at 37°C under 200 rpm shaking. The ODs of the inocula were evaluated at 60 and 30 minute intervals, until they reached values between 0.6 and 0.8, when scFv expression was induced.

For induction of recombinant protein expression, 0.5 mM IPTG (Isopropyl-b-D-galactoside) inducer was added. After the addition of the inducer, the inocula were left at 37°C under 200 rpm orbital shaking for a period of 4 h. Before the addition of IPTG, two aliquots of 400 μL from each inoculum were collected, centrifuged (160 g, 10 min), The supernatant was discarded, and the precipitate was stored at -20°C for later evaluation.

After the expression period, the final OD of the inocula was measured. Ten aliquots of 250 μL from each inoculum were collected, centrifuged, the precipitate was kept and stored at -20°C for further analysis.

To assess cell viability from pre-inoculum to the end of expression, aliquots were taken and diluted serially from 10-5 to 10-9. These dilutions were seeded onto LB-agar kanamycin 50 μg/mL plates in a standard of three 10 μL drops per dilution. The plates were incubated at 37°C for 16 h, and after this period, the number of viable cells was calculated by averaging the number of colonies obtained divided by the lowest dilution factor at which colony formation was observed.

25. Evaluation of scFv anti-VLA-4 expression by SDS-PAGE and Western Blotting

To evaluate the expression of scFv by recombinant *E. coli* SHuffle® clones, aliquots containing inoculum precipitates before and after expression induction were suspended in sample buffer (50 mM Tris-HCl, 2% (w/v) sodium dodecyl sulfate (SDS), 0.1% (w/v) bromophenol blue, 10% (v/v) glycerol, 100 mM 2-mercaptoethanol), according to the formula below:

$$\text{Volume of sample buffer} = \frac{D.O. \times 25}{0.1 \times (1/\text{volume of aliquot in mL})}$$

After addition and homogenization of the sample buffer, the samples were subjected to boiling at 100°C for 5 minutes for evaluation of scFv expression by SDS PAGE and/or Western Blotting. To do so, 15 μL of each sample was added to two 12 % polyacrylamide gels and the run was performed at an amperage of 0.02 A per gel for approximately 60 minutes. After thorough washes in water, the first gel was fixed in a 10 % acetic acid solution and 50 % ethanol for at least 30 minutes. This gel was then briefly washed with water and submerged in GelCode™ Blue Stain Reagent (Thermo Fisher Scientific), heated in a microwave oven in three 10-second intervals and then stirred at RT for about 30 minutes. The dye was then discarded, and the gel incubated in water until the excess dye was eliminated. After this procedure it is possible to visualize the protein bands and record the gel using a Bio-Rad GS-800 densitometer.

The second gel, after migration, was forwarded for *Western Blotting.* This gel was put in contact with a nitrocellulose membrane and, using the Semi-Dry equipment (BioRad), the transfer of proteins from the gel to the membrane was performed, according to the instructions available in the manufacturer's manual. After transfer, the membrane was submerged in solution containing PBS-T 0.05% (Phosphate Buffered Saline Tween® 20 0.05%, Sigma-Aldrich) supplemented with Molico™ skim milk 4 % (w/v) for a period of 50 minutes under light orbital shaking at TA. The membrane was then washed three times with 50 mL of 0.05% PBS-T and submerged in a 0.05 % PBS-T solution containing phosphatase-conjugated anti-histidine tail antibody (6x-His Tag Monoclonal Antibody (3D5), AP, 46-0284, Invitrogen,) at 1:750 dilution for 1 hour. The membrane was washed again, and the AP conjugate substrate kit (BioRad) was used, according to the manufacturer's instructions, for the development of the scFv arranged on the membrane.

Performing SDS-PAGE electrophoresis and Western Blotting allowed visualization of scFv expression by bacteria transformed with each recombinant clone obtained in the cloning step. For the next steps, only transformed bacteria and proteins derived from one of the clones, which showed the best sequencing result, were used.

25. Evaluation of scFv solubility

Solubility of scFv in Tris Triton™ X-100 solutions

To evaluate the solubility of scFv, three aliquots obtained during scFv expression after induction with IPTG, containing the inoculum precipitate, were individually suspended with these respective solutions:

    a. 20 mM Tris Triton™ X-100 0.1 % pH 8.0
    b. 20 mM Tris Triton™ X-100 0.5 % pH 8.0
    c. Cell Lytic® B solution (Sigma-Aldrich) 100 mg/mL

The volumes of the solutions a. and b. followed the calculation demonstrated in item 3.2.3. Solution c., on the other hand, was added according to the manufacturer's instructions. Samples a. and b. were placed in a container containing ice and subjected to ultrasonication lysis using a Qsonica sonicator. For this procedure, three sonication cycles were performed, lasting 10 seconds each, at a power of 0.08 on average, with 30 second intervals between cycles. After lysis, the samples were subjected to centrifugation (Eppendorf 5424R), for 10 minutes at 14000 rpm (18407 g). The sample prepared with c. solution was also centrifuged after a 20 min incubation period in TA. following the addition of the reagent.

Aliquots of 20 μL from each sample before centrifugation, corresponding to the crude sample extract, were separated. After centrifugation, the supernatants, containing the soluble protein fraction, were collected and arranged in different microtubes. The supernatants, as well as the crude extract samples, were prepared for SDS PAGE by adding an equal volume of 2x sample buffer. The precipitates resulting from centrifugation, containing the insoluble protein fraction, were suspended in 1x sample buffer in the same initial volumes prior to lysis. All samples were then subjected to SDS PAGE electrophoresis as described in 3.2.3. The protein band corresponding to scFv was identified, and its presence in the soluble or insoluble fraction provided the indicative for the solubility character of the expression of this protein.

26. Solubility of scFv in Tris Triton™ X-100 in the presence of additives

To observe whether the presence of additives, such as salts and amino acids, was able to increase the solubility of the scFv molecule, six aliquots obtained during scFv expression after induction with IPTG, containing the inoculum precipitate, were individually resuspended with these respective solutions:

    b1. 20 mM Tris Triton™ X-100 0.5 % NaCl 150 mM
    b2. 20 mM Tris Triton™ X-100 0.5 % NaCl 300 mM
    b3. 20 mM Tris Triton™ X-100 0.5 % L-arginine 0.2 M
    b4. 20 mM Tris Triton™ X-100 0.5 % L-arginine 0.4 M
    b5. 20 mM Tris Triton™ X-100 0.5 % Glycerol 10
    b6. 20 mM Tris Triton™ X-100 0.5 % MgCl2

All samples were sonication lysed, centrifuged, and subjected to SDS-PAGE electrophoresis as described in 3.3.1.

27. Expression of scFv at 16, 23 and 30°C and evaluation of solubility

To evaluate whether reducing the expression temperature is able to increase the solubility of scFv, we performed expression of it at three different temperatures using the Fogale Biopod f800 airlift micro bioreactor. To prepare the pre-inoculum, approximately 5 μL of the selected recombinant clone stored in glycerol (item 3.2.2) were added to 50 mL of 50 μg/mL glucose 1 % TB kanamycin medium. The pre-inoculum, arranged in a sterile 200 mL Erlenmeyer flask, was incubated at 37°C under 200 rpm orbital shaking for 16 h.

Three sterile bioreactor vessels containing medium (TB kanamycin 50 μg/mL glucose 1 %) were prepared to receive the pre-inoculum according to the calculation described in 3.2.2. The volume of each inoculum was 70 mL. After the initial OD measurement, the inocula were left to grow and monitor the OD for 1.5 h at 37°C with constant 1 vvm (Air Volume/Media Volume/Minute) aeration according to the *air-lift* system of the equipment. Throughout the process carried out in the bioreactor, antifoaming automatic was added as the system identified excessive foaming induced by aeration.

After the growth period, the O.D. was measured, aliquots of 500 μL of each inoculum were collected and 0.5 mM IPTG was added. After the addition of the expression inducer, the temperatures of each inoculum were reduced to 30, 23, and 16°C. The inocula remained in the expression phase for 7.5, 11.5, and 15.5 hours, respectively. The ODs were then measured and nine aliquots of 100 μL or 200 μL were collected for further evaluation. Aliquots were centrifuged, the supernatants discarded, and the precipitates stored at -20°C for analysis. The remaining contents of each vessel, about 25 to 30 mL, were centrifuged and also stored at -20°C. Cell viability testing was performed for the pre-inoculum and the three inocula, as described in 3.2.2.

Two aliquots of each of the inocula (30, 23, and 16°C) were suspended, according to the calculation described in 3.2.3, individually and respectively in the following solutions:

- Base buffer solution: 20 mM Tris Triton™ X-100 0.5%.
- Additive buffer solution: 20 mM Tris Triton™ X-100 0.5% L-arginine 0.4 M NaCl 50 mM

Samples were lysed by sonication, centrifuged, prepared with sample buffer, and subjected to SDS-PAGE electrophoresis as described in 3.3.1, to analyze the expression profile and solubility of scFv after expression at different temperatures.

28. Evaluation of the solubility of scFv in different urea concentrations

To evaluate the solubility of scFv in urea, three aliquots containing precipitates corresponding to the inoculum produced in a Fogale micro bioreactor at 30°C were used. The aliquots were resuspended and lysed by sonication in a base buffer solution (section 3.3.3), according to item 3.2.3. Samples of 20 μL of this content were obtained and centrifuged, the supernatants were discarded, and the precipitates were resuspended, individually in 100 μL of the following solutions:

i. Tris 20 mM Triton™ X 100 0.5 % 1 M Urea
ii. 20 mM Tris Triton™ X 100 0.5 % 4 M Urea
iii. 20 mM Tris Triton™ X 100 0.5 % 8 M Urea

After 15 minutes of incubation on ice under gentle agitation, the samples were centrifuged, and the supernatants were separated from the precipitates. The soluble and insoluble fractions of each sample were subjected to SDS-PAGE electrophoresis according to 3.3.1.

29. Evaluation of the means of expression

Expression in TB medium at 30°C

SDS-PAGE gels regarding the expression of scFv in 50 μg/mL kanamycin 1% glucose TB medium at 37°C in an Erlenmeyer flask and at 30°C in a micro bioreactor were subjected to yield analysis by densitometry in Quantity One software (BioRad). For this analysis, each sample was automatically identified as a lane, the background of the gel image was removed, and the protein bands were automatically detected (sensitivity 100, noiser filter 5 ). The relative intensity of the protein band corresponding to scFv was measured.

To analyze the influence of temperature on the amount of protein produced, we repeated the expression steps described in 3.2.2, using transformed bacteria derived from a single clone, with all steps performed at 30°C. After performing SDS-PAGE electrophoresis with the samples resulting from this expression, the relative intensity of the band corresponding to scFv was measured and compared with the expression at 37°C.

30. Expression of scFv in express medium

As a strategy to increase the amount of recombinant protein produced, expression of scFv was performed using commercial Enpresso® B 500 (BioSilta, Sigma-Aldrich) culture medium (138). The steps contained in the manufacturer's instruction manual were followed to perform the expression. The expression step is performed at 30°C and the inducing agent used was IPTG at a final concentration of 0.5 mM. The main differential of this medium consists in the addition of an enzyme that allows the gradual and continuous release of glucose into the culture medium. During the pre-inoculum, induction of expression, and termination steps cell viability tests were performed.

The expression profile before and after induction was evaluated by SDS-PAGE electrophoresis, as described in the previous items. The gel from this procedure was also evaluated for relative intensity, as described in section 3.4.1.

31. Expression in TB medium at 30°C with and without continuous glucose supply

To try to simulate the expression performed with the commercial Enpresso medium, as a strategy to increase the amount of protein produced, expression was performed in 50 $\mu$g/mL kanamycin TB medium and continuous feeding of glucose during the process. To prepare the pre-inoculum, approximately 5 $\mu$L of E. coli SHuffle® pET28a+scFv stock were collected and added to 50 mL of 50 $\mu$g/mL 1% glucose TB kanamycin medium. The pre-inoculum, arranged in a sterile 200 mL Erlenmeyer flask, was incubated at 30°C under 200 rpm orbital shaking for 16 h.

Two inocula were prepared for expression in a Fogale f800 micro bioreactor. The first inoculum consisted of single-batch expression, i.e., without the addition of glucose during the procedure. This procedure was started with TB kanamycin 50 $\mu$g/mL glucose 1% medium, initial OD of 0.15 (addition of pre-inoculum according to 3.2.2) and final volume of 70 mL. Growth was performed at 30°C for 2 hours. The OD after growth was measured, two 500 $\mu$L aliquots were collected, centrifuged, and the precipitate was stored for further evaluation. Induction was performed by adding 0.5 mM IPTG at 30°C for 4 h. At the end of the process, ten 100 $\mu$L aliquots were collected, centrifuged, and stored for further evaluation, and the remaining contents were also centrifuged, and the precipitate was stored at - 20°C.

The second inoculum consists of fed-batch expression of glucose. To do so, the volume of pre-inoculum was calculated, as described previously, and added to a final volume of 50 mL of 50 $\mu$g/mL kanamycin TB. The volume at the beginning of the process was 50 mL. The system was programmed to add 0.04 mL of 10% glucose solution every 1 minute for a period of 360 minutes (2 hours of growth and 4 hours of expression). That is, the inoculum is started without adding glucose, and every minute 4 mg of glucose is injected into the medium. Thus, the growth phase followed the feeding system and lasted for two hours. For induction of expression and collection of aliquots at the end of the process, the same procedure as for single-batch expression was performed.

During the growth and expression processes of scFv, with and without feeding, aliquots of 500 $\mu$L from both inocula were collected every half hour for determination of biomass and the amount of glucose in the medium. For biomass determination, a correlation curve between OD and biomass was prepared. For this purpose, 20 mL of a pre-inoculum (30°C, 16 h, 200 rpm, TB kanamycin 50 $\mu$g/mL) was divided in half into two 15 mL Falcon® tubes. The tubes were centrifuged in an Eppendorf 5810R centrifuge at 12000 rpm (18514 g) for 10 minutes. The supernatants were discarded, and the precipitates were suspended in 10 mL of PBS and centrifuged again. After discarding the supernatant and suspending the precipitate in 10 mL of PBS, the samples were transferred to a 50 mL Falcon® tube and 30 mL of PBS was added. From this initial solution, dilutions of 1:2, 1:4, 1:6, 1:8, 1:10, 1:20, and 1:40 were made. Each dilution was analyzed in triplicate at 600 nm, and 5 mL of the same starting solution was filtered onto 0.2 micron Millipore nitrocellulose membranes previously tared to constant mass. The membranes were then taken to the muffle furnace, where they were dried at 100°C until constant mass. The differences between the membrane masses before and after biomass addition and drying were calculated. The values found were multiplied by the initial solution volumes used in the filtration, in liters. The average value of these values was obtained and divided by the dilution factors. The average ODs obtained x biomass factor divided by the dilution factors were then plotted, and a linear function was obtained. The value corresponding to the intercept constant with the vertical axis of the graph is the DO conversion factor to biomass in g/L.

For glucose determination, aliquots collected at 30-minute intervals were centrifuged and the supernatants were used for quantification. The glucose enzymatic method kit (Megalab) was used for this procedure. We followed the instructions provided by the manufacturer.

Cell viability throughout the procedure, evaluation of the scFv expression profile, and quantification of relative protein band intensity in the QuantityOne program were performed as per previous items.

32. ScFv purification

Cell lysis and inclusion body recovery

For scFv purification, a strategy of urea washing cycles was used to concentrate the scFv, expressed in the form of inclusion bodies, in the insoluble protein fraction and reduce the amount of soluble protein present in the sample. This strategy aims to decrease the number of contaminants in the sample before solubilization in 8 M urea and subsequent application to the affinity chromatography column.

The first step for purification consists of cell lysis of the bacterial biomass. For this, the contents resulting from the expression were used in a Fogale f800 micro bioreactor at 30°C for 4 h in single batch. The sample was previously centrifuged, and the precipitate stored, as described in 3.4.3. This precipitate corresponding to the bacterial biomass is equivalent to 25 to 30 mL of inoculum obtained. For cell lysis, the precipitate was suspended in 4 mL (4mL/mg biomass) of 20 mM Tris Triton™ X 100 2 % Urea 0.5 M NaCl. The sample was then placed in a container with ice and subjected to sonication lysis. For lysis, 4 cycles of 10 seconds of sonication were applied, with an average power of 0.11 and 30 second intervals between cycles. After cell lysis, the sample was centrifuged at 12000 rpm (18514 g) for 10 minutes and the precipitate was resuspended in 3 mL of the same buffer solution as before and again taken for sonication under the same conditions. After this second centrifugation, the precipitate was resuspended in 3 mL of 20 mM Tris Triton™ X 100 2 % Urea 1 M NaCl 0.5 M. The precipitates, containing the scFv in inclusion form, were stored at 4°C. During cell lysis and wash cycles, aliquots of 20 to 100 μL of the crude extract were collected for further SDS PAGE running.

The steps for performing scFv recovery in the form of inclusion bodies are shown in Figure 3.1:

33. Purification of scFv by IMAC (Immobilized metal affinity chromatography)

Purification of scFv was performed using affinity chromatography on resin with immobilized nickel, which is able to interact with the histidine tail fused to scFv.

The first step in performing this purification was to solubilize the inclusion bodies by adding 5 mL of 50 mM Tris 0.5 mM NaCl 8 M Imidazole 1 mM Urea. The sample was incubated on ice under gentle agitation for 20 minutes to allow the recombinant protein to solubilize. The sample was then centrifuged, the supernatant was collected for scFv purification, and the precipitate was discarded. A 20 μL aliquot of the supernatant was collected for application to the SDS-PAGE gel.

Purification was performed manually using 1 mL of Ni-NTA resin (Qiagen) previously equilibrated with 10 mL of 50 mM Tris 0.5 mM NaCl 8 M Urea 1 mM Imidazole. The sample application was performed with recirculation through the resin for 16 h at 4°C, to dynamically prolong the contact with the resin and thus promote the adhesion of the greatest number of scFv molecules. After the recirculation period, the liquid containing the proteins that did not interact with the resin (void) was collected and two washes were performed, by gravity flow: 5 mL of Tris 50 mM NaCl 0.5 mM Urea 8 M Imidazole 1 mM (F1) and 4 mL of Tris 50 mM NaCl 0.5 mM Urea 8 M Imidazole 5 mM (F2). For elution of scFv, 2 mL and 50 mM Tris 0.5 mM NaCl 0.5 mM Urea 8 M Imidazole 1 M was added. Elution occurred for 30 minutes under light TA stirring and the fractions (E) were collected in four microtubes, each with 500 μL of eluted sample. The column was washed with 20 mL of water and stored in 20% ethanol.

34. Renaturation of scFv

To remove the excess imidazole and urea present in the samples after purification, and thus promote the renaturation of scFv, we used the buffer exchange methodology using three Amicon® Ultra-4 Centrifugal Filters 3 K (Merck).

Initially, the filters were subjected to 2 washing cycles with 500 μL of water by centrifugation at 7000 g (7049 rpm, Eppendorf 5810R centrifuge, fixed-angle rotor for six tubes) for 10 minutes. The tubes were then equilibrated by 4 washing cycles with standard buffer solution: 20 mM Tris L-arginine 0.4 M NaCl 100 mM. During each wash, the volumes of liquid that bypassed the filters into the lower compartments of each Amicon® were measured. To define the cycles of centrifugation and addition of standard buffer solution with the samples, we calculated the averages of the liquid volumes measured in each cycle for the three Amicon®.

After the washes, 500 μL of purified scFv was applied to each filter, and at each centrifugation cycle, volumes of standard buffer solution equivalent to the value measured by averaging calculated during the washes were added. At least 16 cycles of centrifugation and addition of buffer to the sample were performed, until the theoretical concentration of urea present in the sample reached a value below 0.6 M. After the cycles, the sample was then collected and stored at 4°C for further testing.

35. Protein quantification, purity evaluation and yield calculation

After purification and renaturation, scFv quantification was performed with the Pierce™ BCA Protein Assay Kit, following the instructions in the manufacturer's manual. For purity evaluation, samples were subjected to SDS PAGE electrophoresis on a 4 to 17% acrylamide gradient gel, followed by densitometry analysis. The gels were prepared with the *Gradient former* Model 485 system (Bio-Rad), following the manufacturer's instructions. Samples collected during all the purification steps in 3.5 were prepared for electrophoresis as described in the previous sections. Migration occurred at an amperage of 0.02 A per gel for a period of 16 h.

For developing the gels, they were washed with water after migration and submerged in a 10 % methanol 50 % acetic acid solution to fix the proteins. The gels were heated for 3 minutes on a hot plate and left under gentle agitation for 20 minutes. The solution was discarded, and the submersion, heating and stirring cycle was repeated three times. After these steps, a solution containing 50 mL of 5 % ethanol, 7.5 % acetic acid, and 1 mL of brilliant blue solution (Comassie Brilliant Blue R250, BioRad) was added to the gels. The gels were heated for 6 minutes on a hot plate and brought to gentle agitation for 20 minutes. This cycle was repeated until the protein bands appeared. For the decolorization of the gels, the staining solutions were discarded, and 50 mL of 5% methanol and 7% acetic acid were added. The gels were heated and taken for stirring, and the solutions were renewed until complete decolorization. After decolorization, the gels were recorded and analyzed in the QuantityOne program, as described in 3.4.1. In this way, the percentage purity of each step of the purification process was obtained.

To calculate the yield, samples from the expressions in TB medium at 30°C in an Erlenmeyer flask, in Enpresso® medium and TB medium at 30°C in a micro bioreactor were subjected to all the purification steps contained in section 3.5, with the exception of the gradient gel evaluation. After protein quantification at the end of each procedure, the amount in mg of protein obtained was divided by the corresponding biomass volume used for purification. The values were adjusted for the total culture volume and the yield value in milligrams of purified protein for each liter of culture used in the expression process was obtained.

36. Increased production volume of anti-VLA-4 scFv

Expression of scFv in a 2 L bioreactor

The first step for a higher and more controlled production of scFv anti-VLA-4 was expression in a 2 L bioreactor. For this, a single-batch expression was performed using the BioFLO110 bioreactor. To prepare the pre-inoculum, approximately 250 μL of the stock of transformed cells were collected, quickly and before thawing of the stock aliquot, and added to 250 mL of 50 μg/mL kanamycin 1 % glucose TB medium. The pre-inoculum, arranged in a weak sterile 1 L Erlenmeyer flask, was incubated at 30°C under 200 rpm orbital shaking for 16 h.

For inoculum preparation, the OD of the pre-inoculum was measured, the pre-inoculum volume was calculated for an initial OD of 0.15, and TB supplemented with kanamycin antibiotic 50 μg/mL and glucose 1% to a final volume of 2 L. The growth period was performed at 30°C for 2 hours. The OD after growth was measured, two aliquots of 250 μL were collected, centrifuged, and the precipitate was stored for further evaluation. Induction was carried out by adding 0.5 mM IPTG, and scFv expression was carried out for 4 h at 30°C with a pure air/oxygen cascade at a flow rate of 1 vvm and stirring at 300-500 rpm while maintaining the dissolved oxygen concentration above 80%. At the end of the process, ten 100 μL aliquots were collected, centrifuged, and stored for further evaluation. The remaining content was divided into 12 50 mL Falcon® tubes that were centrifuged, and the precipitates were stored at -20°C.

37. Cell lysis and inclusion body recovery

Cell lysis was performed in a GEA panda 2k homogenizer. For this procedure, 3 precipitates were resuspended in 20 mM Tris Triton X-100 2 % at a ratio of 4 mL per mg wet biomass. After suspension, the three samples were combined in a 200 mL beaker and the sample volume was made up to 200 mL. The sample was inserted into the homogenizer previously cooled to 4°C and equilibrated with 20 mM Tris Triton X-100 2%. A pressure of 800 bar was applied for 10 minutes to allow cell lysis. After this period, the sample, with a clarified appearance, was centrifuged at 4000 rpm (2057 g Eppendorf 5810R centrifuge) for 30 minutes. The supernatant was discarded, and the precipitate was forwarded to the scFv recovery step in the form of inclusion bodies.

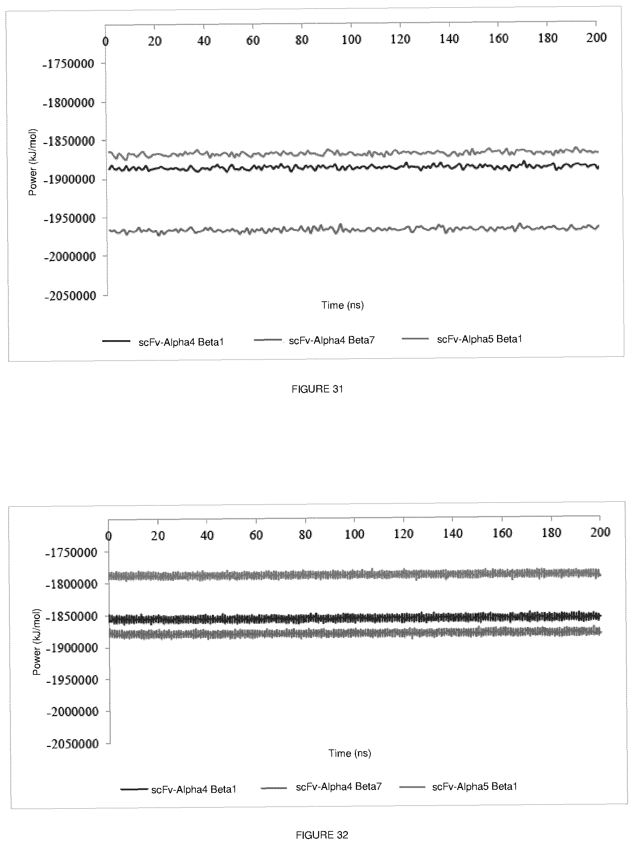

The urea wash cycles for recovery of the inclusion bodies, in this case, dispensed with the sonication lysis steps. However, due to the higher amount of protein, one more wash cycle, with 2 M urea, was added. In addition, the solution volumes for the suspension of the precipitates were 10 times larger compared to the non-scaled yield. The steps for performing the recovery of scFv in the form of inclusion bodies in larger scale production are shown in Figure 32:

38. Purification on IMAC chromatography with linear gradient elution and renaturation

For purification by affinity chromatography, a 1 mL HisTrap™ column (GE Healthcare) on a ÄKTA pure Chromatography System chromatograph (GE Healthcare) was used. The inclusion bodies were resuspended and solubilized in 40 mL of 50 mM Tris 0.5 M NaCl 8 M Urea. The sample was left in gentle agitation on ice for 20 minutes and was then centrifuged in an Eppendorf 5810R centrifuge at 12000 rpm (18514 g) for 10 minutes. The precipitate was discarded, and the supernatant was filtered and transferred to a 50 mL Falcon® tube. The system was equilibrated with 10 mL of 50 mM Tris 0.5 M NaCl 8 M Urea and the sample was applied at a flow rate of 1 mL/min in recirculation for 2 h. Elution of scFv occurred by applying a linear gradient of imidazole, which ranged from 0 to 1 M in a volume of 20 CV (Column volume), remaining at 1 M for another 3 HP. After the imidazole gradient, the system was washed with water and stored in 20 % ethanol.

The collected fractions corresponding to high scFv concentrations are pooled into a single sample, which is destined for renaturation. For this step, dialysis methodology was used, using Slide-A-Lyzer™ Dialysis Cassettes, 3.5K MWCO, 3 mL devices (Thermo Fisher Scientific). Initially, 667 μL of standard buffer solution (Tris 20 mM L-arginine 0.4 M NaCl 100 mM) was added to 2 mL of sample, causing the urea concentration to decrease from 8 to 6 M. Then 1.2 L of Tris 20 mM L-arginine 0.4 M NaCl 100 mM Urea 4 M immersion solution and 2 L of standard buffer solution were prepared. The dialysis cassette was thoroughly rinsed with water and equilibrated with immersion solution. After the cassette was balanced, the sample was carefully injected.

The cassette with the sample was placed in the immersion solution a, and incubated for 2 hours under gentle agitation with the aid of a magnetic device at 4°C. After this period, a certain volume of immersion buffer was replaced by the less volume of exchange buffer. The cassette was incubated again, after changing solutions, under the same conditions. The first three exchanges and corresponding volumes were performed to decrease the urea concentration by 1 M with each exchange. The last four exchanges and volumes decreased the urea concentration by 0.5 M per exchange. This procedure was adopted to reduce the loss of protein by precipitation. The final solution after the last change was 0.5x buffer solution.

39. Final processing: quantification, endotoxin reduction, freeze-drying and reconstitution

After dialysis the sample was quantified by BCA and subjected to bacterial endotoxin content reduction using the Pierce™ High Capacity Endotoxin Removal Resin 88274 kit (Thermo Fisher Scientific), according to the instructions provided in the manufacturer's manual. For quantification of the endotoxin level, the Endosafe® Cartridge Technology kit (Charles River) was used, also pursuant to the instructions available in the manufacturer's manual.

After the previous processing, the sample was divided into threaded microtubes at a ratio of 100 μL per tube and subjected to lyophilization. Once lyophilized, the samples were stored long-term at -20°C. For use, the contents of each tube were hydrated with 50 μL of ultrapure water, without homogenization by pipetting or vortexing. After rehydration the samples were again quantified by BCA and stored at 4 or -20°C.

All scFv production, purification, and processing steps performed were monitored by collecting aliquots during the steps of each process. Aliquots were prepared with sample buffer and subjected to SDS-PAGE electrophoresis for visualization of the protein profile in each procedure.

40. Evaluation of the interaction of scFv immobilized with VLA-4 on the cell surface of Jurkat cells

To evaluate the binding capacity of scFv with its target, a simple experiment was performed to assess the number of cells expressing VLA-4 adhered to scFv-coated surfaces. Initially, 96-well Nunc MaxiSorp™ flat-bottom plates were sensitized, in triplicate, with 0, 1, 10, 100, 200, 400 and 800 ng of scFv in 100 μL of 0.05 M carbonate-bicarbonate buffer solution pH 9.6 (Sigma-Aldrich) for a period of 1 hour at 37°C. After incubation, the plate was washed four times by adding and discarding 150 μL of 0.05 % PBS-T in each well, followed by blocking by adding 300 μL of Molico™ 4 % skim milk solution, BSA (Bovine Albumin Serum) 0.5 % PBS-T 0.05% at 37°C for 1 hour and then washed with 0.05 % PBS-T.

Jurkat strain, Clone E6-1 (ATCC® TIB-152™) cells were used to perform the experiment. This strain consists of immortalized human T lymphocytes derived from the blood of a patient with acute leukemia, with high expression of VLA-4. Cells were thawed and cultured in 15 mL of RPMI-1640 medium (Gibco, Thermo Fisher Scientific) supplemented with 10 % FBS (Fetal Bovine Serum), Gibco, Thermo Fisher Scientific) and antibiotic-antimycotic solution (A5955, Sigma-Aldrich) in a culture bottle with filter (Nunc™ Cell Culture Treated Flasks with Filter Caps, Thermo Fisher Scientific), at a concentration of 5x10⁵ cells/mL. The culture was maintained at 37°C with 5% CO2 always with the cell concentration maintained in the 10⁵ to 10⁶ cells/mL range, this also being the acceptable concentration range for the use of the cells in experiments. The use of cultures with more than 20 passages was avoided during all experiments.

A cell culture volume corresponding to 8x10⁵ cells/mL was collected and centrifuged in an Eppendorf 5810R centrifuge at 400 g (12000 rpm in horizontal rotor) for 5 minutes. The supernatant was discarded, and the precipitate was suspended in RPMI medium without FBS. Then, 100 μL of the cell suspension, at a concentration of 10⁵ cells/100 μL, were added to the wells of the scFv-treated plate. The plate was incubated for 1 hour at 37°C. Then the wells were washed three times with medium warmed to 37°C. The plate was taken for visualization on an Olympus CKX53 inversion microscope. With the aid of a camera attached to the eyepiece of the microscope, three pictures were acquired of each well at different fields. Each acquired photo corresponds to a 1 mm$^2$ area of the well surface.

The number of cells in each photo was counted using the Cell Counter tool available in the ImageJ program. The counts were done blindly without the condition identification being known at the time of analysis. Cells that appeared in another plane of the photo were not counted. The experiments were performed in biological triplicates, resulting in a number of nine counts per condition.

40. Evaluation of the direct interaction between scFv and VLA-4 by ELISA

To evaluate the interaction between anti-VLA-4 scFv and $\alpha 4\beta 1$ integrin, the ELISA (Enzyme-Linked Immunosorbent Assay) technique was used using 400 ng of recombinant $\alpha 4\beta 1$ integrin (R&D Systems) immobilized on a plate and different concentrations of scFv. The sensitization and blocking of the plates were performed as described in section 3.7.

Three different concentrations of scFv were evaluated, in triplicate, in incubations at 37°C for 30 minutes. The plate was then washed, and peroxidase-conjugated anti-human Kappa Light Chain $\kappa$ antibody (Anti-Human Kappa Light Chains (Bound and Free) - Peroxidase antibody produced in goat, A7164, Sigma Aldrich) was added at 1:1000 dilution. After a 1 hour incubation period at 37°C, the plate was washed and 100 $\mu$L of Single Component TMB Peroxidase® (BioRad) was added for developing for 20 minutes at 37°C in the dark After this period, 50 $\mu$L of 2 N H2SO4 was added to each well. The plate was analyzed in a Spectra Max 190 ELISA reader (Molecular Devices), at a wavelength of 450 nm.

41. Evaluation of reduced transmigration of Jurkat cells on VCAM-1 and FN coated surfaces treated with anti-VLA-4 scFv

Preparation of Transwell® chambers with VCAM-1 and fibronectin

6.5 mm diameter Transwell® chambers with 5 $\mu$m pores (Corning, 3421) arranged in 24-well plates (eight inserts per plate) were used for the transmigration experiments on VCAM-1 and FN. To do this, 70 $\mu$L of VCAM-1 2.5 $\mu$g/mL or FN 10 $\mu$g/mL were added to the wells, forming a drop in the center of the wells. The corresponding inserts from each well were placed on the drop, and 60 $\mu$L of VCAM-1 or FN solution was added to each insert, also covering the bottom of the membrane containing the pores. In at least one insert from each plate, a solution of 1% BSA in PBS (w/v) used as a negative control of transmigration was added. The plate was incubated for a period of 16 h at 4°C.

The inserts were washed three times by disposing 300 $\mu$L of PBS on the outside of the insert membrane. Each insert was arranged in a new well. For blocking, the previous procedure was repeated using 1 % BSA, over a 1 hour incubation period at 37°C. After incubation, the inserts were washed and arranged in another well, containing 600 $\mu$L of 0.5 % RPMI BSA medium at warmed to 37°C.

42. Cell preparation and transmigration

Jurkat cells maintained in culture in the concentration range between 5x105 and 106 cells/mL were used for the transmigration experiment. One million cells were separated for the following conditions: treatment with scFv 20 $\mu$g/mL, treatment with Natalizumab 20 $\mu$g/mL, positive control (migration on VCAM-1 or FN without treatment) and negative control (migration on BSA). The cells were incubated with their respective treatments in a volume of 20 $\mu$L for 30 minutes at 37°C. After this period, the cell suspensions were adjusted to 100 $\mu$L and arranged for transmigration in their respective inserts.

The plate with the inserts and cells was incubated at 37°C, 5 % CO2 for 3 hours. After this period, the outsides of the inserts were washed using the contents of each respective well. This content was centrifuged, the supernatant was carefully discarded, and the precipitate was resuspended in 100 $\mu$L of medium. The number of cells present in each cell suspension, referring to different treatments, was quantified using a Neubauer chamber. Each experiment was repeated at least three times to perform the mean and standard deviation analysis.

43. Evaluation of adhesion and migration blockade of memory T cells treated with anti-VLA-4 scFv to VCAM-1 and FN-coated surfaces under flow Surface preparation with VCAM-1 and fibronectin

To evaluate the potential of the scFv molecule to inhibit memory T lymphocyte adhesion on surfaces coated with the two major VLA-4 ligands under shear stress, Ibidi® $\mu$-Slide VI 0.1 plates were used. Initially, a solution of protein A, which recognizes the Igs domains of the VCAM-1 molecule, was prepared at a concentration of 40 $\mu$g/mL, and chemokine CXCL12 2 $\mu$g/mL. In each channel of the chamber, 40 $\mu$L of this solution was added, using a 1 mL syringe to homogeneously distribute the liquid inside the channels, avoiding the formation of bubbles. The plate was incubated at 37°C for 30 minutes.

After incubation, the channels were washed three times by adding and discarding PBS. For channel blocking, 40 μL of 2 % BSA was added to each channel. The plate was incubated for 10 minutes at RT. After this period the channels were washed and 30 μL of VCAM-1 10 μg/mL was added. The plate was incubated for 16 h at 4°C.

To treat the canals with fibronectin, a solution of FN 25 μg/mL CXCL12 2 μg/mL was prepared. In each channel 20 μL of the solution was added. The plate was incubated at 37°C for 30 minutes. The canals were washed and blocked with BSA 2%. The canals were washed again and stored in PBS.

44. Preparing the cells

The preparation of cells for this experiment was performed from PBMCs (Peripheral Blood Mononuclear Cells) derived from blood bags from healthy donors, obtained from the Hôpital de la Conception - Marseille, France. PBMCs were previously separated by separation gradient using LSM medium (Lymphocyte Separation Medium, Corning®), according to the manufacturer's instructions. The cells were then frozen or cultured in 15 mL of RPMI-1640 medium supplemented with 10 % FBS, antimycotic antibiotic solution and GlutaMAX™ (Gibco, Thermo Fisher Scientific). PBMC cultures were maintained at the concentration of 1 to $5x10^6$ cells/mL for a period not exceeding one week.

Memory T lymphocytes were separated from the culture of PBMCs by a combination of the Pan T Cell Isolation Kit, human and CD45RA MicroBeads, human (MACS, Miltenyi Biotec). This combination allows the separation of T-lymphocytes that do not express CD45R, i.e., have already been activated. The culture of PBMCs was centrifuged at 300 g for 15 minutes, suspended in MACS buffer solution, and their concentration was determined using KOVA® Slide II chambers. The cell suspension was centrifuged again, the supernatant was discarded, and the precipitate was resuspended in MACS buffer solution at a ratio of 40 μL/$10^7$ cells. Pan T Cell Biotin Antibody Cocktail solution was added and homogenized to the cell suspension at a ratio of 10 μL/$10^7$ cells and incubated at 4°C for 10 minutes. After this period the solutions of CD45RA microbeads and Pan T cell microbeads were added in the ratio of 20 μL/$10^7$ cells. The sample was then incubated at 4°C for 15 minutes and then transferred to a disposable magnetic bead column, attached to the magnetic field device, equilibrated with MACS buffer. The tube where the cell suspension with the antibodies and microspheres was prepared was washed with 1 mL of MACS buffer, and this content was also placed in the column. The liquid passing through the column, containing memory T cells, was collected in a new Falcon® tube, where the volume was made up to 20 mL. The tube was centrifuged, the supernatant was discarded and the cells were suspended in RPMI-1640 medium supplemented with 10 % FBS, antimicrobial antibiotic solution and GlutaMAX™ at a ratio of $10^6$ cells/mL. The cells were either used in a same-day experiment, after a recovery period of at least 1 hour at 37°C or kept in culture for a maximum of 5 days.

45. Preparing the system for flow-through experiments

An inverted microscope (Olympus France, Rungis, France) in bright field, equipped with a 10 x magnification lens and an attached Sony CCD video camera, was used to perform and monitor the experiment. For flow application, two syringe pumps (A-99; Razel, St. Louis, USA) were used. Albans, VT) with 1 and 5 mL syringes. The microscope and pumps are arranged in a large acrylic box with temperature control, so that the temperature of the system during the experiment must be kept at 36 and 37°C.

Initially, syringes and hoses were filled with culture medium at 37°C and attached to the pumps. The hoses were connected to the first channel of the Ibidi® plate with care not to form bubbles. To prevent bubbles from forming, the hoses and the channel must be completely filled with liquid. After connecting the hoses, the plate was fixed on the microscope plate so that the canal could be seen. The canal was briefly flushed with up to 1 mL of medium, using a syringe connected to one of the hose valves.

For the injection of the cells into the channel, a 1 mL syringe was filled with 400 μL of memory T lymphocytes. The syringe was placed in the valve, connected to the hoses, closest to the canal inlet. At each acquisition for adhesion evaluation, 100 μL of cells were injected.

The experiment was monitored and recorded using the VirtualDubMod program, which allowed the acquisition of videos relating to the different conditions of the experiment. The video signal has been digitized and compressed using the "ffdshow" option.

In each canal a particular cell treatment was used. The performance of each treatment was evaluated by acquiring six videos per channel, each with a different flow, or shear stress, applied, measured in dyn/cm2. In addition, each acquisition differs in its objective, cell injection, acquisition time, use of the 1 or 5 mL syringe, and speed set on the pump, which corresponds to the value corresponding to the shear stress according to the syringe used. The conditions of the six acquisitions per channel are laid out in Table 1:

Table 1 Acquisition conditions per channel.

| Flow (dyn/cm$^2$) | Cell injection (100 $\mu$L) | Time (minutes) | Goal | Syringe volume (mL) | Set speed at the pump |
|---|---|---|---|---|---|
| 0.13 | Yes | 5 | Frequency of membership | 1 | 8 |
| 0.06 | Yes | 5 | Frequency of membership | 1 | 4 |
| 0.03 | Yes | 5 | Frequency of membership | 1 | 2 |
| 1 | No | 10 | Migration | 1 | 60 |
| 2 | No | 10 | Migration | 5 | 18 |
| 4 | No | 10 | Migration | 5 | 35 |

46. Cell treatment and experiments in VCAM-1 and FN

For the experiments performed on VCAM-1-coated surfaces, T lymphocytes were treated in the absence and presence of three different concentrations of scFv. In the control condition, the cells were directly injected into the canals, without prior treatment. The concentrations of scFv evaluated were 0.2, 2 and 20 $\mu$g/mL. For each concentration, individually evaluated in a channel, the cells were incubated for a period of 15 to 30 minutes with the scFv at the mentioned concentrations at 37°C. After this period, the cells could be injected into their respective channels.

For the experiments performed on fibronectin, cells were treated in the absence and presence of scFv 2 $\mu$g/mL and Fab of Natalizumab 3 $\mu$g/mL. The cells were incubated with such antibodies for a period of 15 to 30 minutes at 37°C prior to their application to the channel.

Each treatment mentioned was evaluated and repeated at least 5 times for further analysis and obtaining the average value.

47. Analysis of acquisitions

The first three acquisitions aim to observe the reduced frequency of adhesion of memory T lymphocytes to VLA-4 ligands in the presence of antibodies blocking this integrin, such as the scFv under study. The videos acquired in the first three conditions relating to these objectives (table 3.10.3.1) were then subjected to cell trajectory identification and mapping, and subsequent calculation of the adhesion frequency.

For trajectory mapping, we used the Trajectories 3_2 plug-in developed by the Laboratory of Adhesion and Inflammation (INSERM 1067, Marseille, France). To calculate the adhesion frequency, the Arrests 3_2 plug-in developed by the same laboratory was used. Both plug-ins were installed in the ImageJ program. The first analysis has as input file folders containing the acquired videos. The second analysis, on the other hand, uses the output files from the first analysis as input files for analyzing the trajectories.

The last three acquisitions, aim to evaluate the migration profile of the cells that were able to adhere to the surfaces, even in the presence of blocking antibodies. For evaluation of the acquired videos concerning migration (table 3.10.3.1), The Reducer plug-in was used to convert the videos into images corresponding to their frames. The migration profile was evaluated using the MATLAB 7.13 program and three scripts developed by the Adhesion and Inflammation Laboratory. The first script was used to remove the background from the images and normalize them following the pattern of cells as white dots on a black background. The second script was used to track the number of cells and their positions in each image. The last script, was used to calculate the velocities, directions, and other cell-related parameters from the previous output file.

48. Evaluation of scFv interference on cell activation

Preparing the cells

CD8+ T cells were used to evaluate the interference of scFv on T lymphocyte activation. These cells were purified via the EasySep™ Human CD8+ T Cell Enrichment Kit system (following the manufacturer's instructions) from PBMCs maintained in culture for 10 days in 24-well plates in RPMI-1640 medium supplemented with 10 % SBF, antimicrobial antibiotic solution, GlutaMAX™ and IL-2 50 U/mL. The purified cells were maintained in culture under the same initial conditions at a concentration of approximately 107 cells/mL.

49. Slide preparation

Slides coated with fibronectin and/or anti-CD3 antibody were used to activate the cells and visualize the effects of scFv treatment under a microscope. Initially, solutions of FN 10 μg/mL, anti-CD3 10 μg/mL and a mix solution (FN 10 μg/mL anti-CD3 10 μg/mL) were prepared. Each solution was added to one well of a 76x26x1 mm slide with 10 wells of 7 mm2 area (Marienfeld, 1216824) previously cleaned. The slide with the solutions was incubated for 16 h at 4°C in a humidity chamber.

Cells were treated in the presence and absence of scFv to perform the experiment. To do so, 750 μL of cells at a concentration of 5x104 cells/50 μl were washed and suspended in RPMI BSA 1 %. The cells were divided into two 15 mL Falcon® tubes. In one of the tubes, 200 ng of scFv was added and the cell suspension was gently homogenized in the presence of scFv. Both tubes were incubated for 30 min at 37°C. After this period, the cells were centrifuged at 1500 rpm (190 g) for 5 min. The precipitates were suspended in 700 μL of 1% RPMI BSA and the supernatants were discarded.

The slide wells were washed three times by adding 50 μL of PBS, incubating for 3 min at TA and removing it. After washing, 50 μL of the cell suspensions, treated and untreated with scFv, were arranged over the wells in the different conditions (FN, anti-CD-3 and FN+anti-CD3 mix). The slide was incubated for 20 min at 37°C. After this period, the medium from the wells was gently removed.

To fix the cells in the wells, 50 μL of para-formaldehyde (PFA) was added to each well by incubation for 10 min at 37°C. The PFA was removed and the wells, washed twice, with 50 μL of 3 % Hepes 10 mM BSA, for 2 min at TA.

For permeabilization of the fixed cells, 50 μL of BSA 3 % saponin 0.1 % was added to each well for 15 min at TA. The permeabilization medium was removed and the wells were washed three times with 50 μL of BSA 3 % saponin 0.1 %, for 3 min at TA.

For molecular target labeling to evaluate the effect of scFv on activation, primary anti-CD49d antibodies (Purified Mouse Anti-Human CD49d, Mouse IgG1, κ, 555502, BD Biosciences, dilution 1:50) were used, for recognition of the α4 subunit of integrins, and phosphorylated anti-tyrosine (p-Tyr Antibody PY99: sc-7020, mouse monoclonal IgG2b kappa light chain, dilution 1 :50), for recognition of phosphorylated tyrosine regions, related to signaling pathways. Both antibodies were incubated for 1 hour at RT. After this period, the wells were washed with BSA 3 % saponin 0.1 %.

The secondary antibodies used were mouse anti-IgG1 conjugated to Alexa Fluor® 488 (Goat anti-Mouse IgG1 Cross-Adsorbed Secondary Antibody, Alexa Fluor 488, A-21121, Invitrogen, dilution 1:200) and mouse anti-IgG2 conjugated to Alexa Fluor® 647 (Goat anti-Mouse IgG2b Cross-Adsorbed Secondary Antibody, Alexa Fluor 647, A-21242, Invitrogen, 1:200), respectively, for the development of anti-CD49d and p-Tyr. Phalloidin (Alexa Fluor® 555 phalloidin, A34055, Thermo Fisher Scientific, dilution 1:40) was used for actin labeling. The secondary antibodies and phalloidin were incubated for 1 hour at RT in the dark

The wells were washed with PBS three times and all residual liquid was carefully aspirated, avoiding as much as possible any remaining liquid. Quickly, one drop of DABCO mounting medium (Abeam) was added to each well, the slide was covered with a coverslip and sealed with clear enamel. The slides were stored at 4°C in the dark.

50. Visualization and analysis of the treatments

The slides were viewed on a LSM 710 confocal microscope (Zeiss). About 20 images per treatment were obtained. For quantification of the focal points of phosphorylated tyrosine in the cells, a plug-in developed for analysis of the experiment was used and installed in the ImageJ program. The plugin consisted of selecting the image containing only the channel corresponding to the visualization of phosphorylated tyrosine, subtracting the background, applying a Threshold of 0 and 135, and quantifying the particles with a minimum of 0.1 μm in diameter. The number of phosphorylated tyrosine particles is related to cellular activation.

For quantification of actin packing structures, a plug-in was used, with the same principle as above. However, the minimum size of the diameters of the actin structures was 0.5 μm.

51. Statistical evaluation of the scFv evaluation experiments

To evaluate the data from the scFv evaluation experiments (items 3.7 to 3.11) the GraphPad Prism 7 program was used. The means and standard deviations for each set of experiments were calculated by the program. In addition, the significance of each effect observed was calculated using one-way ANOVA against control or T-student test according to the number of conditions evaluated.

## EXAMPLE 1

### Prospecting

Three antibody light and heavy chain sequences were obtained from Integrity, with the identification codes: 257898, 670484 and 725144.

### Comparative modeling

The templates obtained from the PDB used as reference for assembling the three-dimensional structure of the antibodies showed identity and coverage values above 80%, with E value equal or below zero, confirming that the alignment was reliable and appropriate for performing comparative modeling (table 2).

Table 2 - Molds used for the construction of each antibody and their parameters.

| Antibody | Chain | Model (pdb) | Identity | Coverage | E value | Resolution |
|----------|-------|-------------|----------|----------|---------|------------|
| 257898 | Light | 4LKX B | 93% | 100% | 2,00e-149 | 1,92 A |
| 257898 | Heavy | 1HZH H | 90% | 100% | 0.0 | 2,70 A |
| 670484 | Light | 1MCO H | 83% | 98% | 0.0 | 3,20 A |
| 670484 | Heavy | 1HZH H | 83% | 100% | 0.0 | 2,70 A |
| 725144 | Light | 3NCJ L | 94% | 100% | 2,00e-148 | 1,60 A |
| 725144 | Heavy | 1HZH H | 83% | 100% | 0.0 | 2,70 A |

The light and heavy chains were obtained, presenting the structures shown in Figures 2 and 3.

## EXAMPLE 2

### Evaluation and refinement of the modeled structures

After refinement of the modeled chains done in Coot, the structures were evaluated by the MolProbity and Verify 3D servers. The structures showed the following parameters (table 3) in the evaluations:

Table 3 - Structure quality parameters by MolProbity and Verify 3D server

| Antibody | Chain | Bad Rotamers | Favorable Rotamers | Outliers Residuals (Ramac.) | Favorable residues (Ramac.) | Verify 3D |
|----------|-------|--------------|--------------------|-----------------------------|-----------------------------|-----------|
| 257898 | Light | 0% | 100% | 0% | 98.16% | 98.17% |
| 257898 | Heavy | 0% | 100% | 0.22% | 95.99% | 88.03% |
| 670484 | Light | 0% | 100% | 3.43% | 88.10% | 87.70% |
| 670484 | Heavy | 0% | 99.75% | 0.22% | 95.58% | 86.81% |
| 725144 | Light | 0% | 100% | 0.47% | 96.70% | 85.05% |
| 725144 | Heavy | 0% | 100% | 0.68% | 95.93% | 88.29% |

Rotamers correspond to twists and combinations of angles relative to the side chain of amino acids that correspond to the position of minimum energy (SCHRAUBER; EISENHABER; ARGOS, 1993). In this context, a favorable rotamer is in its conformation of energetically appropriate twists and angles, while bad rotamers correspond to energetically improbable conformations. MolProbity considers those with over 98% favorable rotamers and less than 0.3% bad rotamers as adequate. All the chains modeled met these requirements.

Verify 3D evaluates the compatibility of a 3D model with its primary structure. The server considers structures with a value of 80% or more as approved. The values obtained by the modeled chains were higher than this value.

The dihedral angles $\Psi$ (psi) and $\Phi$ (phi) of an amino acid residue have limits to their conformations, so there are angles that are allowed, favorable, and not allowed. The Ramachandran graph shows the combinations of these angles, differentiating the favorable, allowed, and disallowed regions. The residues are plotted and it is possible to see in which region they fit. MolProbity considers that there should be a minimum of 99 % allowable residues, according to the chart. The Ramachandran plots of the chains (figure 4) showed no or only one residue in disallowed regions, with the exception of the light chain 670484. This proportion is seen in percentages in table 3.

## EXAMPLE 3

### Obtaining the scFvs

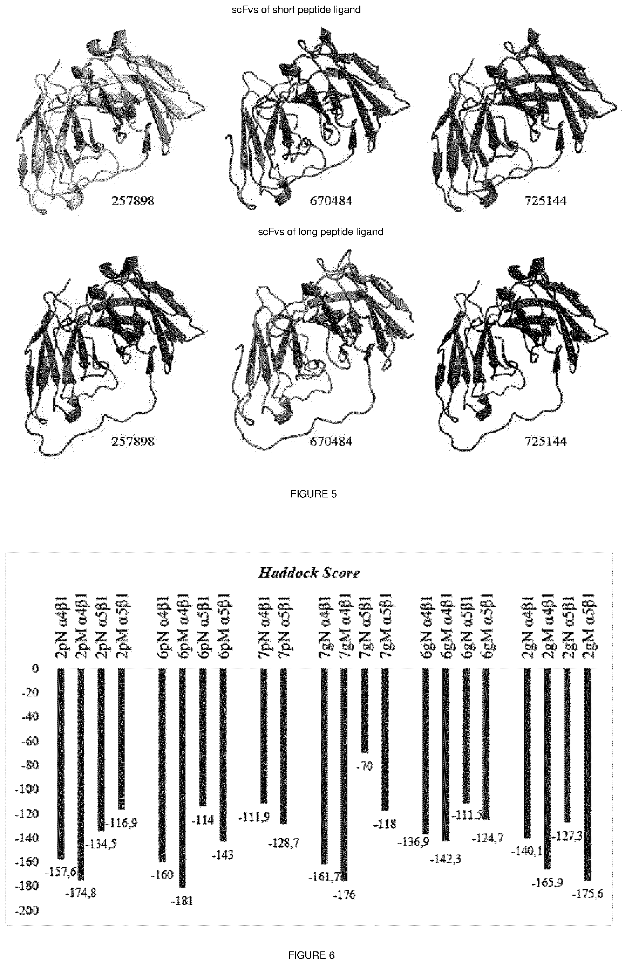

For each antibody, one scFv with long binding peptide and one with short binding peptide were obtained (figure 5).

### Mapping of CDRs and active waste

The CDRs of each scFv were defined. Different amounts of active residues were found in the CDRs of each scFv (table 4). ScFvs of the same sequence, with different binding peptides, showed different numbers of active residues. However, these values showed no direct relationship with the size of the binding peptides.

Table 4 - Quantity of active waste in the CDRs of each scFv

| scFv | Total amount of active waste in the CDRs |
|---|---|
| 257898 of short peptide ligand | 23 |
| 257898 of long peptide ligand | 27 |
| 670484 of short peptide ligand | 28 |
| 670484 of long peptide ligand | 26 |
| 725144 of short peptide ligand | 23 |
| 725144 of long peptide ligand | 19 |

### Docking with integrin α4β1

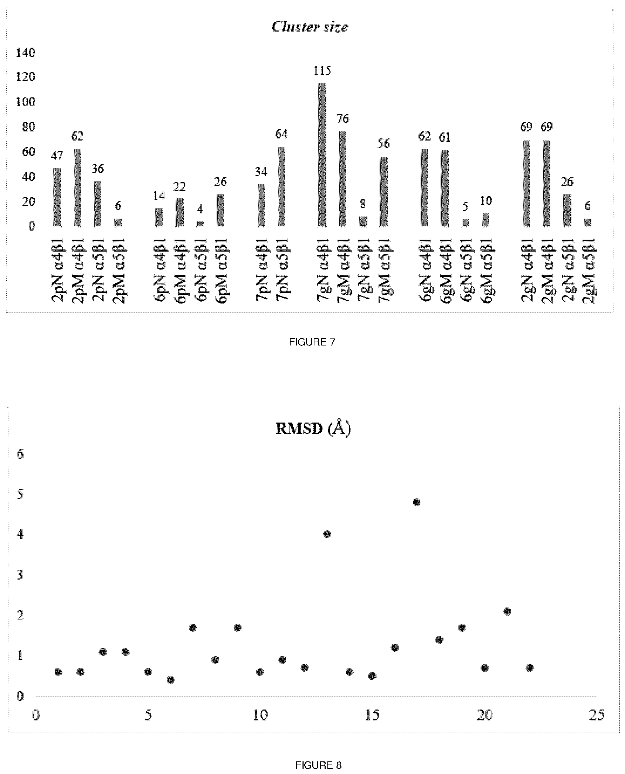

From the knowledge of the active residues of scFvs and integrin α4β1 it was possible to perform docking using Haddock. The results generated are shown in figures 6, 7 and 8.

### Searching for hotspots from the best complexes

All α4β1-scFv complexes showed hotspots important for the stability of the proteins involved and for the scFv-integrin interaction. As with the active residues, the number of hotspots was variable for each complex (table 5).

Table 5 - Number of hotspots in each α4β1-scFv complex

| Integrin α4β1 complex with scFv: | Total amount of *hotspot* waste in each complex |
|---|---|
| 257898 of short peptide ligand | 18 |
| 257898 of long peptide ligand | 15 |
| 670484 of short peptide ligand | 26 |
| 670484 of long peptide ligand | 24 |
| 725144 of short peptide ligand | 9 |
| 725144 of long peptide ligand | 26 |

### Interaction analysis of the complexes and identification of the interacting hotspots

From the PDBePISA results it was observed that almost all scFvs, with the exception of the short peptide-binding scFv 725144, showed hotspots performing hydrogen bonding or salt bridge type interactions with integrin residues. The number of interacting hotspots was higher for long-binding peptide scFvs (Table 6).

Table 6 - Number of interacting hotspots in each scFv

| Integrin $\alpha4\beta1$ complex with scFv: | Total number of interacting hotspots in each scFv |
|---|---|
| 257898 of short peptide ligand | 9 |
| 257898 of long peptide ligand | 17 |
| 670484 of short peptide ligand | 11 |
| 670484 of long peptide ligand | 17 |
| 725144 of short peptide ligand | 0 |
| 725144 of long peptide ligand | 17 |

**Targeted mutagenesis of scFvs**

Different mutations were made in each scFv. The number of mutations made in each scFv was based on the number of residues prone to substitution in the regions near the interacting hotpots (table 7). No mutations were made in the short ligand-binding peptide scFv 725144, as it did not exhibit interacting hotspots that would serve as a basis for making its surroundings a strong point of interaction.

Table 7 - Number of mutations made in each scFv

| Integrin $\alpha4\beta1$ complex with scFv: | Total amount of mutations done in each scFv |
|---|---|
| 257898 of short peptide ligand | 2 |
| 257898 of long peptide ligand | 3 |
| 670484 of short peptide ligand | 1 |
| 670484 of long peptide ligand | 3 |
| 725144 of short peptide ligand | 0 |
| 725144 of long peptide ligand | 4 |

**Docking of scFvs against integrin $\alpha4\beta1$, $\alpha5\beta1$ and $\alpha4\beta7$**

By comparing the results of dockings of native and modified scFvs with the integrins $\alpha4\beta1$ and $\alpha5\beta1$ it was possible to evaluate the effect of the mutations performed in comparison with native scFvs.

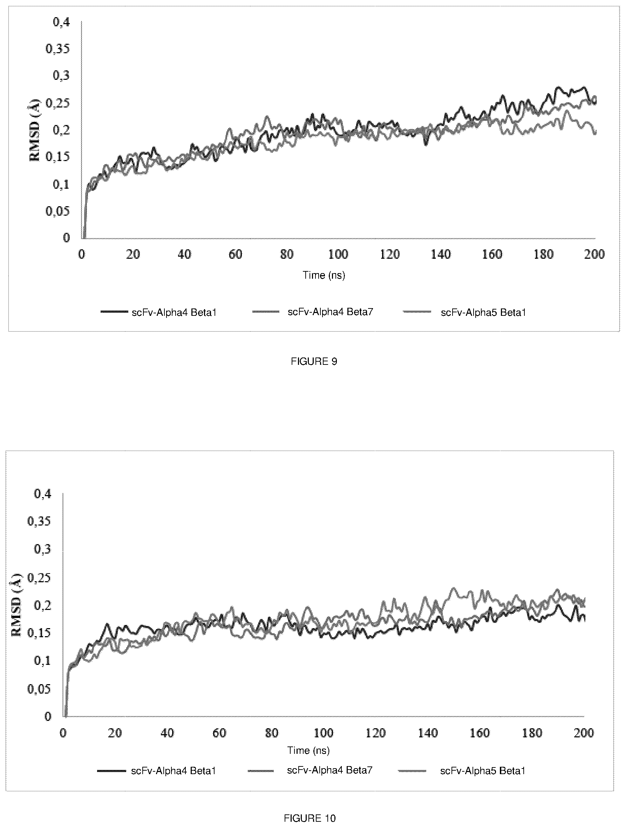

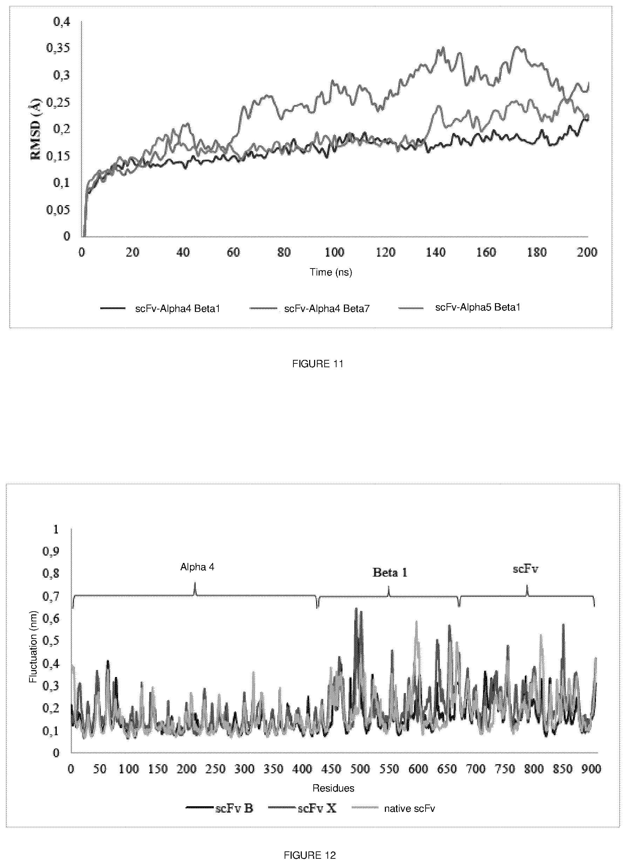

It can be seen that scFv 257898 modified with short peptide ligand showed a decrease in the Haddock score value for $\alpha4\beta1$ and an increase in this value for $\alpha5\beta1$ (figure 9), in comparison with its native form (without mutations). This same scFv showed an increase in Cluster size value for $\alpha4\beta1$ and a decrease in it for $\alpha5\beta1$ (figure 10). Only the dockings of the long peptide native scFvs 670484 and 725144 with the $\alpha5\beta1$ integrin showed RMSD values greater than 2 Å (figure 11). The short peptide modified scFv 257898 showed RMSD value equal to 0.6 and 1.1 Å, respectively, for integrins $\alpha4\beta1$ and $\alpha5\beta1$. Therefore, this scFv was selected as the best.

The docking of the best scFv with integrin $\alpha4\beta7$ showed higher Haddock Score value than the value for $\alpha4\beta1$ (table 8). However, the Cluster size value for $\alpha4\beta7$ was much higher than the value for $\alpha4\beta1$ (table 8), which led to new mutations.

Table 8 - Results of the docking of the short peptide modified scFv 257898 with the integrins $\alpha4\beta1$, $\alpha5\beta1$ and $\alpha4\beta7$

| | $\alpha4\beta1$ | $\alpha5\beta1$ | $\alpha4\beta7$ |
|---|---|---|---|
| Haddock Score | -174.8 +/- 10.5 | -116.9 +/- 17.7 | -136.3 +/- 5.0 |
| Cluster size | 62 | 6 | 160 |
| RMSD (Å) | 0.6 +/- 0.4 | 1.1 +/- 0.9 | 1.1 +/- 0.6 |

**EXAMPLE 4 - Targeted mutagenesis of the best scFv and repeat docking**

The second series of mutations performed generated a modified scFv with three modified residues compared to the original scFv (257898 native short ligand peptide). This new scFv, was named scFv B. After the third series of mutations, the new scFv generated contained eight modified residues compared to the original scFv. This scFv was called scFv X.

It was observed from the docking result of scFvs B and X with the integrins $\alpha4\beta1$, $\alpha5\beta1$ and $\alpha4\beta7$ that the second set of mutations was not enough to effectively decrease the Cluster size value, but the third set of mutations was able to bring about this result (tables 9 and 10).

Table 9 - Results of the docking of scFv B with the integrins $\alpha4\beta1$, $\alpha5\beta1$, and $\alpha4\beta7$

|  | $\alpha4\beta1$ | $\alpha5\beta1$ | $\alpha4\beta7$ |
|---|---|---|---|
| Haddock Score | -163.0 +/- 8.0 | -147.5 +/-7.5 | -136.6 +/-7.9 |
| Cluster size | 52 | 35 | 144 |
| RMSD | 1.0 +/- 1.1 | 0.8 +/- 0.5 | 0.8 +/- 0.5 |

Table 10 - Results of the docking of scFv X with the integrins $\alpha4\beta1$, $\alpha5\beta1$, and $\alpha4\beta7$

|  | $\alpha4\beta1$ | $\alpha5\beta1$ | $\alpha4\beta7$ |
|---|---|---|---|
| Haddock Score | -157.2 +/- 2.1 | -118.4 +/- 20.9 | -96.8 +/- 9.7 |
| Cluster size | 72 | 8 | 45 |
| RMSD | 0.5 +/- 0.3 | 1.0+/-0.7 | 1.1 +/- 0.8 |

**EXAMPLE 5** - **Molecular Dynamics**

DM simulations occurred normally for all integrin-scFv complexes. The output files from each simulation describe the trajectory of the atoms in the system, the structure data, topology and simulation parameters, the energy information throughout the simulation, and the atomic coordinates.

**Analysis of data from DM simulations**

**RMSD Analysis (Root Mean Square Deviation)**

The RSMD value refers to the average deviation of an atom, so this value should remain as small as possible and constant over time. Evaluating the RMSD over time results in an average oscillation value (DYNAMICS, 2016). Small oscillations are normal and are reflected in small RMSD values. Therefore, the higher the RMSD values the less that system will be in equilibrium. In this analysis, the atoms used for the calculation were the alpha carbons of the amino acid residues.

It can be seen that the RMSD results for the native scFv for all three integrins were similar (figure 9). The RMSD in all three cases remained low and constant. For scFv B, the values were similar, but not as uniform as for native scFv (figure 10). For scFv X the RMSD of the scFv- $\alpha4\beta1$ complex was the lowest and the most stable (figure 11), while the other two complexes showed larger and inconstant values, especially, the scFv- $\alpha4\beta7$ complex.

**RMSF Analysis (Root Mean Square Fluctuation)**

The RMSF value represents the fluctuation or flexibility of a residual from comparing the structures throughout the simulation with the structure prior to DM. Low RMSF values also indicate stability, so a region containing residues with high fluctuation will be a less stable region compared to those with low fluctuation. Therefore, secondary protein structures formed by loops tend to exhibit high fluctuation. In the case of CDRs, although they constitute regions of high flexibility, their RMSF should not be high due to the acquired stabilization resulting from the interaction with the integrin.

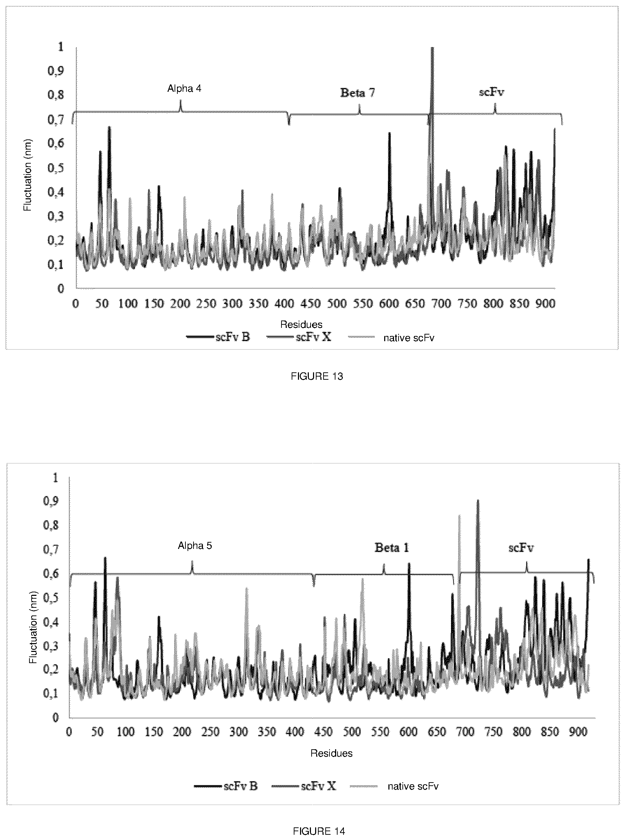

The RMSF results for the scFv - integrin complexes showed that scFv X in all three cases showed the highest fluctuation, compared to native and B (figures 12, 13 and 14). However, the fluctuation of this scFv was considerably less with integrin $\alpha4\beta1$, than for $\alpha5\beta1$ and $\alpha4\beta7$.

**Turning Radius Analysis**

The turning radius is a measure of the compaction of the protein system over time. The variation of this parameter over time indicates an expansion or contraction in the protein system. Ideally, this value should remain small and constant over time.

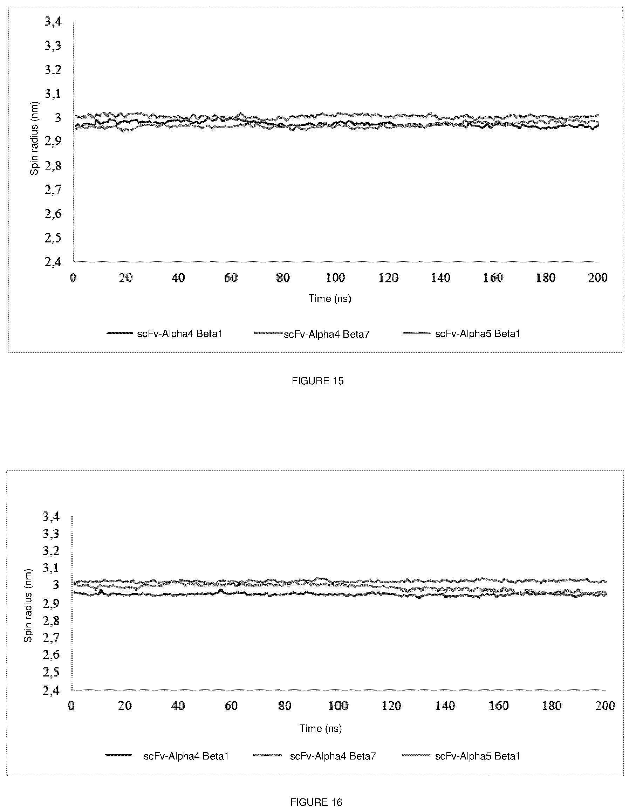

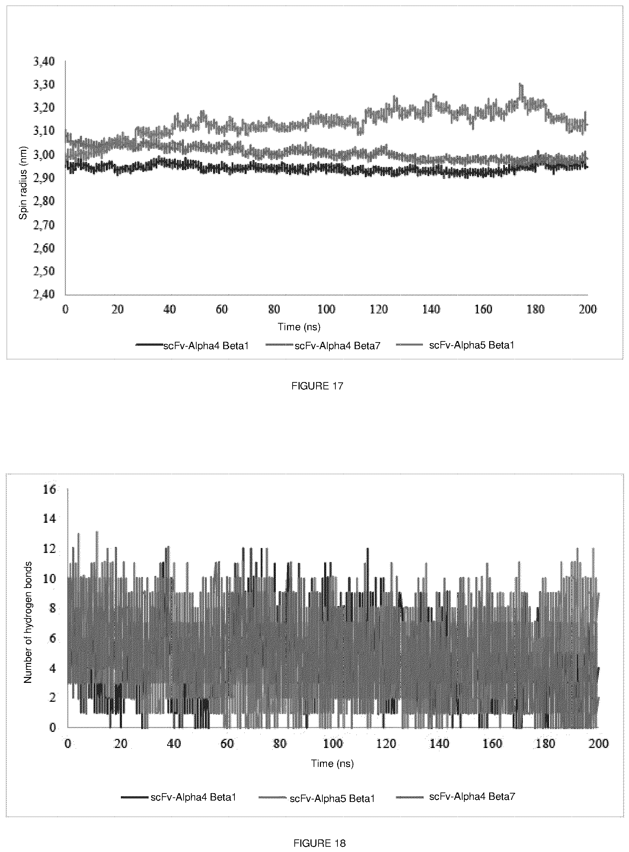

The spin radius values for the native scFv were nearly equal and constant for all integrins (figure 15). For scFv B the values for each integrin show a slight distinction, but all three remain constant (figure 16). The scFv X, on the other hand, obtained a small and constant value for integrin $\alpha4\beta1$, a higher and inconstant value for $\alpha5\beta1$, and a constant value for $\alpha4\beta7$, but higher compared to $\alpha4\beta1$ (figure 17).

The finding that the spin radius remained constant for scFv X and integrin $\alpha4\beta1$ throughout the simulation certifies that the complex formed by the three peptide chains (alpha 4, beta 1, and scFv) remained stable, with no separation of any of the chains.

**Hydrogen Bonds**

Hydrogen bonds are intermolecular interactions between two polar groups that occur between a Hydrogen atom and an Oxygen, Fluorine, or Nitrogen atom. The interaction between proteins usually relies on hydrogen bonds as components of this interaction. The greater the number of connections, the more favorable this contact will be.

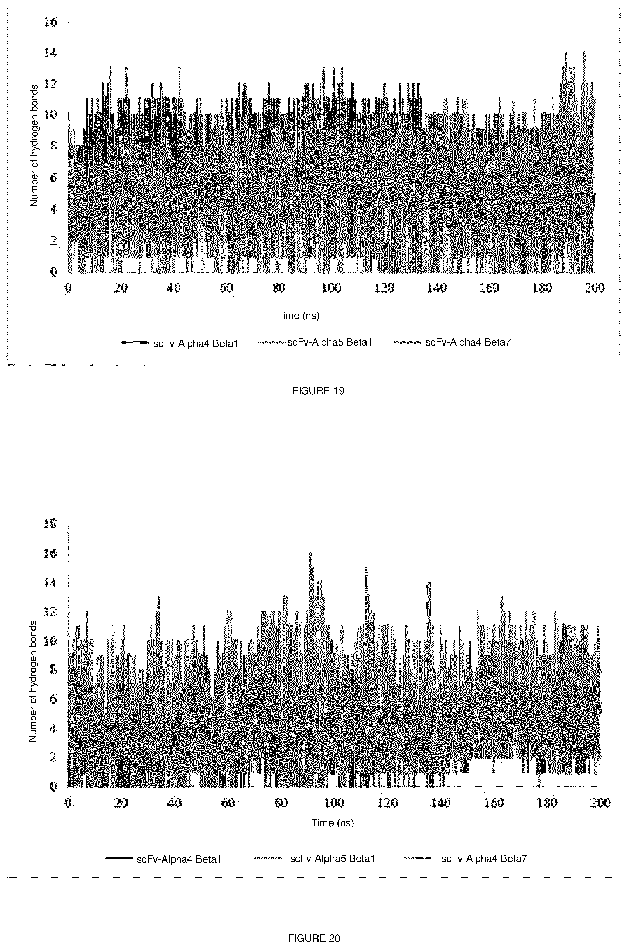

The number of Hydrogen bonds between the native scFv and the three integrins was similar, so that all averaged between 4 and 5 bonds (figure 18). For scFv B, the number of binding to integrin $\alpha4\beta1$ was the highest, averaging 7, followed by the number for $\alpha5\beta1$, averaging 6, and the lowest number was for $\alpha4\beta7$, averaging 3 (figure 19). For scFv X, the number of bonds for $\alpha5\beta1$ was the highest, averaging 6, and for $\alpha4\beta1$ and $\alpha4\beta7$ averaged 3 bonds (figure 20).

**Salt bridges**

Salt bridge type interactions are observed between amino acids with opposite charges. Therefore, the only residues capable of forming salt bridges are arginine, lysine, histidine (positive amino acids) interacting with aspartate and glutamate (negative amino acids). Just like hydrogen bonds they are determinant for the interaction between proteins. In this analysis, the salt bridges were observed with the aid of the VMD program, following the geometric criterion of up to 3.2 angstroms of distance between residues for consideration of a salt bridge.

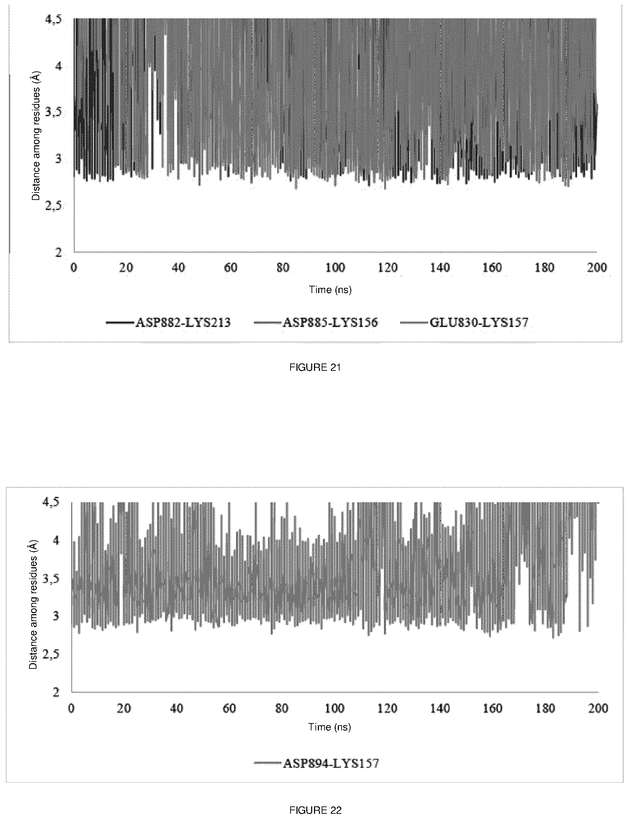

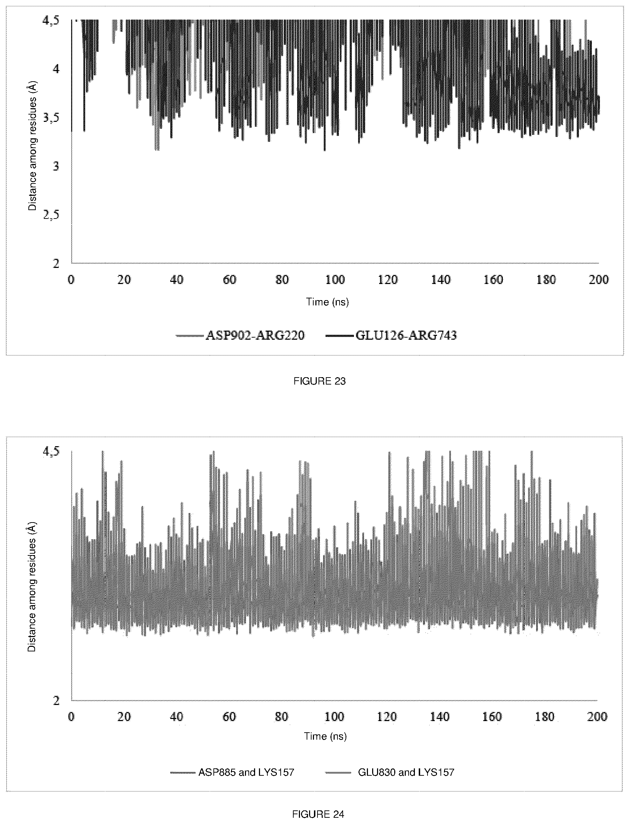

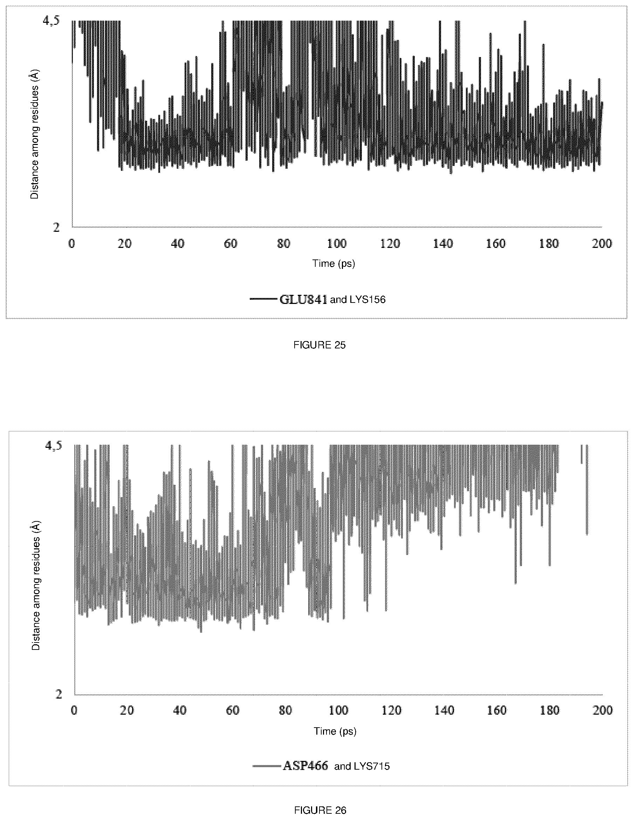

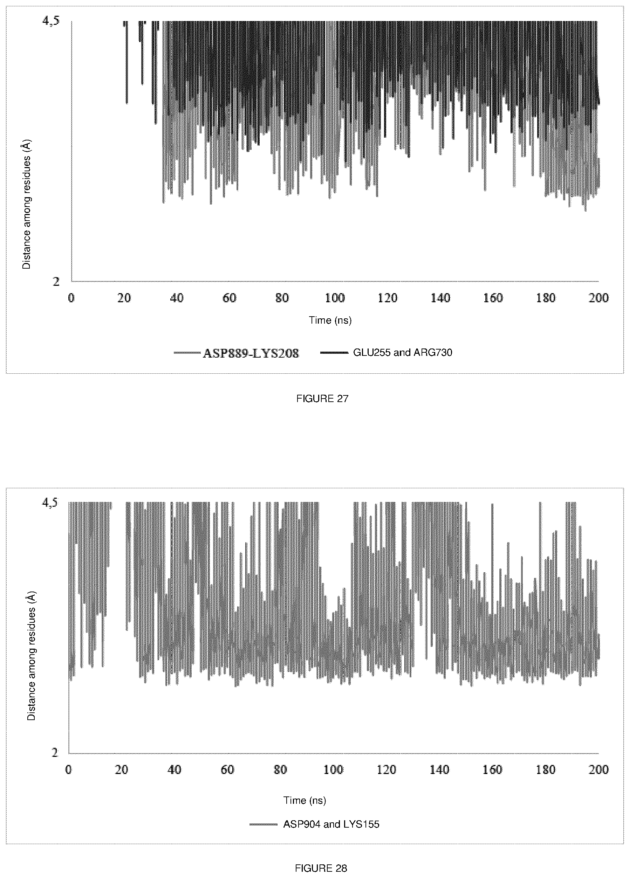

Native scFv showed a higher number of salt bridges for $\alpha4\beta1$, followed by $\alpha5\beta1$ and finally $\alpha4\beta7$ (table 11, figures 21, 22 and 23). The scFv B showed the highest number of salt bridges for $\alpha4\beta1$, and the same amount for $\alpha5\beta1$ and $\alpha4\beta7$ (table 11, figures 24, 25 and 26). However, the salt bridge between this scFv and the $\alpha5\beta1$ integrin appears to break down at the end of the simulation (figure 27). The number of salt bridges remained constant for scFv X compared to scFv B (table 11). However, the two salt bridges that form with $\alpha4\beta1$ begin after approximately 30ns of simulation.

Table 11 - Number of salt bridges between scFv and integrin observed in the simulation

| scFv-integrin complex | Number of salt bridges between scFv and integrin |
|---|---|
| native scFv - $\alpha4\beta1$ | 3 |
| native scFv - $\alpha4\beta7$ | 1 |
| native scFv - $\alpha5\beta1$ | 2 |
| scFv B - $\alpha4\beta1$ | 2 |
| scFv B - $\alpha4\beta7$ | 1 |
| scFv B - $\alpha5\beta1$ | 1 |
| scFv X - $\alpha4\beta1$ | 2 |
| scFv X - $\alpha4\beta7$ | 1 |
| scFv X - $\alpha5\beta1$ | 1 |

**Total Energy**

The total energy refers to the sum of all non-bonded interactions in the system and tends to remain constant throughout the simulation, confirming the equilibrium state. The lower the energy of a system, the more thermodynamically favorable it is.

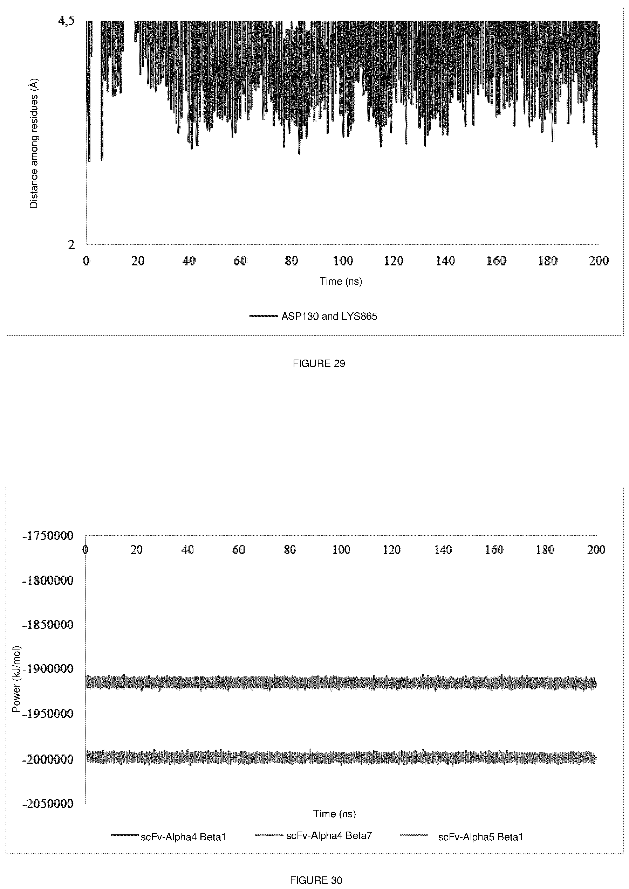

The native scFv showed a low energy value for integrin $\alpha4\beta7$, and similar energy values for the other integrins (figure 30). For scFv B, it is observed that the energy value for $\alpha4\beta7$ is still the lowest, but the energy for $\alpha5\beta1$ becomes higher compared to $\alpha4\beta1$ (figure 31). With scFv X, a decrease in energy is observed for $\alpha4\beta7$, and its value becomes approximate for $\alpha4\beta1$ (figure 32). The value for $\alpha5\beta1$ is the largest in this case.

**Coulomb Potential**

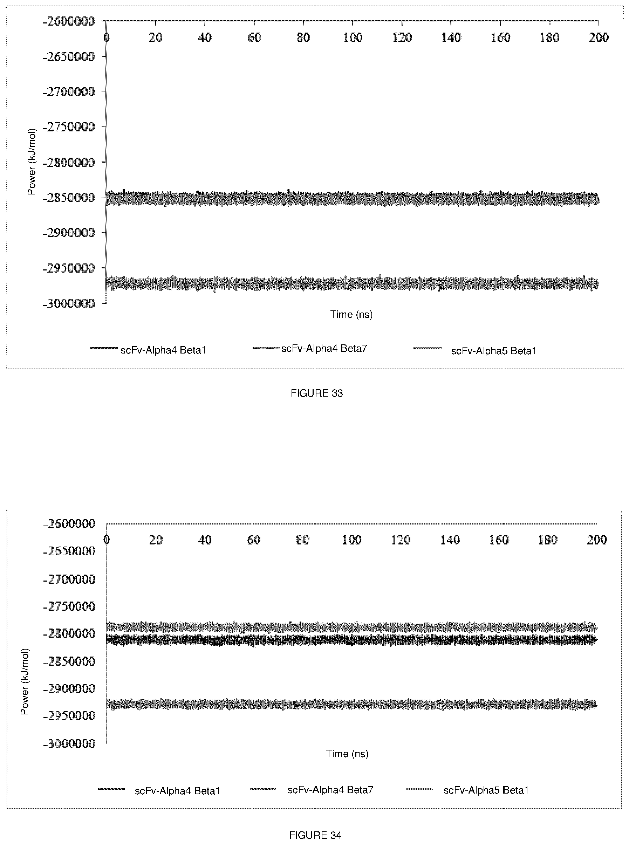

The Coulomb potential refers to the value of potential energy corresponding to electrostatic interactions. This type of interaction occurs between two electric charges, so that Coulomb's law will describe the degree of difficulty to separate these charges (MONTANARI et al., 1998).
The Coulomb potential for the scFvs and integrins showed the same behavior as observed in the total energy analysis (figures 33, 34 and 35).

**Lennard-Jones Potential**

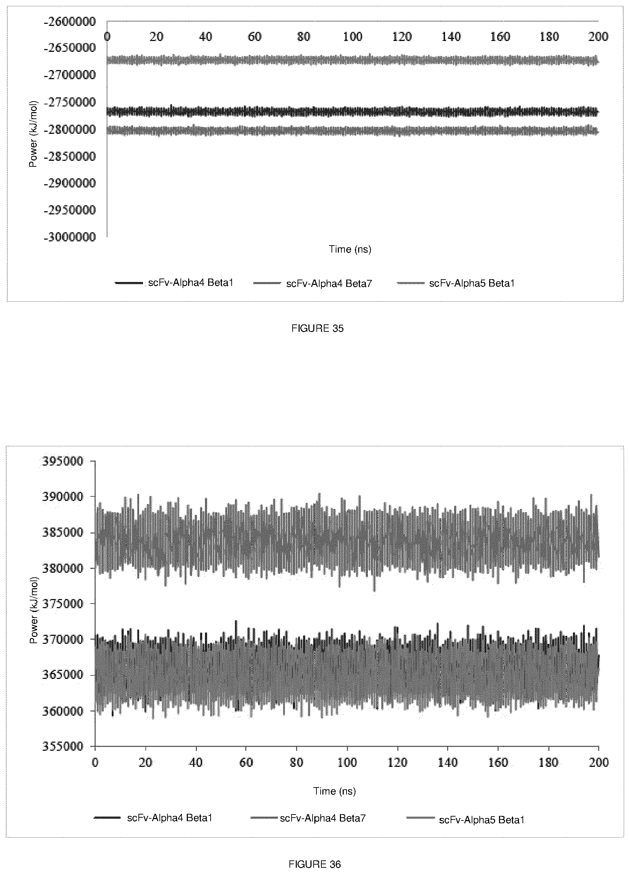

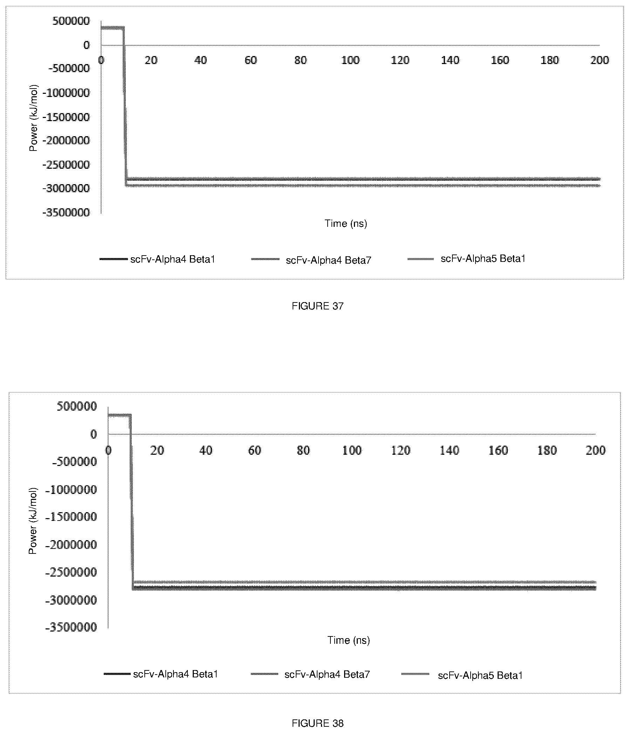

The Lennard-Jones potential deals with the energy related to Van der Waals type interactions. These interactions occur with all atomic types and are associated with the induced dipole moment. The electron density distribution that characterizes the dipoles leads to an attraction that results in Van der Waals interactions that summed together are described by the Lennard-Jones potential (ANDRICOPULO, 2016).
Native scFv showed positive values for the Lennard-Jones potential for all integrins (Figure 36). This type of bond, therefore, did not contribute to the low-energy state of the system. The native scFv showed a decay of this potential, which remained negative around 10ns for all three integrins (figure 37). In this case, the lowest energy value was for $\alpha4\beta7$, and for $\alpha4\beta1$ and $\alpha5\beta1$ it was about equal. For the scFv X, we also observed the decay of the potential, in the same time range, however the energy value for $\alpha4\beta1$ was practically equal to the value for $\alpha4\beta7$, while for $\alpha5\beta1$ this value was considerably higher (figure 38).

**EXAMPLE 6** - **Cloning of the scFv-anti-VLA-4 gene in plasmid pET28a**

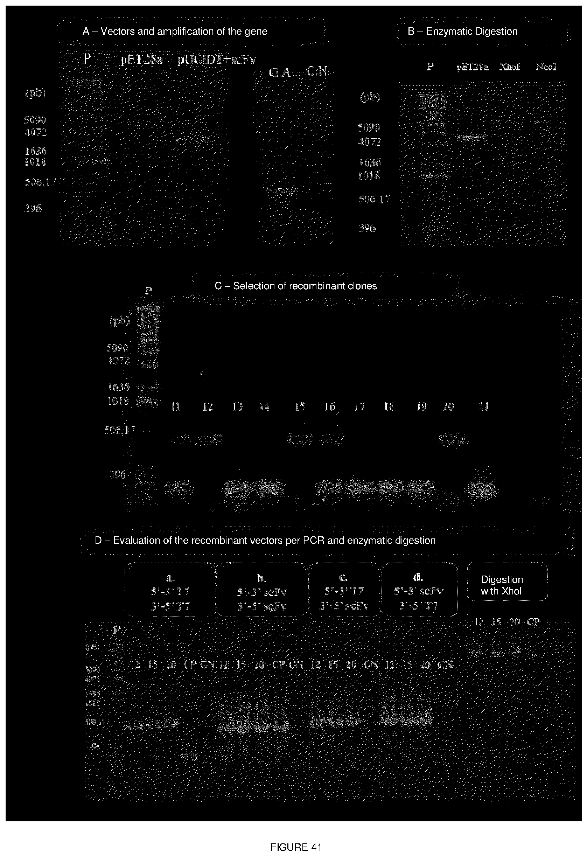

The plasmids pET28a and PUCIDT+scFv were visualized on the 1 % agarose gel, (Figure 41A). These relative molecular weights correspond to what is expected according to the manufacturer's data. After PCR amplification and insertion of restriction sites of the scFv-anti-VLA-4 gene, the resulting nucleotide fragment had a relative weight of 743 bp (Fig. 41A)
Enzymatic reaction of the plasmid pET28a with the restriction enzymes XhoI and NcoI, individually, resulted in linearization of the vector (Figure 41B). This evaluation was important for the certification of the action of both enzymes before the double digestion for cloning, enabling the ligation reaction of the linearized plasmid to the amplified gene.
After transformation of *E. coli* DH5$\alpha$ cells with the recombinant plasmid (pET28a+scFv), and performing the colony PCR procedure, it was possible to visualize a nucleotide band corresponding to the insert in clones 12, 15, and 20 (Figure 41C). Clones containing the recombinant plasmids have 743 bp between the T7 regions, while the intact plasmids have 239 bp between these regions. Therefore, it was possible to identify the recombinant clones by visualization of DNA segments of higher molecular weight, after amplification, when compared to the intact plasmids in 1% agarose gel.
After extraction of the recombinant plasmids, the presence of the insert was confirmed by different PCRs made from combinations of oligonucleotides specific for the vector and for scFv (Figure 41D). The a. reaction indicated the presence of the T7 promoter and terminator regions in all three clones, in addition to an approximately 500 bp higher molecular weight compared to the intact vector (Figure 41D). The b. reaction confirmed the presence of the insert in the recombinant plasmid of all three clones, showing a molecular weight of approximately 743 bp. Reactions c. and d. ratified the results observed in the previous reactions. Furthermore, digestion of the recombinant vector and visualization of it in a higher molecular weight region compared to pET28a, was consistent with the previous results (Figure 41D).
After nucleotide sequencing analysis, it was observed that only clones 15 and 20 showed no inconsistencies when comparing with the nucleotide sequence of scFv. Therefore, although all three clones were evaluated for scFv expression, only clone 20 was selected for further steps to produce and evaluate anti-VLA-4 scFv.

**EXAMPLE 7 - Expression of scFv in *E. coli* SHuffle®**

Evaluation of scFv expression by *E.coli* SHuffler® cells transformed with the recombinant clones 12, 15 and 20 was observed by SDS-PAGE and Western Blotting techniques. The induced protein fractions, after addition of IPTG, show a protein band of about 27 kDa, corresponding to scFv fused to the histidine tail (Figure 42). Such a protein band is not detected in the uninduced fractions, indicating that this protein was expressed after the addition of the expression inducer for T7 promoter.

**EXAMPLE 8** - **Evaluation of scFv solubility**

Evaluation of the solubility of scFv, after confirmation of expression, was critical to define purification strategies. It was observed that scFv is expressed insoluble when cells are disrupted in 20 mM Triton X-100 0.1 and 0.5 % Tris (conditions a. and b.) or in the commercial Cell Lytic® B solution, as in all cases, the recombinant protein was observed entirely in the insoluble "Ins" fraction (Figure 43.1).

The addition of agents that promote an increase in protein solubility-in this case, NaCl, L-arginine, glycerol, and MgCl2-did not alter the insolubility character of scFv (Figure 43.2). However, addition of 0.4 M L-arginine was able to induce a small solubilization of scFv, visualized by the presence of a weak protein band in the respective soluble fraction. However, the amount of insoluble protein in this condition is predominant.

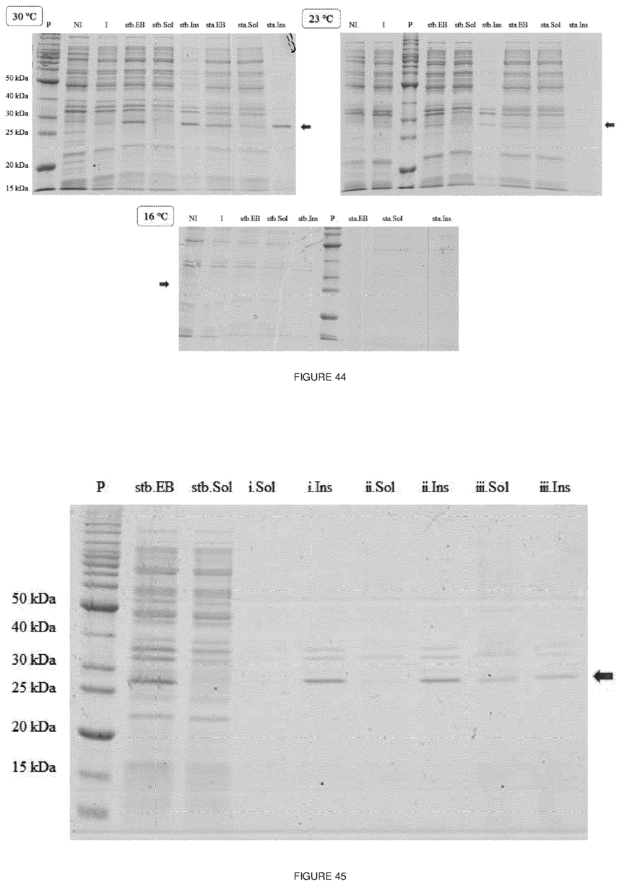

After confirming the insoluble character of the produced recombinant protein, even in the presence of potentially solubilizing substances, expression of scFv was performed at lower temperatures as a new solubilization strategy. Expression at 30 °C showed the same insolubility profile observed both after lysis in base buffer solution (20 mM Triton™ X-100 0.1 %) and in additive buffer solution (20 mM Triton™ X-100 0.5 % L-arginine 0.4 M NaCl 50 mM), and is always seen in the insoluble fractions of both solutions. Expression at 23 °C showed partial solubilization of scFv in the presence of the additive solution, and a weak protein band could be visualized in the soluble "sta.Sol" fraction. However, the amount of protein produced was much lower than that observed at higher temperatures, making it difficult to perform the next steps due to the low amount of protein. Expression at 16 °C was ineffective, so that expression of the recombinant protein could not be detected. The expression and solubility profiles at these temperatures are shown in Figure 44.

After performing solubilization strategies from adding solubilizing substances and reducing the expression temperature both were found to be ineffective. Therefore, the methodology of solubilizing the recombinant protein by adding a chaotropic agent, urea, was adopted. It was observed that only the addition of urea at the 8 M concentration was able to promote a partial solubilization of the scFv molecules (Figure 45).

**EXAMPLE 9** - **Expression media evaluation**

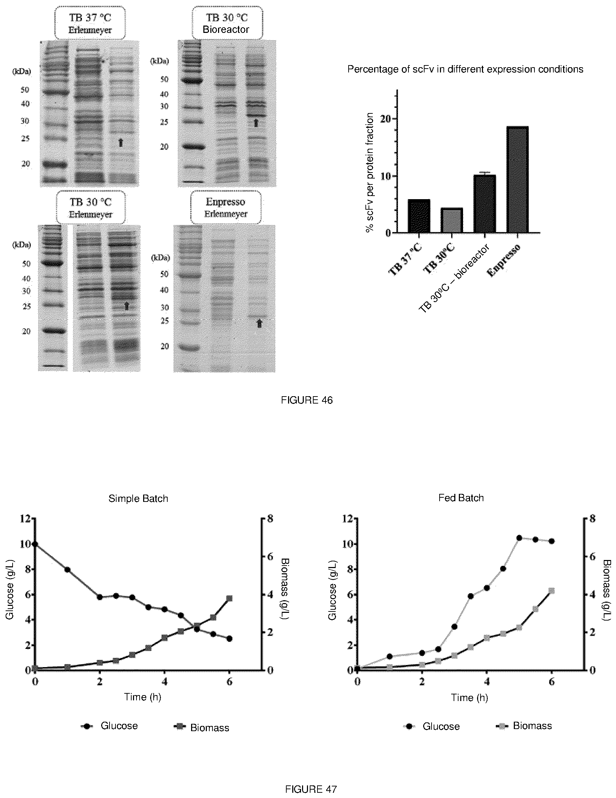

To increase the production of recombinant protein by *E.coli* SHuffle® cells transformed with pET28a+scFv, we performed the evaluation of the scFv expression profile in TB medium, at 37 and 30 °C in Erlenmeyer, microbioreactor, Enpresso medium, and Erlenmeyer at 30°C. The scFv expression performed in TB medium in Erlenmeyer flasks at 30 or 37 °C showed the same scFv production profile (Figure 46). In addition, the OD.600nm of the inoculum at the end of the expression period was 7 and 8. For production in a bioreactor, expression at 30°C was chosen because it is the recommended temperature for protein production using the heterologous system adopted. For production in a bioreactor, expression at 30 °C was chosen because it is the recommended temperature for protein production using the heterologous system adopted.

The expression in bioreactor showed twice as many proteins produced compared to the expression in Erlenmeyer flask (Figure 46) as well as twice the OD600nm (14.3) at the end of expression. This result suggests that the influence of different factors, such as container shape, aeration promoted by the air-lift system, and other parameters were more effective for scFv production and bacterial growth than temperature change.

Among the conditions evaluated, the use of Enpresso medium showed the best scFv expression profile (Figure 46), as well as the final DO600nm of 19.1. The production of scFv in Enpresso medium, therefore, in a preliminary evaluation, appears to show better performance for bacterial growth and recombinant protein production. However, this medium is difficult to scale up and manipulate in bioreactors because of its high cost and longer production time compared to other methods.

To simulate the gradual glucose release effect provided by Enpresso medium and circumvent the limitations of its use in bioreactors, we used the scFv production strategy with linear glucose feed, fed-batch, compared to production with glucose addition at the beginning of the process, single batch. Glucose consumption in the simple batch decreased with production time, while in the fed batch the glucose concentration gradually increased as predicted (Figure 47). The bacterial growth profile, measured through biomass in mg/mL, was identical for both processes, visualized by the corresponding biomass curve, suggesting in this preliminary assay that linear feeding of glucose to the medium did not influence bacterial growth (Figure 47).

In agreement with the bacterial growth profile, which was identical for both types of process, the total percentage of recombinant protein expressed was equivalent in both single and fed-batch (Figure 48). Therefore, expression of scFv anti-VLA-4 was standardized in TB medium, at 30 °C, in a bioreactor, with 2 h of inoculum growth phase and 4 h of expression and induction with 0.5 mM IPTG, without feeding, because it is a less complex procedure, compared to the fed-batch, and of comparable production.

EXAMPLE 10 - ScFv purification

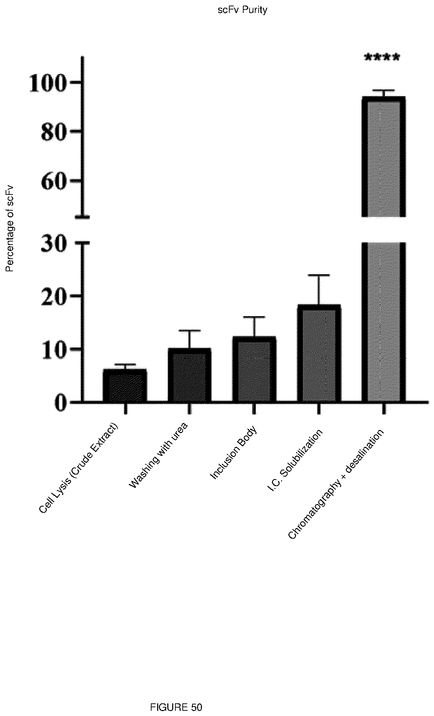

The results demonstrated the insoluble profile of the scFv molecules produced, so these proteins were expressed in the form of inclusion bodies. The washing and sonication cycles with solutions containing low concentrations of urea were able to reduce the amount of protein present in the total sample according to the washes (Figure 49.1). The purity of scFv from the crude protein extract resulting from cell lysis until the inclusion bodies were obtained increased on average by twice the initial value (Figure 50).

Purification assays demonstrated that the recombinant proteins bound to the nickel-containing matrix by the histidine tail in the C-terminal portion of scFv. Elution of the matrix proteins was possible by adding 1 M imidazole. However, a small amount of scFv can be observed that was eluted during the washes with 1 mM and 5 mM imidazole (Figure 49.2).

The purification process started at an average purity of 6 %, calculated at the initial cell lysis step. Throughout the purification steps-including the urea washes, solubilization of the recombinant protein with 8 M urea, affinity chromatography purification, and protein renaturation-the percentage of scFv in the sample increased, in line with the purification methodologies adopted, so that the final purity was greater than 90 % (Figure 50). The yield of the expression and purification process was 24.6 mg/mL of protein per liter of expression inoculum.

## EXAMPLE 11 - Increased production volume of anti-VLA-4 scFv

Expression of scFv in 2 L bioreactor showed the same recombinant protein production profile as in the previous micro bioreactor, i.e., it showed no increase or decrease in scFv expression detectable on the gel (Figure 51.1). In confirmation, it was observed that about 12 % of the total proteins visualized in SDS-PAGE gels correspond to the recombinant protein, while the previous process showed about 10 %. This result suggests that changing the aeration system - from air lift to stirred tank - and increasing the volume of bacterial production and growth did not alter the ratio of expressed proteins per volume of inoculum. However, due to the more than twice as large inoculum volume, production in this system results in a proportionally larger amount of scFv obtained.

The lysis performed in a homogenizer allowed the processing of a larger amount of biomass, compared to using the sonicator. In addition to greater efficacy and control of cell lysis. It should be noted that this method was combined with the use of Triton™ X 100 detergent, which contributes to the disruption of the plasma membrane of the cells, thus increasing the efficiency of the process.

Due to the higher amount of protein produced, in line with the increased production volume, the number of contaminants in the sample was also higher. The inclusion of a 2 M urea wash allowed a reduction of contaminating proteins, present in the soluble fractions, as observed in the SDS-PAGE gels (Figure 51.2)

The purification of scFv by IMAC chromatography using HPLC and linear gradient elution showed differences compared to the previously adopted strategy. The two-hour contact at recirculating AT was sufficient for most of the recombinant protein to bind to the resin, although some residual protein was observed in the void (Figure 51.3). Using the linear gradient, scFv was eluted from the 300 mM imidazole concentration. In the control chromatogram of the purification, the elution of scFv shows the formation of a peak, corresponding to UV absorbance at 280 nm, from 60 to 580 mAU (absorbance unit) (Figure 52). Fractions of 1 mL were collected during elution of the protein monitored by the chromatogram (Figure 51.4), joined together and forwarded for renaturation of the proteins by dialysis.

After dialysis, the concentration of endotoxins present in the sample was 36932 EU/mL (endotoxin unit per milliliter). As a result of the endotoxin reduction procedure, the concentration of these substances was reduced to 2909 EU/mL. The visualization profile of scFv on SDS-PAGE was again checked after this procedure (Figure 51.5).

The freeze-drying step of the sample was important for storage and conservation of the recombinant protein. However, reconstitution of the freeze-dried sample led to a 50 % loss of protein, so that to reach the initial scFv concentration (before freeze-drying) it was necessary to suspend the sample at half the initial liquid volume. However, after suspension the sample showed an intact profile, as expected (Figure 51.6).

The final purity of the process was, like the previous methodology, greater than 90 %. The total yield was 3 mg/L and 6 mg of scFv were obtained at the end of the process. The yield of the process as a whole was affected mainly by the dialysis and freeze-drying steps, where the largest mass losses were observed.

## EXAMPLE 12 - Evaluation of the interaction of immobilized scFv with VLA-4 on the cell surface of Jurkat cells

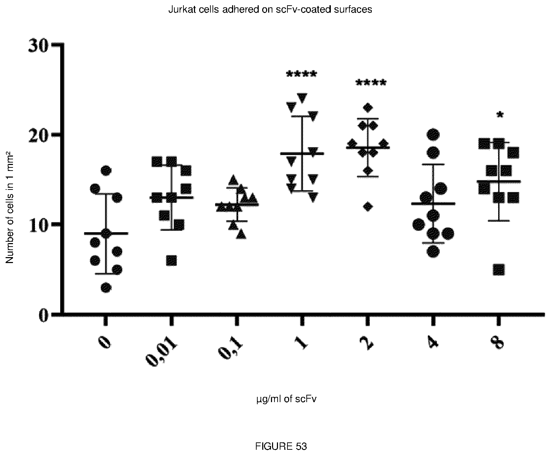

The first evaluation experiment of scFv consisted in checking its ability to interact with the target protein. To this end, Jurkart cells, which are known to express the VLA-4 receptor, were added to surfaces coated with increasing concentrations of scFv. Concentrations of 1 and 2 $\mu$g/mL of scFv induced significant increase in the number of adhered cells compared to the control (Figure 53). The higher and lower concentrations evaluated did not show a significant increase in this number, with the exception of the 8 $\mu$g/mL concentration. This result provides an indication that scFv is able to interact with a certain cell surface component, probably the integrin VLA-4. But a nonspecific interaction with other components of the cell surface cannot be ruled out.

## EXAMPLE 13 - Evaluation of the direct interaction between scFv and VLA-4 by ELISA

To verify the interaction between scFv and VLA-4 an ELISA experiment was performed. From the defined optimum concentration of 2 $\mu$g/mL, tenfold lower and higher concentrations were evaluated against 400 ng of immobilized VLA-4. Assays showed that 0.2 $\mu$g/mL of scFv showed no antigen recognition. The concentration of 2 $\mu$g/mL, on the other hand, shows an increase in signal compared to the negative control. However, the concentration where VLA-4 recognition by scFv could be determined was 20 $\mu$g/mL (Figure 54). This concentration showed a significant increase in absorbance compared to the control. This result, therefore, indicates that scFv is able to recognize the $\alpha4\beta1$ integrin, suggesting that, the approximately 50 % increase in adhered cells was a consequence of the interaction of scFv with VLA-4.

Despite the interaction between scFv and integrin visualized in the ELISA assay, this value is lower than the positive control of the experiment, the Natalizumab Fab.

## EXAMPLE 14 - Evaluation of reduced transmigration of Jurkat cells on VCAM-1 and FN coated surfaces treated with scFv anti-VLA-4

After visualizing the interaction of scFv with VLA-4 through the previous experiments, we sought to evaluate the functionality of this molecule in terms of reducing lymphocyte transmigration. We chose to keep using Jurkat cells because of their well-established transmigration pattern in this type of system, their level of VLA-4 expression, as well as finalizing with this strain a set of initial results regarding interaction and functional effect.

Using the concentration of 20 $\mu$g/mL, which performed best in 4.8, it was possible to observe that scFv reduces the number of transmigrant cells on VCAM-1 compared to the control. As expected, the inhibition of transmigration by scFv was lower compared to Natalizumab (Figure 55)

The transmigration on fibronectin showed fluctuations throughout the experiments, so that a large deviation of the values was observed (Figure 55). However, the average of the experiments suggests that both scFv and Natalizumab exhibit some transmigration inhibition effect on fibronectin.

These results, in agreement with previous findings, suggest that scFv is able to interact with VLA-4 integrin on the cell surface and exhibits a functional effect of reducing transmigration of Jurkat cells front binding VLA-4. However, to ascertain the functionality of this molecule, assays performed with primary cells are necessary.

## EXAMPLE 15 - Evaluation of adhesion and migration blockade of memory T cells treated with scFv anti-VLA-4 to VCAM-1 and FN-coated surfaces under flow

After the interaction and transmigration experiments using Jurkat cells, recombinant VLA-4 and scFv, assays using primary lymphocytes in under shear stress were performed, aiming to bring the conduct of the experiment closer to the physiological context. Thus, memory T lymphocytes obtained from healthy donors (item 3.10) were used. The choice of this cell type is related to its state, which does not require activation with cytokines and anti-CD3 antibodies, its expression of VLA-4, and its response to exposure to CXCL12 chemokines, essential to promote integrin activation and cell adhesion on VLA-4 ligands.

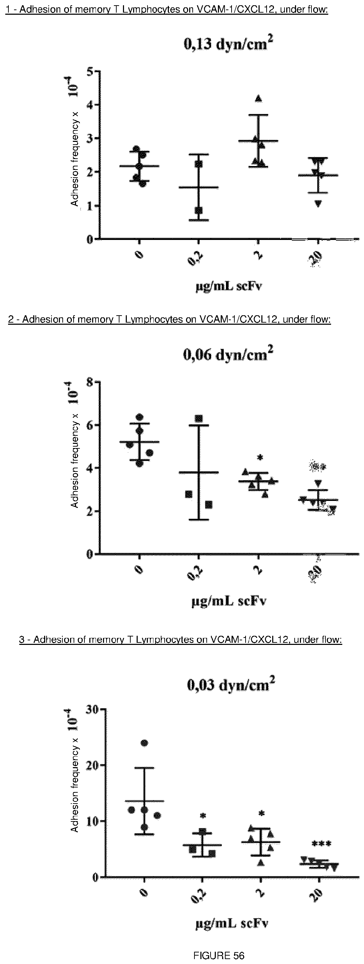

The frequency of T-cell adhesion at 0.13 dyn/cm$^2$ on VCAM-1 is not affected by using different concentrations of scFv (Figure 56.1). However, it can be seen that this frequency index for cells not treated with scFv is less expressive compared to the other flow conditions. From the 0.06 and 0.03 dyn/cm$^2$ fluxes, it is possible to see the reduction in cell adhesion frequency as the scFv concentration increases and the shear stress decreases (Figure 56.2 and Figure 56.3). These results suggest that there is interaction between scFv and VLA-4 on the cell surface of memory T lymphocytes leading to a reduction in the number of cells adhering to the surface through the interaction between VLA-4 and VCAM-1.

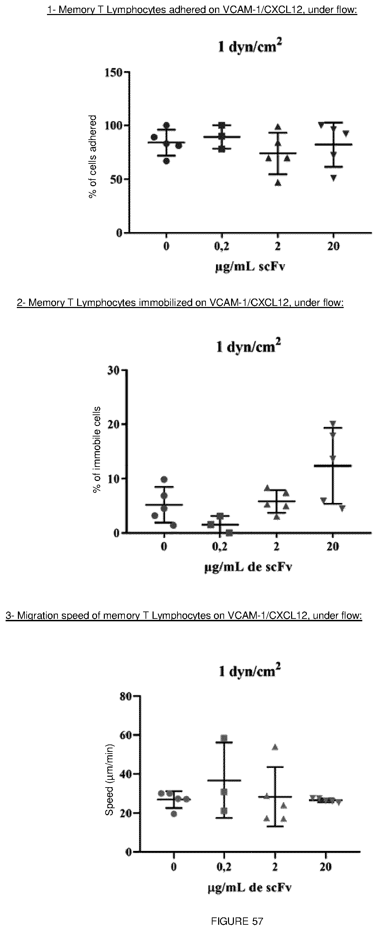

After the acquisitions regarding the observation of cell adhesion frequency, the migration, or motility, profiles of the cells that remained adhered to the VLA-4 ligands under flow were evaluated. It is emphasized that in this experiment there was no addition of new cells and that a higher shear stress is necessary to observe the permanence of these cells on the surfaces with VCAM-1 or FN under higher fluxes, as will be better addressed in Section 5. It was observed that the number of adhered cells, after the application of a 1 dyn/cm$^2$ flux, showed no differences relative to the treatment with different amounts of scFv (Figure 57.1). In other words, the treatment did not influence the ability of the cells to remain in contact with the surface after a higher shear stress was applied. The number of immobile or non-migrating cells also showed no change (Figure 57.2). Likewise, the velocity of cells migrating over the VCAM-1-covered surface was not affected with the application of scFv at different concentrations (Figure 57.3). These results suggest that treatment with such concentrations of scFv reduces the frequency of T cell adhesion on VCAM-1 but does not interfere with cell migration.

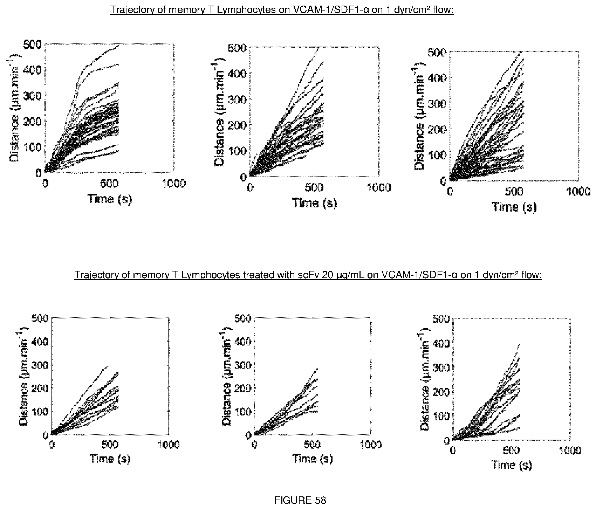

In agreement with the previous results, the trajectory, or distance traveled, of the treated and untreated cells showed no detectable differences by the trajectory profile (Figure 58). Some untreated cells are able to travel a distance of up to 500 $\mu$m/min, however, most cells show a distance traveled of about 300 $\mu$m/min, as observed in the experiments with lymphocytes treated with 20 $\mu$g/mL of scFv.

The experiments evaluating adhesion frequency and migration of cells on FN, in flow, were performed only with the 2 $\mu$g/mL concentration of scFv and with the Fab of Natalizumab at the 3 $\mu$g/mL concentration. The chosen scFv concentration was determined by experiments under flow in VCAM-1, while that of Fab of Natalizumab was determined in advance. These concentration values for scFv and Fab show minimal ability to reduce adhesion frequency on VCAM-1, so they were selected.

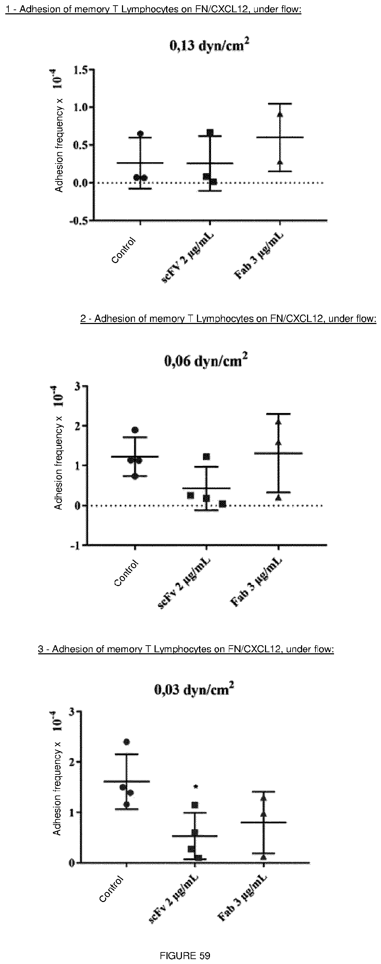

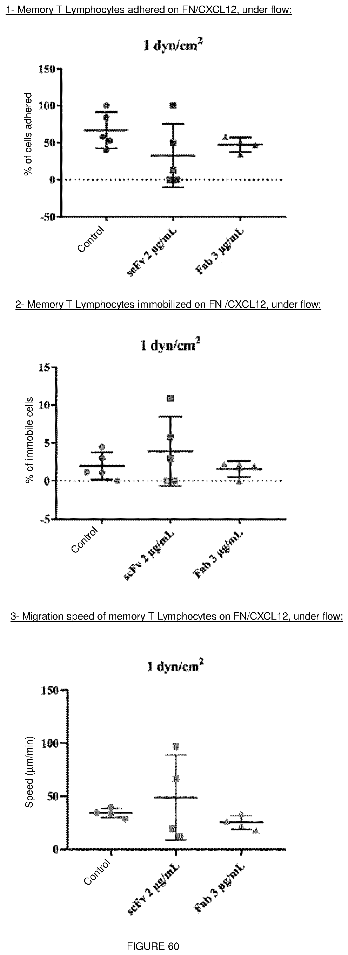

Such experiments demonstrated that the adhesion of T lymphocytes on FN (Figure 59) is lower than the values observed in VCAM-1 (Figure 56). The scFv treatment was able to significantly decrease adhesion only at 0.03 dyn/cm$^2$ flux, while Fab did not reduce this value at any of the applied fluxes (Figure 59). The migration and trajectory profiles for both treatments demonstrated widely dispersed values for all parameters evaluated, as can be seen from the percent adhesion (Figure 60.1) and migration speed (Figure 60.3) data, where the values range from 0 to 100. As a result, the visualization of the trajectories has been affected, so that in some graphs it is only possible to observe the profile of one or two remaining cells. Due to the observation of random migration patterns, i.e., with quite variable parameters, it is suggested that scFv and Natalizumab Fab do not interfere with cell migration on fibronectin.

## EXAMPLE 16 - Evaluation of scFv interference with cellular activation

The involvement of integrin VLA-4 in various cellular functions and pathologies, as well as its participation in signaling pathways, led to the verification of a possible interference of scFv treatment on cell activation, caused by VLA-4 blockade. Primary CD8+ T cells were used because of their expression of VLA-4, and the possibility of activation of these cells by treatment with IL-2 and anti-CD3 antibody to visualize the effects of scFv on cell activation.

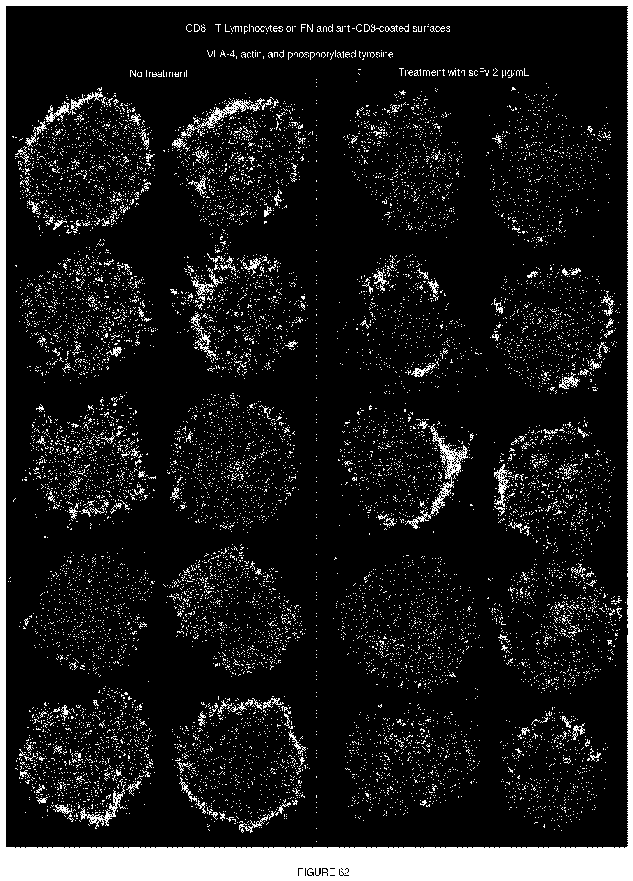

CD8+ T lymphocytes treated on fibronectin and anti-CD3 show a distribution of VLA-4 at the cell periphery, but also visualized at central points in the cell (Figure 62). The distribution of actin in these cells has a uniform trend, and due to contact with the anti-CD3 antibody, several foci of phosphorylated tyrosine are identified, suggesting activation of signaling pathways. Some of these foci are adjacent to VLA-4 located in the center, that is, away from the cell periphery. Treatment with scFv 2 $\mu$g/mL induces changes in actin distribution and amount of tyrosine phosphorylated foci (Figure 62).

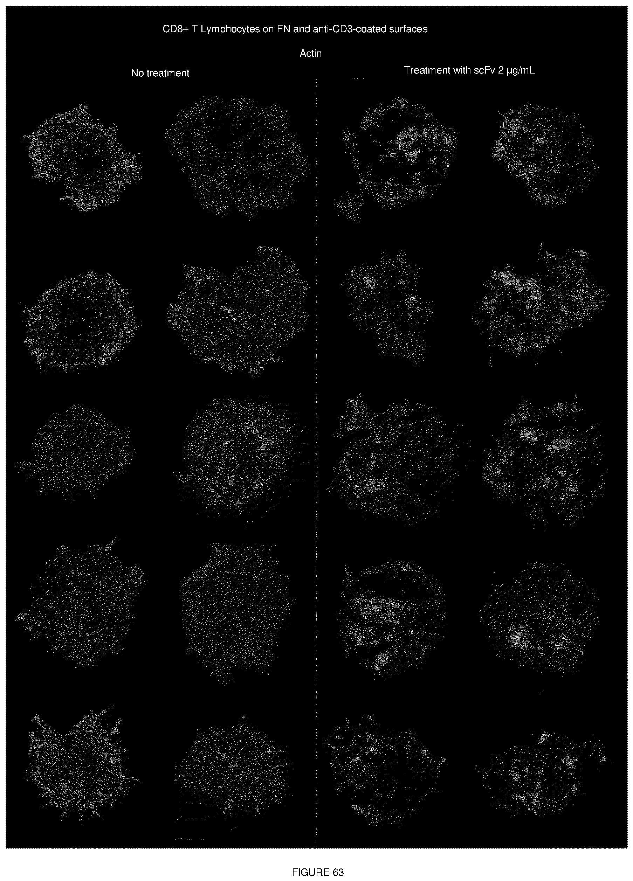

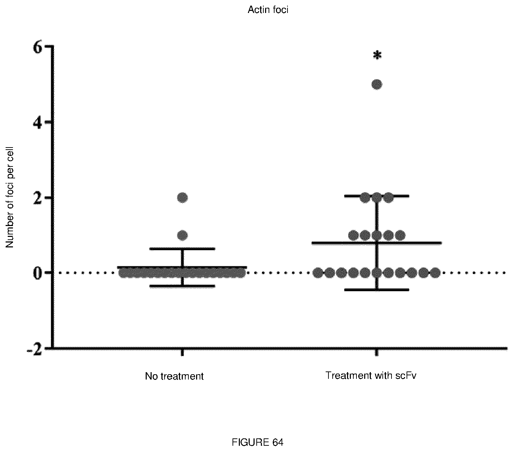

Visualization of actin distribution, independent of visualization of VLA-4 and phosphorylated tyrosine, demonstrates that scFv-treated T lymphocytes exhibit random formation of actin foci. These foci consist of a concentration of actin in certain regions of the cell, different from the homogeneous distribution observed in cells not treated with scFv (Figure 63). Quantification of these actin foci in both treatments confirmed that the number of cells with this actin distribution profile is higher in scFv-treated cells (Figure 64).

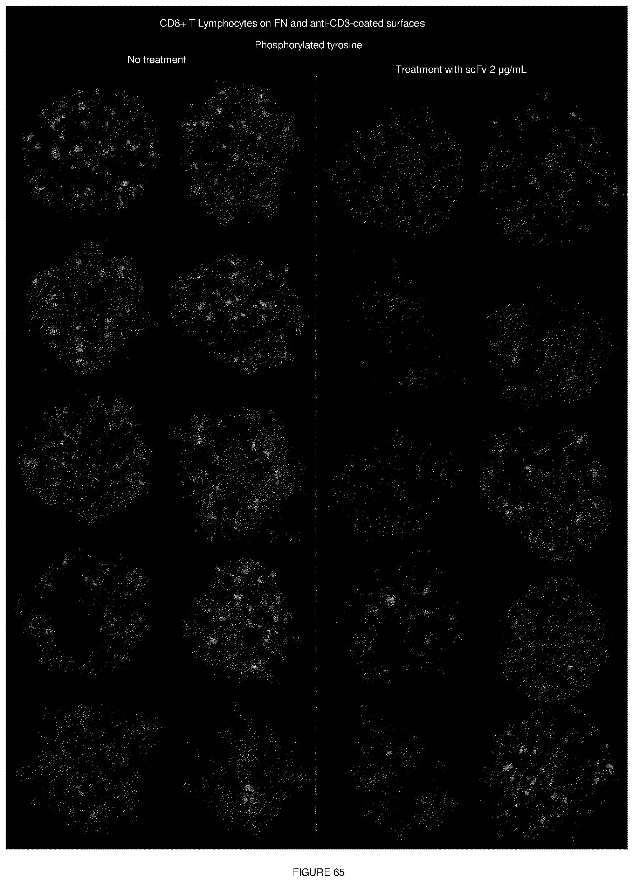

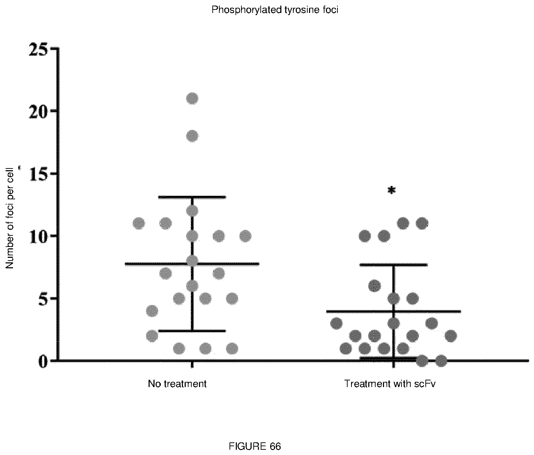

The phosphorylation of tyrosine residues of different cellular receptors is a key step for the initiation of different signaling pathways and cellular processes (SCHLESSINGER, 2000). Independent visualization of phosphorylated tyrosine foci suggests that the number of spots related to activation of cell signaling pathways, is lower in scFv treated cells (Figure 65). Quantification of the phosphorylated tyrosine foci ratifies this result (Figure 66), therefore proposing that scFv treatment may interfere with cellular activation, reducing the activation of some cell signaling pathways by blocking of VLA-4.

The results regarding actin and tyrosine phosphorylated distribution suggest that VLA-4 blockade promoted by scFv treatment causes changes in cell activation. Further studies and experiments are needed to investigate which cellular pathways and responses are potentially affected.

RMSD results in both docking and Molecular Dynamics showed that the three series of mutations that gave rise to scFv X were effective, as they affected the system equilibrium of scFv and integrins $\alpha5\beta1$ and $\alpha4\beta7$, while for integrin $\alpha4\beta1$ the system remained stable. The RMSF analysis confirms, with the RMSD result, that the interaction between scFv X and the $\alpha4\beta1$ integrin is more stable compared to the other integrins, as there was less fluctuation. The finding that the spin radius remained constant for scFv X and integrin $\alpha4\beta1$ throughout the simulation certifies that the complex formed by the three peptide chains (alpha 4, beta 1 and scFv) remained stable, with no separation of any of the chains, ratifying the RMSF and RMSD results.

The results of the Hydrogen bond analysis suggest that the second set of mutations was able to increase the number of Hydrogen bonds for $\alpha4\beta1$ and decrease for $\alpha5\beta1$ and $\alpha4\beta7$. However, the third set of mutations decreased this value to $\alpha4\beta1$ and increased it to $\alpha5\beta1$. Comparing these data to the Haddock score, Cluster size, RMSD, RMSF and spin radius results lead to the conclusion that hydrogen bonds are not the key interaction type for the binding of scFv X to integrin $\alpha4\beta1$. The salt bridge results, on the other hand, indicate that the mutations made were not favorable for making new salt bridges, but were useful in decreasing the number of bridges between scFv B and X with the integrins that are not of interest, $\alpha5\beta1$ and $\alpha4\beta7$. Therefore, these bridges contribute to the improved specificity between scFvs X and B with the $\alpha4\beta1$ integrin compared to the other two integrins.

The demonstrated energy values confirm the Cluster size value observed in docking for $\alpha4\beta7$. In other words, the fact that native scFv and B have a high Cluster size value is seen in the DM by the low energy that this system presented, which makes it more favorable. These results again ratified the need for the third series of mutations that gave rise to scFv X, which showed much more favorable energy values for $\alpha4\beta1$ compared to the other scFvs. Although this value is still somewhat higher than the value for $\alpha4\beta7$, the energies of the unbound interactions along with other factors, such as salt bridges for example, contribute to the interaction of scFv X with integrin $\alpha4\beta1$ standing out compared to the others.

Electrostatic energies, seen in the Coulomb Potential analysis, presented themselves as a key component of the scFv X-integrin $\alpha4\beta1$ interaction. Therefore, the mutations performed favored this type of interaction. Furthermore, the mutations performed led to the contribution of Van de Waals bonds in the total energy of the scFv - $\alpha4\beta1$ system, as can be seen from the results of the Lennard-Jones Potential analysis.

After interpreting the DM and docking results, it is observed that the parameters of RMSD, RMSF, Spin Radius, Salt Bridges, Total Energy, Coulomb Potential and Lennard-Jones Potential analyzed together are consistent with the result of docking for scFv X. Therefore, after the three series of mutations there was an increase in the specificity of scFv for integrin $\alpha4\beta1$.

By 2012 there were eight drugs approved and used for the treatment of multiple sclerosis and seven potential drugs in development (GOLDENBERG, 2012). The eight types of drugs currently used are: Beta interferons, Glatiramer acetate, Mitoxantrone, Fingolimod, and Natalizumab. All these drugs have advantages and disadvantages associated with their use. Therefore, it is valid to compare scFv X, a potential drug for the treatment of multiple sclerosis, with existing therapeutic tools.

Beta interferons are cytokines naturally produced by the immune system. Several mechanisms for its therapeutic action have been proposed, such as inhibition of T-cell activation and proliferation (DHIB-JALBUT; MARKS, 2009). Despite the effectiveness of these molecules in reducing inflammatory damage, their application involves the risk of developing leukopenia, depression, and thyroid disease (GOLDENBERG, 2012). scFv X, in theory, would act by blocking the interaction of VLA-4 with VCAM-1 proteins, therefore, only preventing the migration of T lymphocytes to the site of inflammation causing multiple sclerosis.

Glatiramer acetate is a type of polymer initially synthesized to simulate multiple sclerosis models (ACETATE... 2016). Although its exact mechanism of action is unknown, it is known to act differently from beta interferons. However, this drug is recommended for the initial treatment of the disease and for patients who cannot tolerate beta interferons (KP et al., 1988). Theoretically, scFv X could be used in more advanced stages of the disease by directly blocking the action of T lymphocytes on the myelin sheath.

Myxantrone is an immunosuppressant, which acts on B and T cells and macrophages (GOLDENBERG, 2012). Although it can be used even in more advanced stages of the disease, the suppression of immune system cells makes the body fragile and exposed to pathogens. B-cell suppression, for example, interferes with the production of antibodies, important defense molecules. The target of scFv X is found only in T lymphocytes and monocytes, which, except in cases of infection, is found in low amounts circulating in the blood. Therefore, its application would not affect the immune system as Myxantrone does.

Fingolimod is an immunomodulator that was the first orally administered drug for multiple sclerosis and (GOLDENBERG, 2012). Despite its effectiveness, its use increases the risk of infection and macular edema. There is no way to directly compare scFv X with fingolimod, because since there are not in *vitro* and *in vivo* tests yet, the side effects that this potential drug may cause are not known. However, it is worth mentioning that scFvs in general have reduced immunogenicity and short retention time in the body (AHMAD et al., 2012).

As already mentioned, Natalizumab is a monoclonal antibody that interacts with integrins $\alpha4\beta1$ and $\alpha4\beta7$, the application of which is associated with the risk of PML. It is known that there are populations of lymphocytes that possess integrins of the $\alpha4\beta1$ and $\alpha4\beta7$ type. Studies indicate that blocking $\alpha4\beta1$ integrins, but not $\alpha4\beta7$ integrins, prevents leukocyte infiltration into the CNS (HAANSTRA et al., 2013). Therefore, the application of scFv X would prevent the passage of lymphocytes that have VLA-4 into the CNS but would allow some T cells to continue to be able to migrate normally. That is, the presence of a circulating T-lymphocyte population could prevent PML recrudescence. Therefore, scFv X would be able to prevent the damage to the myelin sheath caused by the erroneous migration of T lymphocytes into healthy tissue but would allow there to be circulating cells able to perform the body's defense by preventing PML.

Two other steps in antibody development were addressed: production and *in vitro* functional evaluation. In the production stages, aspects concerning the scFv production and recovery procedures were mainly evaluated. In the evaluation steps, we evaluated the interaction of scFv with VLA-4, and its effect on transmigration, adhesion and flow migration of T lymphocytes (lineage and primary) on VLA-4 ligands, as well as partially observing its effect on cell activation.

During the production steps, we observed that scFv was expressed in an insoluble form under the initial conditions tested. In fact, the expression of recombinant proteins in *E.coli* has as one of its main limitations the production of inclusion bodies. One possible strategy to increase the solubility of these proteins is to alter environmental factors, such as reducing the expression temperature. Studies related to the expression of the soluble form of scFvs, in this case an anti-TNFα scFv, demonstrated that expression at temperatures around 10°C was beneficial for the production of soluble recombinant proteins (Sina et al, 2015). In the case of the anti-VLA-4 scFv proposed in this work, reducing the expression temperature had a negative impact on its production by *E. coli* cells, due to reduced or inhibited expression of the molecule, so that partial solubilization effects are irrelevant due to the low amount of protein produced.

The addition of additives-such as salts, amino acids, sugars, detergents, and other substances-during cell lysis is another way to improve the solubility of recombinant proteins (Leibly et al, 2012). The use of different additives (glycerol, MgCl2, NaCl and L-arginine) showed no noticeable effects on the solubility of the produced scFv. However, the addition of L-arginine and NaCl to the buffer solutions of the purification steps was essential to reduce precipitate formation and therefore improve the stability of scFv in solution. The use of L-arginine is related to reducing the interactions between the scFv molecules, preventing them from forming aggregates and precipitating in solution. In addition, the use of this amino acid assists in the protein's coagulation during the renaturation steps and in stabilizing the molecule during storage. Addition of NaCl, on the other hand, increases protein solubilization due to the salting-in effect (Xu et al, 2015).

Despite this (detailed in the paragraph above), The addition of chaotropic agents, such as urea, was necessary to promote the solubilization of the scFv molecules present in the inclusion body structures. Substances such as urea and guanidine interact with the proteins and promote a linearization of the proteins by forming a layer around the proteins. The addition of these agents causes, in addition to solubilization, reversible denaturation of the protein (Salvi et al, 2005). The use of chaotropic agents such as urea, however, leads to a loss of mass from the addition of processes to recover the functional recombinant protein.

The selection of the *E. coli* SHuffle® strain to obtain the scFv was due to the characteristics of these bacteria that are favorable for the expression of proteins that have disulfide bridges. These bacteria have an oxidative cytoplasmic environment, compared to other *E. coli* strains, due to the deletion of two reductases and the constitutive expression of a disulfide isomerase. The oxidative cytoplasm prevents scFv expression from necessarily being redirected to the periplasm of the cell and is conducive to disulfide bridge formation, and thus protein functionality (Lobstein et al, 2012). Indeed, the functional evaluation experiments of scFv showed good indications, suggesting the presence of its two disulfide bridges, which are essential for the maintenance of its tertiary structure and interaction with integrin VLA-4.

Other factors evaluated were the yields of the scFv production processes before and after the production increase, whose values were 24.6 and 10 mg/L, respectively, until the renaturation step. Studies related to scFv expression in *E. coli* show variable yield values, such as 2 mg/L (Xiong et al, 2009) and 35 mg/L (Dominici et al, 2014). A work by Zarschler (Zarschler et al, 2013) and collaborators demonstrated the production of two scFvs, cloned in plasmid pET28b, expressed in *E. coli* SHuffle®, in different media: LB, TB and Enpresso® in an Erlenmeyer flask. The authors observed that the ODs of the biomass at the end of the expression process ranged from 8.3 to 10.7 for the TB medium, and 13.1 to 19.8 for the Enpresso medium. The ODs obtained for scFv anti-VLA-4 expression in our work were 8 for expression in TB made in Erlenmeyer flask, 14.3 for TB in bioreactor and 19.1 for Enpresso® medium. Thus, it is observed that the data regarding bacterial growth of scFv anti-VLA-4 expression processes are consistent with results observed in the literature. However, when comparing the yield data, it is observed that the values obtained by the mentioned work are higher than 100 mg/L (Zarschler et al, 2013), that is, about five to ten times higher than the yield values obtained for the anti-VLA-4 scFv. It is suggested that one of the factors that may influence this difference in values is the solubility of the protein and the purification steps, because the scFvs evaluated by Zarschler and collaborators (Zarschler et al, 2013) are in the soluble protein fraction and do not require the addition of urea for solubilization.

The enzymatic process that leads to the gradual release of glucose into the culture medium is one of the main characteristics for the Enpresso® B medium and its high performance. The use of this commercial medium, which in theory mimics a feeding process, provides high yields relative to biomass and recombinant protein production (Ukkonen et al, 2017). Performing a linear fed-batch process of glucose in TB medium for the production of anti-VLA-4 scFv, as an attempt to simulate the enzymatic process of Enpresso medium, showed no positive or negative changes in the generation of biomass or recombinant protein. It is suggested from this preliminary data that only continuous and gradual feeding of glucose into the medium, for the system described in this work, is not sufficient to induce an increase in biomass and scFv production. Other components of the commercial medium can influence its biomass and recombinant protein generation performance. However, we can consider that the way the fed batch process was conducted in this work may not have been ideal, because there was no optimization and in-depth study of this process, and therefore, other ways of conducting the process may result in better productivity.

After performing the yield increase process, we obtained 6 mg of protein at the end (after freeze-drying and reconstituting the samples), while the previous process provided only 1.72 mg of protein in its last step (renaturation). Therefore, the use of higher volumetric capacity methodologies-such as the 2 L bioreactor, homogenizer, and HPLC-allowed an increase in protein yield. Due to the high amount of protein that is required to perform the functional assays, the use of these methodologies was satisfactory to meet such demands. In addition, the increase in production allowed the elaborate scFv batches to be made in a more reproducible and automated manner, which are also important features for performing functional assays.

Functional ELISA and transmigration assays in Transwell chambers provided an initial assessment of the interaction profiles of anti-VLA-4 scFv, with VLA-4 integrin. In these experiments, Natalizumab or Natalizumab Fab were used as controls. In both experiments, the performance of Natalizumab or Fab was superior to scFv. This result is expected, as the Fab molecule and Natalizumab (IgG) have affinities for VLA-4 integrin on the cell surface - as measured by Kd, dissociation constant - of 6.4 nM and 0.28 nM, respectively (Yu et al, 2013). The scFv molecule, on the other hand, has an *in silico* estimated Kd of 0.55 nM for VLA-4. It can be seen that the estimated affinity for VLA-4, based on the theoretical Kd, for scFv is approximately two times lower than for Fab. This difference in values stems from the rational development stage of scFv, as the construction of a molecule with higher specificity for integrin $\alpha4\beta1$, and not necessarily affinity, was prioritized (Chaves, 2016).

The ELISA experiment demonstrated a signal relative to the recognition of recombinant VLA-4 by Natalizumab Fab that was almost ten times higher than the recognition by scFv. The observed divergence can be justified by three main factors: difference in affinity, type of determining epitope - linear or structural - and limitations of the technique related to the application of the peroxidase-conjugated antibody for the development, which was not optimal for scFv development. The difference in affinity was discussed in the previous paragraph. The type of epitope determinant refers to the recognition region of both molecules. Natalizumab and Fab interact with VLA-4 through binding to amino acids of the $\beta$-sheets and loop regions present in the $\beta$-propeller structure of the $\alpha4$ subunit (Yu et al, 2013), while scFv recognizes regions present between the interface of the two subunits, interacting simultaneously with $\alpha4$ and $\beta1$. This suggests that the interaction of scFv with integrin is associated with the structural determinant type of epitope in an evidenced manner compared to Natalizumab or Fab. Therefore, performing interaction assays between scFv and VLA-4 is more susceptible to variables that alter integrin structural dynamics, affecting recognition, due to the type of determinant of this interaction.

When compared with Natalizumab, the affinity of scFv for VLA-4 is theoretically ten times higher. However, Natalizumab is a full IgG-type antibody, meaning that it is able to interact with two $\alpha4$ molecules simultaneously, and therefore possesses higher avidity. This difference in avidity matches the results observed in transmigration on VCAM-1, where Natalizumab shows a greater ability to block cell transmigration (of Jukart cells) than scFv. In addition, because it is a commercial antibody and used for therapeutic purposes, this antibody has a higher stability compared to scFv, influencing its performance and the reproducibility of the data.

In the inflammatory context, blocking the interaction between VLA-4 and VCAM-1 is critical to reduce the passage of leukocytes into the target tissue, given the participation of this molecule in this process (Hyduk & Cybulsky, 2007). In this work, we observed that scFv was able to reduce the transmigration of Jurkat cells on VCAM-1, this being one of the first indications for the potential of this molecule. The data on transmigration over FN, another integrin ligand, showed great variability - consistent also with the results of flow migration of memory T lymphocytes over FN.

The FN molecule has different interaction sites for different integrins and may have more than one site for the same integrin, as well as shared interaction motifs (Pankov & Yamada, 2002). FN has different isoforms according to its location - ECM or blood plasma - a factor that also interferes with FN recognition by integrins and its functionality. In the transmigration, adhesion reduction and migration experiments the blood plasma FN was used to provide a general idea of the performance of scFv and Natalizumab or Natalizumab Fab, since this molecule is also an important ligand of VLA-4. In the case of scFv, we found a reduction in memory T lymphocyte adhesion and Jurkat cell transmigration on FN. In order to confirm the effect of scFv on cellular interaction with FN, experiments using other FN isoforms, extracted from ECM, should also be performed, since the structural and functional differences of this molecule (To & Midwood, 2011) may provide distinct responses and aid in the interpretation of the results regarding blocking the interaction between VLA-4 and FN, in addition to tests with plasma FN.

The reduced adhesion of memory T lymphocytes to VCAM-1 under flow is, in conjunction with the transmigration results, an early indication of the potential use of scFv as an anti-inflammatory biopharmaceutical in chronic diseases. The VCAM-1 molecule is directly associated with pathologies of a chronic inflammatory nature, in addition to other diseases such as cancer (Kong et al, 2018). Therefore, blocking the interaction between VLA-4 and VCAM-1 is a therapeutic strategy, already adopted, as is the case with Natalizumab (Kummer & Ginsberg, 2006). The blocking of this interaction, suggested by the flow-through adhesion frequency reduction experiments, provides a further indication that anti-VLA-4 scFv has the potential to be evaluated in the future in preclinical and clinical phases as a biopharmaceutical.

Data in process of publication, obtained by researchers from the Laboratory of Adhesion and Inflammation (INSERM 1067, Marseille, France), have shown that Natalizumab and Natalizumab Fab are able to reduce the frequency of adhesion of memory T lymphocytes on VCAM-1 by up to tenfold compared to control, while scFv reduces this value by about two to three times. The lower inhibitory capacity of scFv than that of Natalizumab and its Fab should be evaluated and may be beneficial in terms of safety related to PML (Progressive Multifocal Leukoencephalopathy).

Different factors causing immunodeficiency are associated with the onset of PML, so treatment with natalizumab could be one of the factors causing this disease. In general, affected patients are observed to have a deficiency of CD4+ and CD8+ T lymphocytes (Pavlovic et al, 2018), increasing their vulnerability to disease. Both cell types are necessary for containment in disease (Gheuens et al, 2011), however, CD4+ T cells, mainly Th1, are especially important for coordinating CD8+ T responses (Jelcic et al, 2016).

Natalizumab application in MS patients affects CD4+ and CD8+ T lymphocyte activity, interfering with the cellular immune response and causing susceptibility to PML in patients who have JC virus (Gheuens et al, 2011). Thus, it is hypothesized that the lower inhibition potential of VLA-4, compared to Natalizumab, may present an advantage. Such a hypothesis assumes that the reduced blockade of VLA-4 present in lymphocytes would allow some cells with non-blocked integrins to act normally, providing a certain protective effect in PML containment.

The passage of lymphocytes from the blood vessel to the target tissue has as one of the main interactions, the binding of VLA-4 with VCAM-1. On the other hand, the interaction of VLA-4 with FN is related to the permanence of cells in inflamed tissue, and in some diseases such as rheumatoid arthritis (Laffon et al, 2019). Although the adhesion frequency of T lymphocytes, under flow, on surfaces with FN is reduced compared to VCAM-1, a decrease in these adhesion values can be observed after the application of scFv. This decrease is positive for the application of scFv as a biopharmaceutical for cancer treatment, especially in the prevention of metastasis, since FN is one of the mediating molecules in the formation of the pre-metastatic niche in organs such as lung, liver, kidney and others (Schlesinger & Bendas, 2015). As for the performance of Fab in the flow experiments on FN, it is concluded that the interaction of such an antibody fragment exclusively with the $\alpha4$ subunit did not show significant effects of reducing adhesion frequency. In hypothesis, this effect is assumed to be related to the type of recognition of the $\alpha4$ subunit by Fab, as it is seen that interaction between FN and VLA-4 is also mediated by the $\beta1$ subunit (Sánchez-Aparicio et al, 1994).

The interaction of VLA-4 with its ligands is thought to be fundamental to cell motility-related processes-such as migration, adhesion, and spreading or shape-shifting (Wu et al, 1995). Treatment of memory T lymphocytes with scFv showed no changes in the overall profile of cell migration and motility, on FN and VCAM-1 in flow. The absence of significant variation in speed, trajectory, permanence on surfaces, and immobilization is presented as a favorable aspect for the application of the scFv anti-VLA-4 molecule, because cell motility is necessary for certain immunological processes to occur, such as the traffic of lymphocytes to the lymph nodes, where antigen presentation occurs (Wei et al, 2003), and is important during embryogenesis and healing processes (Franz et al, 2002). The scFv, therefore, affects cell transmigration but not VLA-4-dependent cell motility and migration, this being a further indication that such an antibody fragment has an anti-inflammatory potential, since, in theory, this molecule is able to block the passage of T lymphocytes to the target tissue by blocking the interaction with VCAM-1, but does not influence the motility aspects of these cells.

Studies by Mittelbrunn and co-workers demonstrate the presence of the integrin VLA-4 at the periphery of the immune synapse during the process of antigen presentation and cellular activation, and a change in the profile of cytokine production according to the blockade of the integrin at different epitopes (Mittelbrunn et al, 2004). Such studies demonstrate that VLA-4 is related to the process of cellular activation, during immune synapse formation, and that its blockade causes different cellular responses according to the integrin recognition region, inducing activation or repression of different signaling pathways. Indeed, treatment of CD8+ T lymphocytes with anti-VLA-4 scFv, under conditions that simulate and induce immune synapse formation, affects cell activation and actin distribution. Despite the apparent alteration of actin, previous results regarding cell migration have shown that scFv does not affect T lymphocyte motility on VCAM-1 and FN. It is suggested that the formation of the actin foci, together with the decreased formation of phosphorylated actin spots, are related to altered signaling pathways due to VLA-4 blockade by scFv, leading to a differentiated cellular response. Further studies will be needed to identify which pathways are affected, how scFv and VLA-4 blockade influence the cellular response, and how these response profiles are contextualized in the treatment of a chronic inflammatory disease.

Despite the different strategies that can be adopted for the development of drugs and biopharmaceuticals, such a process is time-consuming and requires great care. The Natalizumab antibody, for example, was developed by Ted Yednock, who presented his work on an anti-$\alpha4$ antibody in 1992, and underwent clinical evaluations between 1999 and 2003 (Schwab et al, 2015). In 2004, was approved for use in the treatment of MS, and in 2008, for the treatment of moderate to severe Crohn's disease, that is, about fifteen years were needed for the complete elaboration of this drug. The effects of Natalizumab regarding inhibition of lymphocyte transmigration, achieved during the development processes of this antibody, serve as parameters for the development of VLA-4 inhibitors. However, factors concerning the safety and specificity of this inhibitor must be observed.

Studies show that blocking integrin VLA-4, not LPAM-1, is sufficient to reduce leukocyte infiltration into the CNS and ameliorate the condition of experimental autoimmune encephalomyelitis (Csizmadia et al, 2013). The proposal of anti-VLA-4 scFv is to be specific for this integrin, which would avoid unnecessary inhibition of LPAM-1-mediated leukocyte transmigration. However, in vitro experiments, to confirm the specificity, and in vivo experiments, to evaluate its blocking potential in reducing the clinical score of the disease, are still needed to advance this molecule as a biopharmaceutical.

As conclusions obtained from the work performed it was obtained that:

1. The *in silico* methodology employed to promote the mutations was effective, so that the effectively engineered scFv showed an increased ability to interact with α4β1 integrins.

2. The determinant interactions that favored the interaction of scFv X with α4β1 integrins were salt bridges, electrostatic and Van der Waals interactions.

3. Comparative antibody modeling is a viable alternative for obtaining the three-dimensional structure of these molecules, because it is possible to use templates with high identity.

4. It is recommended to perform Molecular Dynamics and *docking* simulations during the design phase of a drug, as these techniques provide important information about the behavior and interaction form of target molecules.

By employing conventional methodologies for obtaining recombinant proteins, we generated an scFv-like antibody fragment targeted to VLA-4 recognition. The functional results show that scFv interacts with VLA-4 and is able to inhibit adhesion and transmigration of T lymphocytes on ligands of this integrin, without affecting cell motility. In addition, blocking VLA-4 with scFv appears to interfere with cell activation, due to the observation of changes in the patterns of phosphorylated tyrosine foci and actin distribution.

The present work provides a new tool for blocking VLA-4 targeting applications in the treatment of chronic inflammatory diseases by reducing leukocyte transmigration to sites of inflammation.

REFERENCES

ABBAS, Abul K. Imunologia Celular e Molecular. Rio de Janeiro: Elsevier, 2015.

ACETATO de Glatiramer (Copaxone ®). Available from: <https://esclerosemultipla.wordpress.com/2006/05/09/acetato-de-glatiramer-copaxone-®/>. Access at: Oct. 05, 2016.

AHMAD, Zuhaida Asra et al. ScFv Antibody: Principles and Clinical Application. Clinical And Developmental Immunology,[s.l.], v. 2012, p.1-15, 2012. Hindawi Publishing Corporation.

ALTSCHUL, Stephen F. et al. Basic local alignment search tool. Journal Of Molecular Biology, [s.l.], v. 215, n. 3, p.403-410, out. 1990. Elsevier BV.

ANDRICOPULO, Adriano D. Métodos em Química Medicinal. Available from: <http://www.gradadm.ifsc.usp.br/dados/20132/ FFI0763-1/Modulo_11_2.pdf>. Access at: Sept. 30, 2016.

BERMAN, Helen M. et al. The protein data bank. Nucleic Acids Research, Piscataway, v. 28, n. 1, p.235-242, jan. 2000.

Bloomgren G, Richman S, Hotermans C, Subramanyam M, Goelz S, Natarajan A, et al. Risk of Natalizumab-Associated Progressive Multifocal Leukoencephalopathy. N Engl J Med [Internet]. 2012 May 17 [cited 2018 Dec 30];366(20):1870-80. Available from: http://www.nejm-org/doi/abs/10.1056/NEJMoa1107829

BLUNDELL, T. L. et al. Knowledge-based prediction of protein structures and the design of novel molecules. Nature, [s.l.], v. 326, n. 6111, p.347-352, 26 mar. 1987. Nature Publishing Group.

Chaves B. ENGENHARIA DE ANTICORPOS IN SILICO: CONSTRUQAO DE UM SCFV ANTAGONICO A INTEGRINAS α4β1 COMO ALTERNATIVA PARA O TRATAMENTO DE ESCLEROSE MULTIPLA. Universidade Federal do Ceará; 2016.

Chuluyan HE, Issekutz AC. VLA-4 integrin can mediate CD11/CD18-independent transendothelial migration of human monocytes. J Clin Invest [Internet]. 1993 Dec 1 [cited 2018 Dec 24];92(6):2768-77. Available from: http://www.ncbi.nlm.nih.gov/pubmed/7902847

Csizmadia V, Hofman SO, Fedyk ER, Haanstra KG, Blezer ELA, Lopes Estevao DM, et al. Antagonizing the 4 1 Integrin, but Not 4 7, Inhibits Leukocytic Infiltration of the Central Nervous System in Rhesus Monkey Experimental Autoimmune Encephalomyelitis. J Immunol. 2013;190(5):1961-73 .

DHIB-JALBUT, S.; MARKS, S.. Interferon- mechanisms of action in multiple sclerosis. Neurology, [s.l.], v. 74, n. 11, p.17-24, 28 dez. 2009. Ovid Technologies (Wolters Kluwer Health).

DINAMICA Molecular. Available from: <http://www.maxwell.vrac.puc-rio.br/21310/21310_5.PDF>. Access at: Sept. 29, 2016.

DOMINGUEZ, Cyril; BOELENS, Rolf; BONVIN, Alexandre M. J. J. HADDOCK: A Protein-Protein Docking Approach Based on Biochemical or Biophysical Information. J. Am. Chem. Soc., [s.l.], v. 125, n. 7, p.1731-1737, fev. 2003. American Chemical Society (ACS).

Dominici S, Fiori V, Watson R, Weber E. Blocking monocyte transmigration in in vitro system by an anti- CD99 human antibody in single chain fragment variable (scFv) format. Efficient large scale purification of biological active scFv from inclusion bodies in E. coli expression system. J Immunol Methods. 2014;408:35-45.

Duplàa C, Couffinhal T, Dufourcq P, Lianas B, Moreau C, Bonnet J. The integrin very late antigen-4 is expressed in human smooth muscle cell. Involvement of alpha 4 and vascular cell adhesion molecule-1 during smooth muscle cell differentiation. Circ Res [Internet]. 1997 Feb [cited 2018 Dec 26];80(2):159-69. Available from: http://www.ncbi.nlm.nih.gov/pubmed/9012738

Ecoiffier T, Annan J El, Rashid S, Schaumberg D, Dana R. Modulation of Integrin $\alpha4\beta1$ (VLA-4) in Dry Eye Disease. Arch Ophthalmol [Internet]. 2008 Dec 8 [cited 2018 Dec 27];126(12):1695. Available from: http://archopht.jamanetwork.com/article.aspx?doi=10.1001/archopht.126.12.1 695

EMSLEY, P. et al. Features and development of Coot. Acta Crystallogr D Biol Cryst, [s.l.], v. 66, n. 4, p.486-501, 24 mar. 2010. International Union of Crystallography (IUCr).

Franz CM, Jones GE, Ridley AJ. Cell Migration in Development and Disease. Dev Cell [Internet]. 2002 Feb 1 [cited 2019 Mar 7];2(2):153-8. Available from: https://www.sciencedirect.com/science/ article/pii/S153458070200120X

Gheuens S, Bord E, Kesari S, Simpson DM, Gandhi RT, Clifford DB, et al. Role of CD4+ and CD8+ T-Cell Responses against JC Virus in the Outcome of Patients with Progressive Multifocal Leukoencephalopathy (PML) and PML with Immune Reconstitution Inflammatory Syndrome. J Virol [Internet]. 2011 Jul 15 [cited 2019 Mar 7];85(14):7256-63. Available from: http://www.ncbi.nlm.nih.gov/pubmed/21543472

Gismondi A, Morrone S, Humphries MJ, Piccoli M, Frati L, Santoni A. Human natural killer cells express VLA-4 and VLA-5, which mediate their adhesion to fibronectin. J Immunol [Internet]. 1991 Jan 1 [cited 2018 Dec 24];146(1):384-92. Available from: http://www.ncbi.nlm.nih.gov/pubmed/1701798

GOLDENBERG, Marvin M.. Multiple Sclerosis. Pharmacy And Therapeutics. Westfield, p. 175-184. mar. 2012.

GROMACS Groningen Machine for Chemical Simulations: User manual. User manual. Available from: <ftp://ftp.gromacs.org/pub/manual/manual-5.0.7.pdf>. Access at: Aug. 4, 2016.

Grove RA, Shackelford S, Sopper S, Pirruccello S, Horrigan J, Havrdova E, et al. Leukocyte counts in cerebrospinal fluid and blood following firategrast treatment in subjects with relapsing forms of multiple sclerosis. Eur J Neurol [Internet]. 2013 Jul 1 [cited 2018 Dec 29];20(7):1032-42. Available from: http://doi.wiley.com/10.1111/ene.12097

HAANSTRA, K. G. et al. Antagonizing the $\alpha4\beta1$ Integrin, but Not $\alpha4\beta7$, Inhibits Leukocytic Infiltration of the Central Nervous System in Rhesus Monkey Experimental Autoimmune Encephalomyelitis. The Journal Of Immunology, [s.l.], v. 190, n. 5, p.1961-1973, 30 jan. 2013. The American Association of Immunologists.

HAYRYAN, Shura et al. A new analytical method for computing solvent-accessible surface area of macromolecules and its gradients. Journal Of Computational Chemistry, [s.l.], v. 26, n. 4, p.334-343, 10 jan. 2005. Wiley-Blackwell.

HINTZE, Bradley J. et al. Molprobity's ultimate rotamer-library distributions for model validation. Proteins: Structure, Function, and Bioinformatics, [s.l.], v. 84, n. 9, p.1177-1189, 23 jun. 2016. Wiley-Blackwell.

Hyduk SJ, Cybulsky MI. VCAM-1 and its functions in development and inflammatory diseases. In: Adhesion Molecules: Function and Inhibition [Internet]. Basel: Birkhäuser Basel; 2007 [cited 2019 Mar 7]. p. 141-74. Available from: http://www.springerlink.com/index/10.1007/978-3-7643-7975-9 6

HYNES, Richard O. Integrins. Cell, [s.l.], v. 110, n. 6, p.1032673-687, set. 2002. Elsevier BV.

Hyun Y-M, Chung H-L, McGrath JL, Waugh RE, Kim M. Activated integrin VLA-4 localizes to the lamellipodia and mediates T cell migration on VCAM-1. J Immunol [Internet]. 2009 Jul 1 [cited 2018 Dec 26];183(1):359-69. Available from: http://www.ncbi.nlm.nih.gov/pubmed/19542447

Jelcic I, Jelcic I, Kempf C, Largey F, Planas R, Schippling S, et al. Mechanisms of immune escape in central nervous system infection with neurotropic JC virus variant. Ann Neurol [Internet]. 2016 Mar 1 [cited 2019 Mar 7];79(3):404-18. Available from: http://doi.wilev.com/10.1002/ana.24574

JLTG, Gregor; ANDERLUH, Marko; TOMAŽIč, Tihomir. Comparative evaluation of several *docking* tools for *docking* small molecule ligands to DC-SIGN. Journal Of Molecular Modeling, [s.l.], v. 21, n. 6, p.1-12,jun. 2015. Springer Science + Business Media.

KHAN, Faez Iqbal et al. Current updates on computer aided protein modeling and designing. International Journal of Biological Macromolecules, [s.l.], v. 85, p.48-62, abr. 2016. Elsevier BV.

KITCHEN, Douglas B. et al. Docking and scoring in virtual screening for drug discovery: methods and applications. Nature Reviews Drug Discovery, [s.l.], v. 3, n. 11, p.935-949, nov. 2004. Nature Publishing Group.

KOEHL, Patrice; LEVITT, Michael. A brighter future for protein structure prediction. Nature Structural Biology, [s.l.], v. 6, n. 2, p.108-111, 1 fev. 1999. Springer Nature.

Kong D-H, Kim YK, Kim MR, Jang JH, Lee S. Emerging Roles of Vascular Cell Adhesion Molecule-1 (VCAM-1) in Immunological Disorders and Cancer. Int J Mol Sci [Internet]. 2018 Apr 2 [cited 2019 Feb 10];19(4). Available from: http://www.ncbi.nlm.nih.gov/pubmed/29614819

KORTEMME, Tanja. Computational Alanine Scanning of Protein-Protein Interfaces. San Francisco: Science's Stke, 2004.

KP, Johnson et al. Extended use of glatiramer acetate (Copaxone) is well tolerated and maintains its clinical effect on multiple sclerosis relapse rate and degree of disability. Copolymer 1 Multiple Sclerosis Study Group. Neurology, [s.i], p. 701-708. mar. 1988.

KRISSINEL, Evgeny; HENRICK, Kim. Inference of Macromolecular Assemblies from Crystalline State. Journal Of Molecular Biology, [s.l.], v. 372, n. 3, p.774-797, set. 2007. Elsevier BV.

Kummer C, Ginsberg MH. New approaches to blockade of α4-integrins, proven therapeutic targets in chronic inflammation. Biochem Pharmacol [Internet]. 2006 Nov 30 [cited 2018 Dec 22];72(11):1460-8. Available from: https://www.sciencedirect.com/science/ article/abs/pii/S0006295206003613?via%3Dihub

Laff6n A, Garcia-Vicufla R, Humbria A, Postigo AA, Corbi AL, De Landizuri M 0, et al. Upregulated Expression and Function of VLA-4 Fibronectin Receptors on Human Activated T Cells in Rheumatoid Arthritis [Internet]. [cited 2019 Feb 10]. Available from: https://www.ncbi.nlm.nih.gov/pmc/articles/PMC295383/pdf/jcinvest00061-0192.pdf

Leibly DJ, Nguyen TN, Kao LT, Hewitt SN, Barrett LK, van Voorhis WC. Stabilizing Additives Added during Cell Lysis Aid in the Solubilization of Recombinant Proteins. PLoS One. 2012;7(12).

LOBB, R R; HEMLER, M e. The pathophysiologic role of alpha 4 integrins in vivo. Journal Of Clinical Investigation, [s.l.], v. 94, n. 5, p.1722-1728, 1 nov. 1994. American Society for Clinical Investigation.

Lobstein J, Emrich CA, Jeans C, Faulkner M, Riggs P, Berkmen M. SHuffle, a novel Escherichia coli protein expression strain capable of correctly folding disulfide bonded proteins in its cytoplasm. Microb Cell Fact [Internet]. 2012 Dec 8 [cited 2019 Jan 7];11(1):753. Available from: http://www.ncbi.nlm.nih.gov/pubmed/22569138

LU, Yuan et al. SiRNA delivered by EGFR-specific scFv sensitizes EGFR-TKI-resistant human lung cancer cells.Biomaterials, [s.l.], v. 76, p.196-207, jan. 2016. Elsevier BV.

LUTHY, Roland; BOWIE, James U.; EISENBERG, David. Assessment of protein models with three-dimensional profiles. Nature, [s.l.], v. 356, n. 6364, p.83-85, 5 mar. 1992. Springer Nature.

MCCONKEY, Brendan J.; SOBOLEV, Vladimir; EDELMAN, Marvin. The performance of current methods in ligand-protein docking. Current Science. Rehovot, p. 845-856. 10 out. 2002

McDonald KA, Horwitz AF, Knudsen KA. Adhesion molecules and skeletal myogenesis. Semin Dev Biol [Internet]. 1995 Apr 1 [cited 2018 Dec 26];6(2):105-16.

MENG, Xuan-yu et al. Molecular Docking: A Powerful Approach for Structure-Based Drug Discovery. Current Computer Aided-drug Design, [s.l.], v. 7, n. 2, p.146-157, 1 jun. 2011. Bentham Science Publishers Ltd..

Miller DH, Weber T, Grove R, Wardell C, Horrigan J, Graff O, et al. Firategrast for relapsing remitting multiple sclerosis: a phase 2, randomised, double-blind, placebo-controlled trial. Lancet Neurol [Internet]. 2012 Feb 1 [cited 2018 Dec 29];11(2):131-9. Available from: http://www.ncbi.nlm.nih.gov/pubmed/22226929

Mittelbrunn M, Molina A, Escribese MM, Yanez-Mo M, Escudero E, Ursa A, et al. VLA-4 integrin concentrates at the peripheral supramolecular activation complex of the immune synapse and drives T helper 1 responses. Proc Natl Acad Sci [Internet]. 2004 Jul 27 [cited 2019 Feb 10];101(30):11058-63. Available from: http://www.ncbi.nlm.nih.gov/pubmed/15263094

MONTANARI, Maria Luiza C. et al. Sistemas transportadores de drogas. Química Nova, [s.l.], v. 21, n. 4, p.470-476, jul. 1998. FapUNIFESP (SciELO).

MORICOLI, Diego et al. Blocking monocyte transmigration in in vitro system by a human antibody scFv anti-CD99. Efficient large scale purification from periplasmic inclusion bodies in E. coli expression system. Journal Of Immunological Methods, [s.l.], v. 408, p.35-45, jun. 2014. Elsevier BV.

MULTIPLE SCLEROSIS INTERNATIONAL FEDERATION. What is MS? Available from: <https://www.msif.org/about-ms/what-is-ms/>. Access at: Oct. 28, 2015.

MURPHY, Kenneth; TRAVERS, Paul; WALPORT, Mark. Imunobiologia de Janeway. 7. ed. São Paulo: Artmed, 2010.

NAMBA, A. M.; SILVA, V. B. da; DA SILVA, C. H. T. P. Dinâmica molecular: teoria e aplicações em planejamento de fármacos. Eclética Química, [s.l.], v. 33, n. 4, p.13-23, dez. 2008. FapUNIFESP (SciELO).

Nieto M, Gómez M, Sánchez-Mateos P, Fernández E, Marazuela M, Sacedón R, et al. Expression of functionally active alpha 4 beta 1 integrin by thymic epithelial cells. Clin Exp Immunol [Internet]. 1996 Oct [cited 2018 Dec 26];106(1):170-8. Available from: http://www.ncbi.nlm.nih.gov/pubmed/8870716

Olson DL, Burkly LC, Leone DR, Dolinski BM, Lobb RR. Anti-a4 integrin monoclonal antibody inhibits multiple myeloma growth in a murine model [Internet]. 2005 [cited 2018 Dec 30]. Available from: http://mct.aacrjournals.org/content/molcanther/4/1/91.full.pdf

Pankov R, Yamada KM. Fibronectin at a glance. J Cell Sci [Internet]. 2002 Oct 15 [cited 2018 Dec 27];115(Pt 20):3861-3. Available from: http://www.ncbi.nlm.nih.gov/pubmed/12244123

Pavlovic D, Patel MA, Patera AC, Peterson I. T cell deficiencies as a common risk factor for drug associated progressive multifocal leukoencephalopathy. Immunobiology [Internet]. 2018 Jun 1 [cited 2019 Mar 7];223(6-7):508-17. Available from: https://www.sciencedirect.com/science/article/pii/S0171298518300020

PIÑEIRO, Angel. Introduction to Molecular Dynamics Simulations for Biological Systems. 2009. Available from: <http://smmb.usc.es/docs/mdproteins.pdf>. Access at: Mar. 01, 2016.

Polman CH, O'Connor PW, Havrdova E, Hutchinson M, Kappos L, Miller DH, et al. A Randomized, Placebo-Controlled Trial of Natalizumab for Relapsing Multiple Sclerosis. N Engl J Med [Internet]. 2006 Mar 2 [cited 2018 Dec 30];354(9):899-910. Available from: http://www.ncbi.nlm.nih.gov/pubmed/16510744

RATNIKOV, B. I.; PARTRIDGE, A. W.; GINSBERG, M. H. Integrin activation by talin. J Thromb Haemost, [s.l.], v.

3, n. 8, p.1783-1790, ago. 2005. Wiley-Blackwell.

REBOUÇAS, Alison de Sousa. Prospecção tecnológica, modelagem e Dinâmica Molecular do receptor CD20, alvo molecular do anticorpo monoclonal Rituximab. 2015. 44 f. TCC (Graduação) - Curso de Biotecnologia, Universidade Federal do Ceará, Fortaleza, 2015.

Rosemblatt M, Vuillet-Gaugler MH, Leroy C, Coulombel L. Coexpression of two fibronectin receptors, VLA-4 and VLA-5, by immature human erythroblastic precursor cells. J Clin Invest [Internet]. 1991 Jan [cited 2018 Dec 27];87(1):6-11. Available from: http://www.ncbi.nlm.nih.gov/pubmed/1824634

Ryan DH, Nuccie BL, Abboud CN, Winslow JM. Vascular cell adhesion molecule-1 and the integrin VLA-4 mediate adhesion of human B cell precursors to cultured bone marrow adherent cells. J Clin Invest [Internet]. 1991 Sep 1 [cited 2018 Dec 24];88(3):995-1004. Available from: http://www.ncbi.nlm.nih.gov/pubmed/1715889

SAKURAI, Ken et al. Investigation of the teratogenic potential of VLA-4 antagonist derivatives in rats. Reproductive Toxicology, [s.l.], v. 49, p.162-170, nov. 2014.

Salvi G, De Los Rios P, Vendruscolo M. Effective interactions between chaotropic agents and proteins. Proteins Struct Funct Genet. 2005;61(3):492-9.

Sánchez-Aparicio P, Dominguez-Jiménez C, Garcia-Pardo A. Activation of the alpha 4 beta 1 integrin through the beta 1 subunit induces recognition of the RGDS sequence in fibronectin. J Cell Biol [Internet]. 1994 Jul [cited 2019 Feb 10];126(1):271-9. Available from: http://www.ncbi.nlm.nih.gov/pubmed/7517944

SANCHEZ, Roberto; I·ALI, Andrej. Advances in comparative protein-structure modelling. Current Opinion In Structural Biology, [s.l.], v. 7, n. 2, p.206-214, abr. 1997. Elsevier BV.

Sato T, Tachibana K, Nojima Y, D'Avirro N, Morimoto C. Role of the VLA-4 molecule in T cell costimulation. Identification of the tyrosine phosphorylation pattern induced by the ligation of VLA-4. J Immunol [Internet]. 1995 Sep 15 [cited 2018 Dec 24];155(6):2938-47. Available from: http://www.ncbi.nlm.nih.gov/pubmed/7673711

Schleimer RP, Sterbinsky SA, Kaiser J, Bickel CA, Klunk DA, Tomioka K, et al. IL-4 induces adherence of human eosinophils and basophils but not neutrophils to endothelium. Association with expression of VCAM-1. J Immunol [Internet]. 1992 Feb 15 [cited 2018 Dec 26];148(4):1086-92. Available from: http://www.ncbi.nlm.nih.gov/pubmed/1371130

Schlessinger J. Cell Signaling by Receptor Review Tyrosine Kinases receptor) are monomers in the cell membrane. Ligand binding induces dimerization of these receptors re-sulting in autophosphorylation of their cytoplasmic do [Internet]. Vol. 103, Cell. 2000 [cited 2019 Mar 7]. Available from: https://ac.els-cdn.com/S0092867400001148/1-s2.0-S0092867400001148-main.pdf?_tid=ba209770-f078-40c1-a6a2-0a85f6565840&acdnat=1551963267c4c439f597c6036e73a0e12c611e413a

SCHLESINGER, Martin et al. The role of VLA-4 binding for experimental melanoma metastasis and its inhibition by heparin. Thrombosis Research, [s.l.], v. 133, n. 5, p.855-862, maio 2014. Elsevier BV.

Schlesinger M, Bendas G. Contribution of very late antigen-4 (VLA-4) integrin to cancer progression and metastasis. Cancer Metastasis Rev [Internet]. 2015 Dec 7 [cited 2018 Dec 28];34(4):575-91. Available from: http://link.springer.com/10.1007/s10555-014-9545-x

SCHRAUBER, Hannelore; EISENHABER, Frank; ARGOS, Patrick. Rotamers: To be or not to be?. Journal Of Molecular Biology, [s.l.], v. 230, n. 2, p.592-612, mar. 1993. Elsevier BV.

Schwab N, Schneider-Hohendorf T, Wiendl H. Therapeutic uses of anti- 4-integrin (anti-VLA-4) antibodies in multiple sclerosis. Int Immunol [Internet]. 2015 Jan 1 [cited 2018 Dec 29];27(1):47-53. Available from: http://www.ncbi.nlm.nih.gov/pubmed/25326459

SHEN, Zhihong et al. Single-Chain Fragment Variable Antibody Piezoimmunosensors. Analytical Chemistry, [s.l.], v. 77, n. 3, p.797-805, fev. 2005. American Chemical Society (ACS).

Sina M, Farajzadeh D, Dastmalchi S. Effects of environmental factors on soluble expression of a humanized anti-TNF-α scFv antibody in Escherichia coli. Adv Pharm Bull [Internet]. 2015;5(4):455-61. Available from: http://dx.doi.org/10.15171/apb.2015.062

Singh J, Adams S, Carter MB, Cuervo H, Lee W-C, Lobb RR, et al. Rational design of potent and selective VLA-4 inhibitors and their utility in the treatment of asthma. Curr Top Med Chem [Internet]. 2004 [cited 2018 Dec 30];4(14):1497-507. Available from: http://www.ncbi.nlm.nih.gov/pubmed/15544540

SPAIN, Elaine et al. Detection of prostate specific antigen based on electrocatalytic platinum nanoparticles conjugated to a recombinant scFv antibody. Biosensors And Bioelectronics, [s.l.], v. 77, p.759-766, mar. 2016. Elsevier BV.

SRICHAI, Manakan Betsy; ZENT, Roy. Integrin Structure and Function. In: ZENT, Roy. Cell-Extracellular Matrix Interactions in Cancer. Nashville: Springer, 2010. p. 19-41.

Sugiura T, Kageyama S, Andou A, Miyazawa T, Ejima C, Nakayama A, et al. Oral treatment with a novel small molecule alpha 4 integrin antagonist, AJM300, prevents the development of experimental colitis in mice. J Crohns Colitis [Internet]. 2013 Dec 1 [cited 2018 Dec 29];7(11):e533-42. Available from: http://www.ncbi.nlm.nih.gov/pubmed/23623333

Takada Y, Ye X, Simon S. The integrins. Genome Biol [Internet]. 2007 [cited 2018 Dec 17];8(5):215. Available from:

http://www.ncbi.nlm.nih.gov/pubmed/17543136

TASHIRO, Ken-ichiro et al. An IKLLI-containing peptide derived from the laminin a1 chain mediating heparin-binding, cell adhesion, neurite outgrowth and proliferation, represents a binding site for integrin a3b1 and heparan sulphate proteoglycan. Biochemical Journal. Great Britain, p. 119-126. maio 1999.

THOMPSON, Julie D; HIGGINS, Desmond G; GIBSON, Toby J. CLUSTAL W: improving the sensitivity of progressive multiple sequence alignment through sequence weighting, position-specific gap penalties and weight matrix choice. Nucleic Acids Research, Cambridge, v. 22, n. 22, p.4673-4680, nov. 1994.

To WS, Midwood KS. Plasma and cellular fibronectin: distinct and independent functions during tissue repair. Fibrogenesis Tissue Repair [Internet]. 2011 Sep 16 [cited 2019 Mar 7];4:21. Available from: http://www.ncbi.nlm.nih.gov/pubmed/21923916

Ukkonen K, Neubauer A, Pereira VJ, Vasala A. High Yield of Recombinant Protein in Shaken E. coli Cultures with Enzymatic Glucose Release Medium EnPresso B. In: Burgess-Brown NA, editor. Heterologous Gene Expression in E.coli: Methods and Protocols [Internet]. New York, NY: Springer New York; 2017. p. 127-37. Available from: https://doi.org/10.1007/978-1-4939-6887-9_8

UNGER, Ron et al. A 3D building blocks approach to analyzing and predicting structure of proteins. Proteins: Structure, Function, and Genetics, [s.l.], v. 5, n. 4, p.355-373, 1989. Wiley-Blackwell.

VAN DIJK, M. Information-driven protein-DNA docking using HADDOCK: it is a matter of flexibility. Nucleic Acids Research,[s.l.], v. 34, n. 11, p.3317-3325, 28 jun. 2006. Oxford University Press (OUP).

VERLI, Hugo. Bioinformática da Biologia à Flexibilidade Molecular. Porto Alegre: Hugo Verli., 2014.

Wei SH, Parker I, Miller MJ, Cahalan MD. A stochastic view of lymphocyte motility and trafficking within the lymph node. Immunol Rev [Internet]. 2003 Oct [cited 2019 Feb 10];195:136-59. Available from: http://www.ncbi.nlm.nih.gov/pubmed/12969316

White J, Krishnamoorthy S, Gupta D, Lancelot M, Moore N, Samaik S, et al. VLA-4 blockade by natalizumab inhibits sickle reticulocyte and leucocyte adhesion during simulated blood flow. Br J Haematol [Internet]. 2016 Sep [cited 2018 Dec 30];174(6):970-82. Available from: http://www.ncbi.nlm.nih.gov/pubmed/27291690

Wu C, Fields AJ, Kapteijn BA, McDonald JA. The role of alpha 4 beta 1 integrin in cell motility and fibronectin matrix assembly. J Cell Sci [Internet]. 1995 Feb [cited 2019 Feb 10];108 ( Pt 2):821-9. Available from: http://www.ncbi.nlm.nih.gov/pubmed/7539441

Yoshimura N, Watanabe M, Motoya S, Tominaga K, Matsuoka K, Iwakiri R, et al. Safety and Efficacy of AJM300, an Oral Antagonist of $\alpha$4 Integrin, in Induction Therapy for Patients With Active Ulcerative Colitis. Gastroenterology [Internet]. 2015 Dec [cited 2018 Dec 29];149(7):1775-1783.e2. Available from: http://www.ncbi.nlm.nih.gov/pubmed/26327130

Yu Y, Schürpf T, Springer TA. How natalizumab binds and antagonizes $\alpha$4 integrins. J Biol Chem [Internet]. 2013 Nov 8 [cited 2018 Dec 26];288(45):32314-25. Available from: http://www.ncbi.nlm.nih.gov/pubmed/24047894

Xiong H, Li S, Yang Z, Burgess RR, Dynan WS. E. coli expression of a soluble, active single-chain antibody variable fragment containing a nuclear localization signal. Protein Expr Purif [Internet]. 2009;66(2):172-80. Available from: http://dx.doi.org/10.1016/ ipep.2009.03.002

XIONG, J.-p.. Crystal Structure of the Extracellular Segment of Integrin alpha Vbeta 3. Science, [s.l.], v. 294, n. 5541, p.339-345, 6 set. 2001. American Association for the Advancement of Science (AAAS).

Xu HN, Liu Y, Zhang L. Salting-out and salting-in: Competitive effects of salt on the aggregation behavior of soy protein particles and their emulsifying properties. Soft Matter. 2015;11(29):5926-32.

Zarschler K, Witecy S, Kapplusch F, Foerster C, Stephan H. High-yield production of functional soluble single-domain antibodies in the cytoplasm of Escherichia coli. Microb Cell Fact [Internet]. 2013 Oct 27 [cited 2019 Jan 8];12:97. Available from: http://www.ncbi.nlm.nih.gov/pubmed/24161153

## Claims

1. Protein of the scFv type, **characterized in that** the said protein comprises a first polypeptide chain and a second polypeptide chain joined by a ligand, presenting the formula as follows:

    (VH domain) - (peptide ligand) - (VL domain),
    where the VH domain comprises at least amino acids N173, Y176, K181, Y217, Y222 from SEQ ID NO: 3 and the VL domain comprises at least amino acids K31, Y33, N35 from SEQ ID NO: 3.

2. Protein according to claim 1, **characterized in that** the VH domain comprises complementarity determining regions (CDR1, CDR2, CDR3) consisting of SEQ ID NO: 10, SEQ ID NO: 11 and SEQ ID NO: 12 and the VL domain comprises complementarity determining regions (CDR1, CDR2, CDR3) consisting of SEQ ID NO: 7, SEQ ID NO:

8 and SEQ ID NO: 9.

3. Protein according to claim 1 or 2, **characterized in that** wherein the VH domain comprises the amino acids Q117 to S237 of SEQ ID NO: 3 and the VL domain comprises the amino acids D1 to I111 of SEQ ID NO: 3.

4. Protein according to any one of claims 1 to 3, **characterized in that** the ligand is 5 to 15 amino acids in length.

5. Protein according to any one of claims 1 to 4, **characterized in that** the binding peptide comprises at least the sequence GGGGS.

6. Protein according to any one of claims 1 to 5, **characterized in that** the protein selectively binds to $\alpha4\beta1$ integrin.

7. Protein according to any of claims 1 to 6, **characterized in that** it is for use in a method for prognosis or treatment of chronic inflammatory diseases, preferably multiple sclerosis.

8. Polynucleotide **characterized in that** it comprises the nucleotide sequence shown in SEQ ID NO: 1.

9. Vector **characterized in that** it comprises the polynucleotide as defined in claim 8.

10. Host cell **characterized in that** it comprises the vector as defined in claim 9.

11. Host cell according to claim 10, **characterized in that** it is a bacterial cell.

12. Host cell according to claim 11, **characterized in that** it is an *E. coli* cell.

13. Composition, **characterized in that** it comprises the protein as defined in any one of claims 1 to 7 and a pharmaceutically acceptable excipient.

14. Composition, according to claim 13, **characterized in that** it is for use in the treatment or prognosis of chronic inflammatory diseases, preferably multiple sclerosis.

15. A method for treating a disease or condition that results directly or indirectly from $\alpha4\beta1$ integrin activity, **characterized in that** it comprises administering to a human a protein as defined in any one of claims 1 to 7 or a composition as defined in claim 13 or 14.

16. Method, according to claim 15, **characterized in that** the disease or condition is a chronic inflammatory disease, preferably multiple sclerosis.

17. *In vitro* method to prognosticate a chronic inflammatory disease **characterized in that** it comprises:

    containing at least one protein as defined in any of claims 1 to 7 or a composition as defined in claim 13 or 14 with a cell, tissue or sample from an individual,
    detect the binding of the protein to the cell, tissue or sample,
    quantify the expression of VLA-4, and
    indicate a more suitable treatment for the patient.

18. Use of a protein, as defined in any of claims 1 to 7, or of a composition, as defined in claims 13 or 14, **characterized in that** it is for preparing a drug to treat or prognosticate chronic inflammatory diseases, preferably multiple sclerosis.

Key:
Pink: CDR1
Yellow: CDR2
Blue: CDR3

scFv - *Single Chain Fragment Variable*

FIGURE 1

Structures of light chains

257898

670484

725144

FIGURE 2

Structures of heavy chains

FIGURE 3

Ramachandran plots after modeling and refinement of light chains

Ramachandran plots after modeling and refinement of heavy chains

FIGURE 4

scFvs of short peptide ligand

257898 670484 725144

scFvs of long peptide ligand

257898 670484 725144

FIGURE 5

Haddock Score

FIGURE 6

52

FIGURE 7

FIGURE 8

FIGURE 9

FIGURE 10

FIGURE 11

FIGURE 12

FIGURE 13

FIGURE 14

56

FIGURE 15

FIGURE 16

FIGURE 17

FIGURE 18

FIGURE 19

FIGURE 20

FIGURE 21

FIGURE 22

FIGURE 23

FIGURE 24

FIGURE 25

FIGURE 26

FIGURE 27

FIGURE 28

FIGURE 29

FIGURE 30

FIGURE 31

FIGURE 32

FIGURE 33

FIGURE 34

FIGURE 35

FIGURE 36

FIGURE 37

FIGURE 38

FIGURE 39

Suspension of the
precipitate in 40 mL
of 20 mM Tris Triton™
X 100 2 % 0.5 M Urea
0.5 M NaCl

→ Centrifugation
(10 min, 18514 g) → Discarding the
supernatant

Aliquot collection
(Soluble fraction 1, Fso1l)

Aliquot collection
(Crude Extract 1, EB1)

Suspension of the precipitate
in 40 mL of 20 mM Tris Triton™
X 100 2 % 0.5 M Urea 0.5 M NaCl

Discarding
the supernatant ← Centrifugation ←

Aliquot collection
(Soluble fraction 2, Fsol2)

Aliquot collection
(Crude Extract 2, EB2)

Suspension of the
precipitate in 40 mL
of 20 mM Tris Triton™
X 100 2% 1 M Urea
0.5 M NaCl

→ Centrifugation → Discarding the
supernatant

Aliquot collection
(Crude Extract 3, EB3)

Aliquot collection
(Soluble fraction 3, Fsol3)

Suspension of the precipitate
in 40 mL of 20 mM Tris Triton™
X 100 2 % 2 M Urea 0.5 M NaCl

Discarding the
supernatant ← Centrifugation ←

Aliquot collection
(Soluble fraction 4, Fsol4)

Aliquot collection
(Crude Extract 4, EB4)

Storage of the Inclusion
Bodies (precipitate)

FIGURE 40

70

FIGURE 41

FIGURE 42

FIGURE 43

FIGURE 44

FIGURE 45

FIGURE 46

FIGURE 47

Gradient gel: expression in simple and fed batch

SDS-PAGE: expression in simple and fed batch

Percentage of scFv produced

FIGURE 48

1 - Cell lysis and inclusion body recovery

2 - scFv purification per IMAC affinity chromatography

FIGURE 49

scFv Purity

FIGURE 50

FIGURE 51

FIGURE 52

Jurkat cells adhered on scFv-coated surfaces

FIGURE 53

ELISA: recognition of VLA-4

FIGURE 54

Number of cells that have transmigrated on VCAM-1

Number of cells that have transmigrated on FN

FIGURE 55

1 - Adhesion of memory T Lymphocytes on VCAM-1/CXCL12, under flow:

1 - Adhesion of memory T Lymphocytes on VCAM-1/CXCL12, under flow:

3 - Adhesion of memory T Lymphocytes on VCAM-1/CXCL12, under flow:

FIGURE 56

1- Memory T Lymphocytes adhered on VCAM-1/CXCL12, under flow:

**1 dyn/cm²**

2- Memory T Lymphocytes immobilized on VCAM-1/CXCL12, under flow:

**1 dyn/cm²**

3- Migration speed of memory T Lymphocytes on VCAM-1/CXCL12, under flow:

**1 dyn/cm²**

FIGURE 57

Trajectory of memory T Lymphocytes on VCAM-1/SDF1-α on 1 dyn/cm² flow:

Trajectory of memory T Lymphocytes treated with scFv 20 µg/mL on VCAM-1/SDF1-α on 1 dyn/cm² flow:

FIGURE 58

1 - Adhesion of memory T Lymphocytes on FN/CXCL12, under flow:

2 - Adhesion of memory T Lymphocytes on FN/CXCL12, under flow:

3 - Adhesion of memory T Lymphocytes on FN/CXCL12, under flow:

FIGURE 59

1- Memory T Lymphocytes adhered on FN/CXCL12, under flow:

1 dyn/cm$^2$

2- Memory T Lymphocytes immobilized on FN /CXCL12, under flow:

1 dyn/cm$^2$

3- Migration speed of memory T Lymphocytes on FN/CXCL12, under flow:

1 dyn/cm$^2$

FIGURE 60

Trajectory of memory T Lymphocytes on FN/CXCL12 on 1 dyn/cm² flow:

Trajectory of memory T Lymphocytes treated with scFv 2 µg/mL on FN/SDF1-α on 1 dyn/cm² flow:

Trajectory of memory T Lymphocytes treated with Fab3 µg/mL on FN/SDF1-α on 1 dyn/cm² flow:

FIGURE 61

FIGURE 62

CD8+ T Lymphocytes on FN and anti-CD3-coated surfaces

Actin

No treatment
Treatment with scFv 2 µg/mL

FIGURE 63

Actin foci

FIGURE 64

CD8+ T Lymphocytes on FN and anti-CD3-coated surfaces

Phosphorylated tyrosine

No treatment

Treatment with scFv 2 µg/mL

FIGURE 65

Phosphorylated tyrosine foci

FIGURE 66

SEQ ID NO: 3
DVVMTQSPLSLPVTPGEPASISCRSSQSLAKSYSNTYLSWYLQK
PGQSPQLLIYGIGNGGSGVPARFSGSGSGTDFTLLISRVEAEDV
GVYYCLQGTHQPYTFGQGTKVEIGGGGSQVQLVQSGAEVKKPGA
SVKVSCKGSGYTFTSYWMHWVRQAPGQRLEWIGEIGPSESNTNY
NQKFKGRVTLTVDISASTAYMELSSLRSEDTAVYYCARGGYAGW
DYAIDYWGQGTLVTVSS

SEQ ID NO: 10
SGYTFTSYWMH   (S141 to H151 of the SEQ ID NO: 3)

SEQ ID NO: 11
EIGPSESNTNYNQKFKGRV   (E166 to V184 of the SEQ ID NO: 3)

SEQ ID NO: 12
GGYAGWDYAIDYWG   (G215 to G228 of the SEQ ID NO: 3)

SEQ ID NO: 7
SCRSSQSLAKSYSNTYLSW   (S22 to W40 of the SEQ ID NO: 3)

SEQ ID NO: 8
LIYGIGNGGSGVP   (L52 to P64 of the SEQ ID NO: 3)

SEQ ID NO: 9
LQGTHQPYTF   (L94 to F103 of the SEQ ID NO: 3)

SEQ ID NO: 5
DVVMTQSPLSLPVTPGEPASISCRSSQSLAKSYSNTYLSWYLQK
PGQSPQLLIYGIGNGGSGVPARFSGSGSGTDFTLLISRVEAEDV
GVYYCLQGTHQPYTFGQGTKVEI   (D1 to I111 of the SEQ ID NO: 3)

SEQ ID NO: 6
QVQLVQSGAEVKKPGASVKVSCKGSGYTFTSYWMHWVRQAPGQR
LEWIGEIGPSESNTNYNQKFKGRVTLTVDISASTAYMELSSLRS
EDTAVYYCARGGYAGWDYAIDYWGQGTLVTVSS
(Q117 to S237 of the SEQ ID NO: 3)

FIGURE 67

# INTERNATIONAL SEARCH REPORT

| | |
|---|---|
| International application No. | |
| **PCT/BR2021/050226** | |

**A. CLASSIFICATION OF SUBJECT MATTER**
**C07K16/28 (2006.01), A61K 39/395 (2006.01), C12N 1/21 (2006.01), C12N 15/13 (2006.01), C12N 15/63 (2006.01), G01N 33/563 (2006.01), A61P 29/00 (2006.01)**

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

**EPODOC, NPL, MEDLINE, GENESEQ, USGENE, WOGENE, GENBANK (continua em Quadro Suplementar)**

**C. DOCUMENTS CONSIDERED TO BE RELEVANT**

| Category* | Citation of document, with indication, where appropriate, of the relevant passages | Relevant to claim No. |
|---|---|---|
| X | WO 2018017863 A1 ( DEV CT BIOTECHNOLOGY) 25 JAN 2018 (25.01.2018) | 1, 4 to 7, 13 and 14 |
| A | Abstract, paragraph [0012]; Tables 1, 3 to 5, 7 and 8; claims; SED ID N° 9, 12, 14 and 17 | 2, 3, 8 to 12, 17 and 18 |
| A | SILVA, J. H. M. & CHAVES, B. *In silico* development of a scFv antagonistic to α4β1 integrin. *In*: IV Seminário Anual Científico e Tecnológico de Bio-Manguinhos, 2016, Rio de Janeiro. Anais do III Simpósio Internacional de Imunobiológicos e IV Seminário Anual Científico e Tecnológico de Bio-Manguinhos. Rio de Janeiro: Bio-Manguinhos, 2016. pages 88-89. doi: 10.35259/isi.sact.2016_28407. Sections "Introdução", "Metodologia" and "Resultados". | 1 to 14, 17, 18 |

☒ Further documents are listed in the continuation of Box C.    ☒ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 20 JUL 2021 (20.07.2021) | 02 AUG 2021 (02.08.2021) |

| Name and mailing address of the ISA/ | Authorized officer |
|---|---|
| **ISA/BR** | |
| Facsimile No. | Telephone No. |

Form PCT/ISA/210 (second sheet) (January 2015)

**INTERNATIONAL SEARCH REPORT**

International application No.

**PCT/BR2021/050226**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☒  forming part of the international application as filed:

        ☒  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet (1)) (January 2015)

International application No.

PCT/BR2021/050226

C (Continuation).   DOCUMENTS CONSIDERED TO BE RELEVANT

| Category* | Citation of document, with indication, where appropriate, of the relevant passages | Relevant to claim No. |
|---|---|---|
| A | CHAVES, B. *et al.* Guidelines To Predict Binding Poses of Antibody–Integrin Complexes. ACS Omega. Junho de 2020, Vol. 5, No. 27, pages 16379–16385. https://doi.org/10.1021/acsomega.0c00226. The whole document. | 1 to 14, 17, 18 |
| A | CHAVES, B. & SILVA, J. H. M. Generation of a scFv antagonistic to VLA-4 integrin as potential therapeutic target in muscular dystrophies: in silico phase. *In*: V Seminário Anual Científico e Tecnológico de Bio-Manguinhos, 2017, Rio de Janeiro. Anais do V Seminário Anual Científico e Tecnológico de Bio-Manguinhos. 2017. pages 54-55. doi: 10.35259/isi.sact.2017_24000. The whole document. | 1 to 14, 17, 18 |
| A | CHAVES, B. *et al.* Production of a scFv antagonistic to VLA-4 protein as a potential biopharmaceutical for chronic inflammatory diseases. In: VI Seminário Anual Científico e Tecnológico de Bio-Manguinhos, 2018, Rio de Janeiro. Anais do VI Seminário Anual Científico e Tecnológico de Bio-Manguinhos. Rio de Janeiro: Bio-Manguinhos, 2018. pages 60-61. doi: 10.35259/isi.sact.2018_27260. The whole document. | 1 to 14, 17, 18 |
| A | RUDICK, R. A. & SANDROCK, A. Natalizumab: alpha 4-integrin antagonist selective adhesion molecule inhibitors for MS. Expert Rev Neurother. 2004. Vol. 4, No. 4, pages 571-80. doi: 10.1586/14737175.4.4.571. The whole document. | 1 to 14, 17, 18 |
| A | YUAN, Q. *et al.* Intracellular single-chain antibody inhibits integrin VLA-4 maturation and function. Biochem J. 1996. Vol. 318(Pt 2), pages 591–596. doi: 10.1042/bj3180591. Abstract, section "Materiais e Métodos". | 1 to 14, 17, 18 |
| A | WO 2006096653 A2 ( VAN VLIJMEN HERMAN [US]) 14 SEP 2006 (14.09.2006) page 31, last paragraph of page 48, Example 3. | 1 to 14, 17, 18 |
| A | US 10335485 B2 (BIOGEN MA INC [US]) 02 JUL 2019 (02.07.2019) Cited in the Description. Abstract, Examples 1 and 2, claims. | 1 to 14, 17, 18 |
| P,A | SAVINO, W. *et al.* Integrin-directed antibody-based immunotherapy: focus on VLA-4. Immunotherapy Advances. Janeiro 2021. Vol. 1, No. 1, pages 1-11. https://doi.org/10.1093/immadv/ltab002. The whole document. | 1 to 14, 17, 18 |

Form PCT/ISA/210 (continuation of second sheet) (January 2015)

**INTERNATIONAL SEARCH REPORT**

International application No.

**PCT/BR2021/050226**

| Box No. II | Observations where certain claims were found unsearchable (Continuation of item 2 of first sheet) |

This international search report has not been established in respect of certain claims under Article 17(2)(a) for the following reasons:

1. ☒ Claims Nos.: 15 and 16
   because they relate to subject matter not required to be searched by this Authority, namely:

   Methods for treatment of the human or animal body by surgery or therapy, as well as diagnostic methods, pursuant to PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

**INTERNATIONAL SEARCH REPORT**
Information on patent family members

International application No.

PCT/BR2021/050226

| | | | |
|---|---|---|---|
| WO 2018017863 A1 | 2018-01-25 | EP 3487532 A1 | 2019-05-29 |
| | | JP 2019528051 A | 2019-10-10 |
| | | TW 201815825 A | 2018-05-01 |
| | | US 2019309092 A1 | 2019-10-10 |
| WO 2006096653 A2 | 2006-09-14 | WO 2006096653 A3 | 2007-07-12 |
| | | AU 2006220709 A1 | 2006-09-14 |
| | | CA 2600929 A1 | 2006-09-14 |
| | | CN 101171263 A | 2008-04-30 |
| | | EP 1856708 A2 | 2007-11-21 |
| | | EP 1869084 A2 | 2007-12-26 |
| | | JP 2008531060 A | 2008-08-14 |
| | | US 2006258852 A1 | 2006-11-16 |
| | | US 7678371 B2 | 2010-03-16 |
| | | US 2006197635 A1 | 2006-09-07 |
| | | US 7999642 B2 | 2011-08-16 |
| | | US 2011140814 A1 | 2011-06-16 |
| | | US 8258900 B2 | 2012-09-04 |
| | | US 2010093980 A1 | 2010-04-15 |
| | | US 8349324 B2 | 2013-01-08 |
| | | US 2014048395 A1 | 2014-02-20 |
| | | US 9284183 B2 | 2016-03-15 |
| | | US 2012306602 A1 | 2012-12-06 |
| | | US 2014077906 A1 | 2014-03-20 |
| | | WO 2006096645 A2 | 2006-09-14 |
| US 10335485 B2 | 2019-07-02 | US 2018221480 A1 | 2018-08-09 |
| | | AU 2011239512 A1 | 2012-10-18 |
| | | CA 2794863 A1 | 2011-10-20 |
| | | CN 103038257 A | 2013-04-10 |
| | | CY 1119154 T1 | 2018-02-14 |
| | | DK 2558499 T3 | 2017-07-24 |
| | | DK 3202789 T3 | 2020-05-18 |
| | | EA 201291065 A1 | 2013-03-29 |
| | | EP 2558499 A2 | 2013-02-20 |
| | | EP 3202789 A1 | 2017-08-09 |
| | | EP 3466977 A1 | 2019-04-10 |
| | | ES 2633105 T3 | 2017-09-19 |
| | | ES 2805873 T3 | 2021-02-15 |
| | | HR P20171045 T1 | 2017-10-06 |
| | | HR P20201219 T1 | 2021-02-05 |
| | | HU E035098 T2 | 2018-05-02 |
| | | HU E050858 T2 | 2021-01-28 |
| | | JP 2013530681 A | 2013-08-01 |
| | | JP 6085554 B2 | 2017-02-22 |
| | | JP 2017123846 A | 2017-07-20 |
| | | JP 6525440 B2 | 2019-06-05 |
| | | JP 2018123165 A | 2018-08-09 |
| | | JP 2020019811 A | 2020-02-06 |
| | | KR 20130066584 A | 2013-06-20 |
| | | LT 2558499 T | 2017-07-25 |
| | | LT 3202789 T | 2020-06-10 |
| | | ME 02793 B | 2018-01-20 |

Form PCT/ISA/210 (patent family annex) (January 2015)

**INTERNATIONAL SEARCH REPORT**
Information on patent family members

| International application No. |
| --- |
| PCT/BR2021/050226 |

| | | ME 03813 B | 2021-04-20 |
| --- | --- | --- | --- |
| | | MX 2012012010 A | 2013-02-26 |
| | | MX 340683 B | 2016-07-21 |
| | | NZ 602676 A | 2015-02-27 |
| | | PL 2558499 T3 | 2017-10-31 |
| | | PL 3202789 T3 | 2020-09-21 |
| | | PT 2558499 T | 2017-07-14 |
| | | PT 3202789 T | 2020-06-16 |
| | | RS 56082 B1 | 2017-10-31 |
| | | RS 60897 B1 | 2020-11-30 |
| | | SG 184318 A1 | 2012-11-29 |
| | | SG 10201502967R A | 2015-05-28 |
| | | SI 2558499 T1 | 2017-08-31 |
| | | SI 3202789 T1 | 2020-11-30 |
| | | US 2014161794 A1 | 2014-06-12 |
| | | US 2019365892 A1 | 2019-12-05 |
| | | WO 2011130603 A2 | 2011-10-20 |
| ------------------------- | ---------------- | ------------------------------ | ---------------- |

Form PCT/ISA/210 (patent family annex) (January 2015)

INTERNATIONAL SEARCH REPORT

International application No.

PCT/BR2021/050226

REGISTRY, CAPLUS, USGENE, PCTGEN, DWPI, BIOSIS, EMBASE, MEDLINE, SCISEARCH.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180369330 A
- US 10335485 B

**Non-patent literature cited in the description**

- **ABBAS, ABUL K.** Imunologia Celular e Molecular. Elsevier, 2015
- *ACETATO de Glatiramer (Copaxone),* 05 October 2016, https://esclerosemultipla.word-press.com/2006/05/09/acetato-de-glatiramer-co-paxone-®/
- ScFv Antibody: Principles and Clinical Application. **AHMAD, ZUHAIDA ASRA et al.** Clinical And Developmental Immunology,[s.l. Hindawi Publishing Corporation, 2012, vol. 2012, 1-15
- Basic local alignment search tool. **ALTSCHUL, STEPHEN F. et al.** Journal Of Molecular Biology. Elsevier BV, 1990, vol. 215, 403-410
- **ANDRICOPULO, ADRIANO D.** *Métodos em Química Medicinal,* 30 September 2016, http://www.gra-dadm.ifsc.usp.br/dados/20132/FFI0763-1/Modulo_11_2.pdf
- **BERMAN, HELEN M. et al.** The protein data bank. *Nucleic Acids Research, Piscataway,* January 2000, vol. 28 (1), 235-242
- **BLOOMGREN G ; RICHMAN S ; HOTERMANS C ; SUBRAMANYAM M ; GOELZ S ; NATARAJAN A et al.** Risk of Natalizumab-Associated Progressive Multifocal Leukoencephalopathy. *N Engl J Med [Internet].,* 17 May 2012, vol. 366 (20), 1870-80, ht-tp://www.ne-jm-org/doi/abs/10.1056/NEJMoa1107829
- Knowledge-based prediction of protein structures and the design of novel molecules. **BLUNDELL, T. L. et al.** Nature. Nature Publishing Group, 26 March 1987, vol. 326, 347-352
- Chaves B. ENGENHARIA DE ANTICORPOS IN SILICO: CONSTRUQAO DE UM SCFV ANTAGONICO A INTEGRINAS α4β1 COMO ALTERNATIVA PARA O TRATAMENTO DE ESCLEROSE MULTIPLA. Universidade Federal do Ceará, 2016
- **CHULUYAN HE ; ISSEKUTZ AC.** VLA-4 integrin can mediate CD11/CD18-independent transendothelial migration of human monocytes. *J Clin Invest [Internet].,* 01 December 1993, vol. 92 (6), 2768-77, ht-tp://www.ncbi.nlm.nih.gov/pubmed/7902847
- **CSIZMADIA V ; HOFMAN SO ; FEDYK ER ; HAAN-STRA KG ; BLEZER ELA ; LOPES ESTEVAO DM et al.** Antagonizing the 4 1 Integrin, but Not 4 7, Inhibits Leukocytic Infiltration of the Central Nervous System in Rhesus Monkey Experimental Autoimmune Encephalomyelitis. *J Immunol.,* 2013, vol. 190 (5), 1961-73
- Interferon- mechanisms of action in multiple sclerosis. **DHIB-JALBUT, S. ; MARKS, S.** Neurology. Ovid Technologies (Wolters Kluwer Health), 2009, vol. 74, 17-24
- *DINAMICA Molecular,* 29 September 2016, ht-tp://www.max-well.vrac.puc-rio.br/21310/21310_5.PDF
- A Protein-Protein Docking Approach Based on Biochemical or Biophysical Information. **DOMINGUEZ, CYRIL ; BOELENS, ROLF ; BONVIN, ALEXANDRE M. J. J. HADDOCK.** J. Am. Chem. Soc. American Chemical Society (ACS), February 2003, vol. 125, 1731-1737
- **DOMINICI S ; FIORI V ; WATSON R ; WEBER E.** Blocking monocyte transmigration in in vitro system by an anti- CD99 human antibody in single chain fragment variable (scFv) format. Efficient large scale purification of biological active scFv from inclusion bodies in E. coli expression system. *J Immunol Methods.,* 2014, vol. 408, 35-45
- **DUPLÀA C ; COUFFINHAL T ; DUFOURCQ P ; LI-ANAS B ; MOREAU C ; BONNET J.** The integrin very late antigen-4 is expressed in human smooth muscle cell. Involvement of alpha 4 and vascular cell adhesion molecule-1 during smooth muscle cell differentiation. *Circ Res,* February 1997, vol. 80 (2), 159-69, http://www.nc-bi.nlm.nih.gov/pubmed/9012738
- **ECOIFFIER T ; ANNAN J EL ; RASHID S ; SCHAUMBERG D ; DANA R.** Modulation of Integrin α4β1 (VLA-4) in Dry Eye Disease. *Arch Ophthalmol,* 08 December 2008, vol. 126 (12), 1695, http://ar-chopht.jamanetwork.com/article.as-px?doi=10.1001/archopht.126.12.1 695

- Features and development of Coot. **EMSLEY, P. et al.** Acta Crystallogr D Biol Cryst. International Union of Crystallography (IUCr), 24 March 2010, vol. 66, 486-501
- **FRANZ CM ; JONES GE ; RIDLEY AJ.** Cell Migration in Development and Disease. *Dev Cell,* 01 February 2002, vol. 2 (2), 153-8, https://www.sciencedirect.com/science/ article/pii/S153458070200120X
- **GHEUENS S ; BORD E ; KESARI S ; SIMPSON DM ; GANDHI RT ; CLIFFORD DB et al.** Role of CD4+ and CD8+ T-Cell Responses against JC Virus in the Outcome of Patients with Progressive Multifocal Leukoencephalopathy (PML) and PML with Immune Reconstitution Inflammatory Syndrome. *J Virol,* 15 July 2011, vol. 85 (14), 7256-63, http://www.ncbi.nlm.nih.gov/pubmed/21543472
- **GISMONDI A ; MORRONE S ; HUMPHRIES MJ ; PICCOLI M ; FRATI L ; SANTONI A.** Human natural killer cells express VLA-4 and VLA-5, which mediate their adhesion to fibronectin. *J Immunol,* 01 January 1991, vol. 146 (1), 384-92, http://www.ncbi.nlm.nih.gov/pubmed/1701798
- **GOLDENBERG, MARVIN M.** Multiple Sclerosis. *Pharmacy And Therapeutics. Westfield,* March 2012, 175-184
- *GROMACS Groningen Machine for Chemical Simulations: User manual. User manual,* 04 August 2016, ftp://ftp.gromacs.org/pub/manual/manual-5.0.7.pdf
- **GROVE RA ; SHACKELFORD S ; SOPPER S ; PIRRUCCELLO S ; HORRIGAN J ; HAVRDOVA E et al.** Leukocyte counts in cerebrospinal fluid and blood following firategrast treatment in subjects with relapsing forms of multiple sclerosis. *Eur J Neurol,* 01 July 2013, vol. 20 (7), 1032-42, http://doi.wiley.com/10.1111/ene.12097
- Antagonizing the $\alpha4\beta1$ Integrin, but Not $\alpha4\beta7$, Inhibits Leukocytic Infiltration of the Central Nervous System in Rhesus Monkey Experimental Autoimmune Encephalomyelitis. **HAANSTRA, K. G. et al.** The Journal Of Immunology. The American Association of Immunologists, 30 January 2013, vol. 190, 1961-1973
- A new analytical method for computing solvent-accessible surface area of macromolecules and its gradients. **HAYRYAN, SHURA et al.** Journal Of Computational Chemistry. Wiley-Blackwell, 10 January 2005, vol. 26, 334-343
- Molprobity's ultimate rotamer-library distributions for model validation. **HINTZE, BRADLEY J. et al.** Proteins: Structure, Function, and Bioinformatics. Wiley-Blackwell, 23 June 2016, vol. 84, 1177-1189
- **HYDUK SJ ; CYBULSKY MI.** VCAM-1 and its functions in development and inflammatory diseases. *Adhesion Molecules: Function and Inhibition [Internet]. Basel: Birkhäuser Basel,* 2007, 141-74, http://www.springerlink.com/index/10.1007/978-3-7643-7975-9 6
- Integrins. **HYNES, RICHARD O.** Cell. Elsevier BV, 2002, vol. 110, 673-687
- **HYUN Y-M ; CHUNG H-L ; MCGRATH JL ; WAUGH RE ; KIM M.** Activated integrin VLA-4 localizes to the lamellipodia and mediates T cell migration on VCAM-1. *J Immunol,* 01 July 2009, vol. 183 (1), 359-69, http://www.ncbi.nlm.nih.gov/pubmed/19542447
- **JELCIC I ; JELCIC I ; KEMPF C ; LARGEY F ; PLANAS R ; SCHIPPLING S et al.** Mechanisms of immune escape in central nervous system infection with neurotropic JC virus variant. *Ann Neurol [Internet].,* 01 March 2016, vol. 79 (3), 404-18, http://doi.wiley.com/10.1002/ana.24574
- Current updates on computer aided protein modeling and designing. **KHAN, FAEZ IQBAL et al.** International Journal of Biological Macromolecules. Elsevier BV, 2016, vol. 85, 48-62
- Docking and scoring in virtual screening for drug discovery: methods and applications. **KITCHEN, DOUGLAS B et al.** Nature Reviews Drug Discovery. Nature Publishing Group, November 2004, vol. 3, 935-949
- A brighter future for protein structure prediction. **KOEHL, PATRICE ; LEVITT, MICHAEL.** Nature Structural Biology. Springer Nature, 01 February 1999, vol. 6, 108-111
- **KONG D-H ; KIM YK ; KIM MR ; JANG JH ; LEE S.** Emerging Roles of Vascular Cell Adhesion Molecule-1 (VCAM-1) in Immunological Disorders and Cancer. *Int J Mol Sci,* 02 April 2018, vol. 19 (4, http://www.ncbi.nlm.nih.gov/pubmed/29614819
- **KORTEMME, TANJA.** Computational Alanine Scanning of Protein-Protein Interfaces. *San Francisco: Science's Stke,* 2004
- **KP, JOHNSON et al.** Extended use of glatiramer acetate (Copaxone) is well tolerated and maintains its clinical effect on multiple sclerosis relapse rate and degree of disability. Copolymer 1 Multiple Sclerosis Study Group. *Neurology,* March 1988, 701-708
- Inference of Macromolecular Assemblies from Crystalline State. **KRISSINEL, EVGENY ; HENRICK, KIM.** Journal Of Molecular Biology. Elsevier BV, 2007, vol. 372, 774-797

- **KUMMER C ; GINSBERG MH.** New approaches to blockade of α4-integrins, proven therapeutic targets in chronic inflammation. *Biochem Pharmacol,* 30 November 2006, vol. 72 (11), 1460-8, https://www.sciencedirect.com/science/article/abs/pii/S0006295206003613?via%3Dihub
- **LAFF6N A ; GARCIA-VICUFLA R ; HUMBRIA A ; POSTIGO AA ; CORBI AL ; DE LANDIZURI M 0 et al.** Upregulated Expression and Function of VLA-4 Fibronectin Receptors on Human Activated T Cells. *Rheumatoid Arthritis,* 10 February 2019, https://www.ncbi.nlm.nih.gov/pmc/articles/PMC295383/pdf/jcinvest00061-0192.pdf
- **LEIBLY DJ ; NGUYEN TN ; KAO LT ; HEWITT SN ; BARRETT LK ; VAN VOORHIS WC.** Stabilizing Additives Added during Cell Lysis Aid in the Solubilization of Recombinant Proteins. *PLoS One.,* 2012, vol. 7 (12
- The pathophysiologic role of alpha 4 integrins in vivo. **LOBB, R R ; HEMLER, M E.** Journal Of Clinical Investigation. American Society for Clinical Investigation, 01 November 1994, vol. 94, 1722-1728
- **LOBSTEIN J ; EMRICH CA ; JEANS C ; FAULKNER M ; RIGGS P ; BERKMEN M.** SHuffle, a novel Escherichia coli protein expression strain capable of correctly folding disulfide bonded proteins in its cytoplasm. *Microb Cell Fact,* 08 December 2012, vol. 11 (1), 753, http://www.ncbi.nlm.nih.gov/pubmed/22569138
- SiRNA delivered by EGFR-specific scFv sensitizes EGFR-TKI-resistant human lung cancer cells. **LU, YUAN et al.** Biomaterials. Elsevier BV, January 2016, vol. 76, 196-207
- Assessment of protein models with three-dimensional profiles. **LUTHY, ROLAND ; BOWIE, JAMES U. ; EISENBERG, DAVID.** Nature. Springer Nature, 05 March 1992, vol. 356, 83-85
- **MCCONKEY, BRENDAN J. ; SOBOLEV, VLADIMIR ; EDELMAN, MARVIN.** The performance of current methods in ligand-protein docking. *Current Science. Rehovot,* 2002, 845-856
- **MCDONALD KA ; HORWITZ AF ; KNUDSEN KA.** Adhesion molecules and skeletal myogenesis. *Semin Dev Biol,* 01 April 1995, vol. 6 (2), 105-16
- Molecular Docking: A Powerful Approach for Structure-Based Drug Discovery. **MENG, XUAN-YU et al.** Current Computer Aided-drug Design. Bentham Science Publishers Ltd, 01 June 2011, vol. 7, 146-157
- **MILLER DH ; WEBER T ; GROVE R ; WARDELL C ; HORRIGAN J ; GRAFF O et al.** Firategrast for relapsing remitting multiple sclerosis: a phase 2, randomised, double-blind, placebo-controlled trial. *Lancet Neurol,* 01 February 2012, vol. 11 (2), 131-9, http://www.ncbi.nlm.nih.gov/pubmed/22226929
- **MITTELBRUNN M ; MOLINA A ; ESCRIBESE MM ; YANEZ-MO M ; ESCUDERO E ; URSA A et al.** VLA-4 integrin concentrates at the peripheral supramolecular activation complex of the immune synapse and drives T helper 1 responses. *Proc Natl Acad Sci,* 27 July 2004, vol. 101 (30), 11058-63, http://www.ncbi.nlm.nih.gov/pubmed/15263094
- Sistemas transportadores de drogas. **MONTANARI, MARIA LUIZA C. et al.** Química Nova. FapUNIFESP (SciELO), July 1998, vol. 21, 470-476
- Blocking monocyte transmigration in in vitro system by a human antibody scFv anti-CD99. Efficient large scale purification from periplasmic inclusion bodies in E. coli expression system. **MORICOLI, DIEGO et al.** Journal Of Immunological Methods. Elsevier BV, June 2014, vol. 408, 35-45
- What is MS?. MULTIPLE SCLEROSIS INTERNATIONAL FEDERATION, 28 October 2015
- **MURPHY, KENNETH ; TRAVERS, PAUL ; WALPORT, MARK.** Imunobiologia de Janeway. Artmed, 2010
- Dinâmica molecular: teoria e aplicações em planejamento de fármacos. **NAMBA, A. M. ; SILVA, V. B. DA ; DA SILVA, C. H. T. P.** Eclética Química. FapUNIFESP (SciELO), 2008, vol. 33, 13-23
- **NIETO M ; GÓMEZ M ; SÁNCHEZ-MATEOS P ; FERNÁNDEZ E ; MARAZUELA M ; SACEDÓN R et al.** Expression of functionally active alpha 4 beta 1 integrin by thymic epithelial cells. *Clin Exp Immunol,* October 1996, vol. 106 (1), 170-8, http://www.ncbi.nlm.nih.gov/pubmed/8870716
- **OLSON DL ; BURKLY LC ; LEONE DR ; DOLINSKI BM ; LOBB RR.** *Anti-a4 integrin monoclonal antibody inhibits multiple myeloma growth in a murine model,* 2005, http://mct.aacrjournals.org/content/molcanther/4/1/91.full.pdf
- **PANKOV R ; YAMADA KM.** Fibronectin at a glance. *J Cell Sci,* 15 October 2002, vol. 115, 3861-3, http://www.ncbi.nlm.nih.gov/pubmed/12244123
- **PAVLOVIC D ; PATEL MA ; PATERA AC ; PETERSON I.** T cell deficiencies as a common risk factor for drug associated progressive multifocal leukoencephalopathy. *Immunobiology,* 01 June 2018, vol. 223 (6-7), 508-17, https://www.sciencedirect.com/science/article/pii/S0171298518300020
- **PIÑEIRO, ANGEL.** *Introduction to Molecular Dynamics Simulations for Biological Systems.,* 01 March 2016, http://smmb.usc.es/docs/mdproteins.pdf
- **POLMAN CH ; O'CONNOR PW ; HAVRDOVA E ; HUTCHINSON M ; KAPPOS L ; MILLER DH et al.** A Randomized, Placebo-Controlled Trial of Natalizumab for Relapsing Multiple Sclerosis. *N Engl J Med,* 02 March 2006, vol. 354 (9), 899-910, http://www.ncbi.nlm.nih.gov/pubmed/16510744

- Integrin activation by talin. **RATNIKOV, B. I. ; PARTRIDGE, A. W. ; GINSBERG, M. H.** J Thromb Haemost. Wiley-Blackwell, 2005, vol. 3, 1783-1790
- **ROSEMBLATT M ; VUILLET-GAUGLER MH ; LEROY C ; COULOMBEL L.** Coexpression of two fibronectin receptors, VLA-4 and VLA-5, by immature human erythroblastic precursor cells. *J Clin Invest,* January 1991, vol. 87 (1), 6-11, http://www.ncbi.nlm.nih.gov/pubmed/1824634
- **RYAN DH ; NUCCIE BL ; ABBOUD CN ; WINSLOW JM.** Vascular cell adhesion molecule-1 and the integrin VLA-4 mediate adhesion of human B cell precursors to cultured bone marrow adherent cells. *J Clin Invest,* 01 September 1991, vol. 88 (3), 995-1004, http://www.ncbi.nlm.nih.gov/pubmed/1715889
- **SAKURAI, KEN et al.** Investigation of the teratogenic potential of VLA-4 antagonist derivatives in rats. *Reproductive Toxicology,* November 2014, vol. 49, 162-170
- **SALVI G ; DE LOS RIOS P ; VENDRUSCOLO M.** Effective interactions between chaotropic agents and proteins. *Proteins Struct Funct Genet.,* 2005, vol. 61 (3), 492-9
- **SÁNCHEZ-APARICIO P ; DOMINGUEZ-JIMÉNEZ C ; GARCIA-PARDO A.** Activation of the alpha 4 beta 1 integrin through the beta 1 subunit induces recognition of the RGDS sequence in fibronectin. *J Cell Biol,* July 1994, vol. 126 (1), 271-9, http://www.ncbi.nlm.nih.gov/pubmed/7517944
- Advances in comparative protein-structure modelling. **SANCHEZ, ROBERTO ; L·ALI, ANDREJ.** Current Opinion In Structural Biology. Elsevier BV, 1997, vol. 7, 206-214
- **SATO T ; TACHIBANA K ; NOJIMA Y ; D'AVIRRO N ; MORIMOTO C.** Role of the VLA-4 molecule in T cell costimulation. Identification of the tyrosine phosphorylation pattern induced by the ligation of VLA-4. *J Immunol,* 15 September 1995, vol. 155 (6), 2938-47, http://www.ncbi.nlm.nih.gov/pubmed/7673711
- **SCHLEIMER RP ; STERBINSKY SA ; KAISER J ; BICKEL CA ; KLUNK DA ; TOMIOKA K et al.** IL-4 induces adherence of human eosinophils and basophils but not neutrophils to endothelium. Association with expression of VCAM-1. *J Immunol,* 15 February 1992, vol. 148 (4), 1086-92, http://www.ncbi.nlm.nih.gov/pubmed/1371130
- **SCHLESSINGER J.** Cell Signaling by Receptor Review Tyrosine Kinases receptor) are monomers in the cell membrane. Ligand binding induces dimerization of these receptors re-sulting in autophosphorylation of their cytoplasmic do. *Cell,* vol. 103, https://ac.els-cdn.com/S0092867400001148/1-s2.0-S0092867400001148-main.pdf?_tid=ba209770-f078-40c1-a6a2-0a85f6565840&acdnat=1551963267c4c439f597c6036e73a0e12c611e413a
- The role of VLA-4 binding for experimental melanoma metastasis and its inhibition by heparin. **SCHLESINGER, MARTIN et al.** Thrombosis Research. Elsevier BV, 2014, vol. 133, 855-862
- **SCHLESINGER M ; BENDAS G.** Contribution of very late antigen-4 (VLA-4) integrin to cancer progression and metastasis. *Cancer Metastasis Rev,* 07 December 2015, vol. 34 (4), 575-91, http://link.springer.com/10.1007/s10555-014-9545-x
- Rotamers: To be or not to be?. **SCHRAUBER, HANNELORE ; EISENHABER, FRANK ; ARGOS, PATRICK.** Journal Of Molecular Biology. Elsevier BV, March 1993, vol. 230, 592-612
- **SCHWAB N ; SCHNEIDER-HOHENDORF T ; WIENDL H.** Therapeutic uses of anti- 4-integrin (anti-VLA-4) antibodies in multiple sclerosis. *Int Immunol,* 01 January 2015, vol. 27 (1), 47-53, http://www.ncbi.nlm.nih.gov/pubmed/25326459
- Single-Chain Fragment Variable Antibody Piezoimmunosensors. **SHEN, ZHIHONG et al.** Analytical Chemistry. American Chemical Society (ACS), February 2005, vol. 77, 797-805
- **SINA M ; FARAJZADEH D ; DASTMALCHI S.** Effects of environmental factors on soluble expression of a humanized anti-TNF-α scFv antibody in Escherichia coli. *Adv Pharm Bull,* 2015, vol. 5 (4), 455-61, http://dx.doi.org/10.15171/apb.2015.062
- **SINGH J ; ADAMS S ; CARTER MB ; CUERVO H ; LEE W-C ; LOBB RR et al.** Rational design of potent and selective VLA-4 inhibitors and their utility in the treatment of asthma. *Curr Top Med Chem,* 2004, vol. 4 (14), 1497-507, http://www.ncbi.nlm.nih.gov/pubmed/15544540
- Detection of prostate specific antigen based on electrocatalytic platinum nanoparticles conjugated to a recombinant scFv antibody. **SPAIN, ELAINE et al.** Biosensors And Bioelectronics. Elsevier BV, March 2016, vol. 77, 759-766
- Integrin Structure and Function. In: ZENT, Roy. **SRICHAI, MANAKAN BETSY ; ZENT, ROY.** Cell-Extracellular Matrix Interactions in Cancer. Springer, 2010, 19-41
- **SUGIURA T ; KAGEYAMA S ; ANDOU A ; MIYAZAWA T ; EJIMA C ; NAKAYAMA A et al.** Oral treatment with a novel small molecule alpha 4 integrin antagonist, AJM300, prevents the development of experimental colitis in mice. *J Crohns Colitis,* 01 December 2013, vol. 7 (11), e533-42, http://www.ncbi.nlm.nih.gov/pubmed/23623333
- **TAKADA Y ; YE X ; SIMON S.** The integrins. *Genome Biol,* 2007, vol. 8 (5), 215, http://www.ncbi.nlm.nih.gov/pubmed/17543136

- **TASHIRO, KEN-ICHIRO et al.** An IKLLI-containing peptide derived from the laminin a1 chain mediating heparin-binding, cell adhesion, neurite outgrowth and proliferation, represents a binding site for integrin a3b1 and heparan sulphate proteoglycan. *Biochemical Journal. Great Britain,* 1999, 119-126
- **THOMPSON, JULIE D ; HIGGINS, DESMOND G ; GIBSON, TOBY J.** CLUSTAL W: improving the sensitivity of progressive multiple sequence alignment through sequence weighting, position-specific gap penalties and weight matrix choice. *Nucleic Acids Research, Cambridge,* November 1994, vol. 22 (22), 4673-4680
- **TO WS ; MIDWOOD KS.** Plasma and cellular fibronectin: distinct and independent functions during tissue repair. *Fibrogenesis Tissue Repair,* 16 September 2011, vol. 4, 21, http://www.ncbi.nlm.nih.gov/pubmed/21923916
- High Yield of Recombinant Protein in Shaken E. coli Cultures with Enzymatic Glucose Release Medium EnPresso B. **UKKONEN K ; NEUBAUER A ; PEREIRA VJ ; VASALA A.** Heterologous Gene Expression in E.coli: Methods and Protocols. Springer, 2017, 127-37
- A 3D building blocks approach to analyzing and predicting structure of proteins. **UNGER, RON et al.** Proteins: Structure, Function, and Genetics. Wiley-Blackwell, 1989, vol. 5, 355-373
- Information-driven protein-DNA docking using HADDOCK: it is a matter of flexibility. **VAN DIJK, M.** Nucleic Acids Research. Oxford University Press (OUP), 28 June 2006, vol. 34, 3317-3325
- **VERLI, HUGO.** Bioinformática da Biologia à Flexibilidade Molecular. *Porto Alegre: Hugo Verli,* 2014
- **WEI SH ; PARKER I ; MILLER MJ ; CAHALAN MD.** A stochastic view of lymphocyte motility and trafficking within the lymph node. *Immunol Rev,* October 2003, vol. 195, 136-59, http://www.ncbi.nlm.nih.gov/pubmed/12969316
- **WHITE J ; KRISHNAMOORTHY S ; GUPTA D ; LANCELOT M ; MOORE N ; SAMAIK S et al.** VLA-4 blockade by natalizumab inhibits sickle reticulocyte and leucocyte adhesion during simulated blood flow. *Br J Haematol,* September 2016, vol. 174 (6), 970-82, http://www.ncbi.nlm.nih.gov/pubmed/27291690
- **WU C ; FIELDS AJ ; KAPTEIJN BA ; MCDONALD JA.** The role of alpha 4 beta 1 integrin in cell motility and fibronectin matrix assembly. *J Cell Sci,* February 1995, vol. 108, 821-9, http://www.ncbi.nlm.nih.gov/pubmed/7539441
- **YOSHIMURA N ; WATANABE M ; MOTOYA S ; TOMINAGA K ; MATSUOKA K ; IWAKIRI R et al.** Safety and Efficacy of AJM300, an Oral Antagonist of $\alpha$4 Integrin, in Induction Therapy for Patients With Active Ulcerative Colitis. *Gastroenterology,* December 2015, vol. 149 (7), 1775-1783.e2, http://www.ncbi.nlm.nih.gov/pubmed/26327130
- **YU Y ; SCHÜRPF T ; SPRINGER TA.** How natalizumab binds and antagonizes $\alpha$4 integrins. *J Biol Chem,* 08 November 2013, vol. 288 (45), 32314-25, http://www.ncbi.nlm.nih.gov/pubmed/24047894
- **XIONG H ; LI S ; YANG Z ; BURGESS RR ; DYNAN WS.** E. coli expression of a soluble, active single-chain antibody variable fragment containing a nuclear localization signal. *Protein Expr Purif,* 2009, vol. 66 (2), 172-80, http://dx.doi.org/10.1016/ipep.2009.03.002
- Crystal Structure of the Extracellular Segment of Integrin alpha Vbeta 3. **XIONG, J.-P.** Science. American Association for the Advancement of Science (AAAS), 2001, vol. 294, 339-345
- **XU HN ; LIU Y ; ZHANG L.** Salting-out and salting-in: Competitive effects of salt on the aggregation behavior of soy protein particles and their emulsifying properties. *Soft Matter.,* 2015, vol. 11 (29), 5926-32
- **ZARSCHLER K ; WITECY S ; KAPPLUSCH F ; FOERSTER C ; STEPHAN H.** High-yield production of functional soluble single-domain antibodies in the cytoplasm of Escherichia coli. *Microb Cell Fact,* 27 October 2013, vol. 12, 97, http://www.ncbi.nlm.nih.gov/pubmed/24161153